# EUROPEAN PATENT APPLICATION

(11) **EP 2 281 883 A1**
(43) Date of publication of application: **09.02.2011**
(21) Application number: 09728349.3
(22) Date of filing: 06.04.2009
(51) Int. Cl.: C12N 15/09, C12Q 1/68, G01N 33/53

(54) **METHOD FOR DETERMINATION OF DNA METHYLATION**

(30) Priority: 04.04.2008 JP 2008098006
(71) Applicant: Sumitomo Chemical Company, Limited, Tokyo 104-8260 (JP)
(72) Inventor: TOMIGAHARA, Yoshitaka, Toyonaka-shi Osaka 560-0013 (JP); SATOH, Hideo, Ibaraki-shi Osaka 567-0826 (JP); TARUI, Hirokazu, Toyonaka-shi Osaka 561-0802 (JP)
(74) Representative: Duckworth, Timothy John
(86) International application number: PCT/JP2009/057411
(87) International publication number: WO 2009/123367

(57) **Abstract**

The present invention relates to a method of measuring the content of methylated DNA in a DNA region of interest in a genomic DNA contained in a biological specimen, and so on.

SEQUENCE LISTING

<110> Sumitomo Chemical Co., Ltd.

<120> Method for measuring DNA methylation

<130> S20525W001

<150> JP2008-098006
<151> 2008-04-04

<160> 134

<210> 1
<211> 1138
<212> DNA
<213> Homo sapiens

<400> 1
taggaaagaa tagttttgct ttttcttatg attaaaagaa gaagccatac tttccctatg 60
acaccaaaca ccccgattca atttggcagt taggaaggtt gtatcgcgga ggaaggaaac 120
ggggcggggg cggatttctt tttaacagag tgaacgcact caaacacgcc tttgctggca 180
ggcgggggag cgcggctggg agcagggagg ccggagggcg gtgtgggggg caggtgggga 240
ggagcccagt cctccttcct tgccaacgct ggctctggcg agggctgctt ccggctggtg 300
cccccggggg agacccaacc tggggcgact tcaggggtgc cacattcgct aagtgctcgg 360
agttaatagc acctcctccg agcactcgct cacggcgtcc ccttgcctgg aaagataccg 420
cggtccctcc agaggatttg agggacaggg tcggaggggg ctcttccgcc agcaccggag 480
gaagaaagag gaggggctgg ctggtcacca gagggtgggg cggaccgcgt gcgctcggcg 540
gctgcggaga gggggagagc aggcagcggg cggcggggag cagcatggag ccggcggcgg 600
ggagcagcat ggagccttcg gctgactggc tggccacggc cgcggcccgg ggtcgggtag 660
aggaggtgcg ggcgctgctg gaggcggggg cgctgcccaa cgcaccgaat agttacggtc 720
ggaggccgat ccaggtgggt agagggtctg cagcgggagc aggggatggc gggcgactct 780
ggaggacgaa gtttgcaggg gaattggaat caggtagcgc ttcgattctc cggaaaaagg 840

ggaggcttcc tggggagttt tcagaagggg tttgtaatca cagacctcct cctggcgacg 900
ccctgggggc ttgggaagcc aaggaagagg aatgaggagc cacgcgcgta cagatctctc 960
gaatgctgag aagatctgaa ggggggaaca tatttgtatt agatggaagt atgctcttta 1020
tcagatacaa aatttacgaa cgtttgggat aaaaagggag tcttaaagaa atgtaagatg 1080
tgctgggact acttagcctc caattcacag atacctggat ggagcttatc tttcttac 1138

<210> 2
<211> 875
<212> DNA
<213> Homo sapiens

<400> 2
tagccagata ttccctgcgg ggcccgagag tcttccctat cagaccccgg gatagggatg 60
aggcccacag tcacccacca gactctttgt atagccccgt taagtgcacc ccggcctgga 120
gggggtggtt ctgggtagaa gcacgtccgg gccgcgccgg atgcctcctg gaaggcgcct 180
ggacccacgc caggtttccc agtttaattc ctcatgactt agcgtcccag cccgcgcacc 240
gaccagcgcc ccagttcccc acagacgccg gcgggcccgg gagcctcgcg gacgtgacgc 300
cgcgggcgga agtgacgttt tcccgcggtt ggacgcggcg ctcagttgcc gggcggggga 360
gggcgcgtcc ggtttttctc aggggacgtt gaaattattt ttgtaacggg agtcggagaa 420
ggacggggcg tgccccgacg tgcgcgcgcg tcgtcctccc cggcgctcct ccacagctcg 540
ctggctcccg ccgcggaaag gcgtcatgcc gcccaaaacc ccccgaaaaa cggccgccac 600
cgccgccgct gccgccgcgg aacccccggc accgccgccg ccgccccctc ctgaggagga 720
cccagagcag gacagcggcc cggaggacct gcctctcgtc aggtgagcga gcagagccgc 780
cgtcgcctca cgcgggaagg gcgccccggg tgtgcgtagg gcgggcgcca aggcggctcg 840
gcggggatcc gtcctcgcca ggggccgggt cccggcggga ggaggcgccc tccctgcccc 900
cgccacggcg gagcgtctgc agaatggtga caggattctg ggttcttggg cgaggggtct 960
cggcttcaac ttgacaggtg tcgggcgggt ggggc 875

<210> 3
<211> 596
<212> DNA
<213> Homo sapiens

<400> 3
taggggaacc aaactctgtt tccaggggag tggagagaga aactgggtcc ccctcccgta 60
gctcctggga cacagctgag ccagccacag gatctgggga caaccggggc ggatcccccc 120
tttcgggagg cggtggcatc agttcagagt ccgcattttt attcatcggg gaagcgtggg 180
gagaaggatg ggctggagct gggtcctggt ctgaaggaca gcagtccgga gctaacggtt 240
gagtctccaa agtcttcata ctgcagagga agcacagcgg agattagcct cagccaggat 300
ggcttcgaag ttctcaggga tccgacgcag agctaaagaa acccacctgt gcttccctcc 360
tcttctggga gtaggcagaa gactcccggg aggagaggcg aacagcggac gccaattctt 420
ttgaaagcac tgtgttcctt agcaccgcgg gtcgctacgg gcctcttgct gtcgcgggat 480
ttcggtccac cttccgattg ggccgccgca tcccggatca gatttcgcgg gcgacccacg 540
gaacccgcgg agccgggacg tgaaaggtta gaaggtttcc cgttcccatc aagccc 596

<210> 4
<211> 831
<212> DNA
<213> Homo sapiens

<400> 4
tagggctcct cgtggctgct gggagttgta gtctgaacgc ttctatcttg gcgagaagcg 60
cctacgctcc ccctaccgag tcccgcggta attcttaaag cacctgcacc gcccccccgc 120
cgcctgcaga gggcgcagca ggtcttgcac ctcttctgca tctcattctc caggcttcag 180
acctgtctcc ctcattcaaa aaatatttat tatcgagctc ttacttgcta cccagcactg 240
atataggcac tcaggaatac aacaatgaat aagatagtag aaaaattcta tatcctcata 300
aggcttacgt ttccatgtac tgaaagcaat gaacaaataa atcttatcag agtgataagg 360
gttgtgaagg agattaaata agatggtgtg atataaagta tctgggagaa aacgttaggg 420
tgtgatatta cggaaagcct tcctaaaaaa tgacatttta actgatgaga agaaaggatc 480
cagctgagag caaacgcaaa agctttcttc cttccaccct tcatatttga cacaatgcag 540
gattcctcca aaatgatttc caccaattct gccctcacag ctctggcttg cagaattttc 600
caccccaaaa tgttagtatc tacggcacca ggtcggcgag aatcctgact ctgcaccctc 660
ctccccaact ccatttcctt tgcttcctcc ggcaggcgga ttacttgccc ttacttgtca 720
tggcgactgt ccagctttgt gccaggagcc tcgcaggggt tgatgggatt ggggttttcc 780
cctcccatgt gctcaagact ggcgctaaaa gttttgagct tctcaaaagt c 831

<210> 5

<211> 623

<212> DNA

<213> Homo sapiens

<400> 5

tagggggcga gctgcctgga gttgcgttcc aggcgtccgg cccctgggcc gtcaccgcgg 60

ggcgcccgcg ctgagggtgg gaagatggtg gtgggggtgg gggcgcacac agggcgggaa 120

agtggcggta ggcgggaggg agaggaacgc gggccctgag ccgcccgcgc gcgcgcctcc 180

ctacgggcgc ctccggcagc ccttcccgcg tgcgcagggc tcagagccgt tccgagatct 240

tggaggtccg ggtgggagtg ggggtggggt gggggtgggg gtgaaggtgg ggggcgggcg 300

cgctcaggga aggcgggtgc gcgcctgcgg ggcggagatg ggcagggggc ggtgcgtggg 360

tcccagtctg cagttaaggg ggcaggagtg gcgctgctca cctctggtgc caaagggcgg 420

cgcagcggct gccgagctcg gccctggagg cggcgagaac atggtgcgca ggttcttggt 480

gaccctccgg attcggcgcg cgtgcggccc gccgcgagtg agggttttcg tggttcacat 540

cccgcggctc acgggggagt gggcagcgcc aggggcgccc gccgctgtgg ccctcgtgct 600

gatgctactg aggagccagc gtc 623

<210> 6

<211> 675

<212> DNA

<213> Homo sapiens

<400> 6

tagtcccgaa tcctctggca cacacccacc cactcaggcc gcgggtccag cccgcgaggt 60

ttaggacgga tccaggcaga ccgcaggctc cgggtcgggg caccgggtca gcgcgccggc 120

ctgaaggcgg cgtcctgggc tcgacttccc gcgcgcggag agccggcgag cccgcgtccg 180

agttcctgga cgagagccga gcctcgctta gaccgcgctc aggacccggc tcctccgcat 240

tctccggctg cccctgtgtc ctcgactcac ccctcctttc tgccgctcct tcctttcctt 300

gccctgcttt tactgttccc aaacaggacc gcttttcctg tctcccagct ggaaaggagg 360

aagggagaga gtccagaaag gatcggtgat gtggaagaaa aggggaggag gggacatgga 420

gggggagacc ggagagagaa cgtacgccga ggagtcaggc ggcgggatca aggggagtcg 480

gggtgtctgg gcgcggggca gagcgtggag gcggcagcgg ccaacggtcg ccaagacaac 540
cattctacgc gaggacgcgg cgacaggagg ggagcggcca gcaggggagg ggagcgcggg 600
ggaagaggaa agaggaagaa gcgctcagat gctccgcggc tgtcgtgaag gttaaaaccg 660
aaaataaaaa tgggc 675

<210> 7
<211> 185
<212> DNA
<213> Homo sapiens

<400> 7
taggaaggag agagtgcgcc ggagcagcgt gggaaagaag ggaagagtgt cgttaagttt 60
acggccaacg gtggattatc cgggccgctg cgcgtctggg ggctgcggaa tgcgcgagga 120
gaacaagggc atgcccagtg ggggcggcag cgatgagggt ctggccagcg ccgcggcgcg 180
gggac 185

<210> 8
<211> 450
<212> DNA
<213> Homo sapiens

<400> 8
tagaacccag tcagagggca gcttagcaat gtgtcacagg tggggcgccc gcgttccggg 60
cggacgcact ggctccccgg ccggcgtggg tgtggggcga gtgggtgtgt gcggggtgtg 120
cgcggtagag cgcgccagcg agcccggagc gcggagctgg gaggagcagc gagcgccgcg 180
cagaacccgc agcgccggcc tggcagggca gctcggaggt gggtgggccg cgccgccagc 240
ccgcttgcag ggtccccatt ggccgcctgc cggccgccct ccgcccaaaa ggcggcaagg 300
agccgagagg ctgcttcgga gtgtgaggag gacagccgga ccgagccaac gccggggact 360
ttgttccctc cgcggagggg actcggcaac tcgcagcggc agggtctggg gccggcgcct 420
gggagggatc tgcgcccccc actcactccc 450

<210> 9
<211> 1315

<212> DNA
<213> Homo sapiens

<400> 9
tagtctccgg cgctggcgcc tatggtcggc ctccgacagc gctccggagg gaccggggga 60
gctcccaggc gcccgggtga gtagccaggc gcggctcccc ggtccccccg acccccggcg 120
ccagcttttg ctttcccagc cagggcgcgg tggggtttgt ccgggcagtg cctcgagcaa 180
ctgggaaggc caaggcggag ggaaacttgg cttcggggag aagtgcgatc gcagccggga 240
ggcttcccca gccccgcggg ccgggtgaga acaggtggcg ccggcccgac caggcgcttt 300
gtgtcggggc gcgaggatct ggagcgaact gctgcgcctc ggtgggccgc tcccttccct 360
cccttgctcc cccgggcggc cgcacgccgg gtcggccggg taacggagag ggagtcgcca 420
ggaatgtggc tctggggact gcctcgctcg ggaaggggga gagggtggcc acggtgttag 480
gagaggcgcg ggagccgaga ggtggcgcgg gggtgccacc gttgccgcag gctggagaga 540
gattgctccc agtgaggcgc gtaccgtctg ggcgagggct tcattcttcc gcggcgtccc 600
tggaggtggg aaagctgggt gggcatgtgt gcagagaaag gggaggcggg gaggccagtc 660
acttccggag ccggttctga tcccaacaga ccgcccagcg tttggggacg ccgacctcgg 720
ggtgccgtgg tgcccggccc cacgcgcgcg cggggctgag gggtcggggg cgtccctggc 780
cgcccagctt taacaaaggg tgctcctctc caccccgcga ggaggggcag ctccggagac 840
ccggtcttca gcgagcgggg tcttagcgcc ggggaggtct acttcctttt ggggttgcca 900
ttttactatt attattgcct tttttttttc ttcaaaagga ctggagactg atgcatgagg 960
gggctacgga ggcgcaggag cggtggtgat ggtctgggaa gcggagctga agtgccctgg 1020
gctttggtga ggcgtgacag tttatcatga ccgtgttcag gcaggaaaac gtggatgatt 1080
actacgacac cggcgaggaa cttggcaggt aaagggggta ccagaagcgt accctcctgg 1140
attgtggaaa tgcataacga tggggccatt gggtggtaaa caaatgcagt ttgaatcagg 1200
cgtctccctc gccctttctg gagatgcgca aatcatagag aaaagagtta ctaacccagc 1260
ggtaaaccgc ctgatccaag ggcctggggg tggaggagag gcagcagttc agggc 1315

<210> 10
<211> 294
<212> DNA
<213> Homo sapiens

<400> 10

tagcacagta aagctggcct ccagaaacac gggtatctcc gcgtggtgct ttgcggtcgc 60
cgtcgttgtg gccgtccggg gtggggtgtg aggaggggac gaaggaggga aggaagggca 120
aggcgggggg ggctctgcga gagcgcgccc agccccgcct tcgggcccca cagtccctgc 180
acccaggttt ccattgcgcg gctctcctca gctccttccc gccgcccagt ctggatcctg 240
ggggaggcgc tgaagtcggg gcccgccctg tggccccgcc cggcccgcgc ttgc 294

<210> 11
<211> 592
<212> DNA
<213> Homo sapiens

<400> 11
tagttagaaa aagccaaaga ttgtgcgatt tatgccccaa acccccttgt aaggggattc 60
tcacctcaac ttgtcttctg tggtcagtgt ttcccgcccc tgaatcaggg ttactgtcac 120
tatggctttc aattggcccg gcgtaggcgc atgctctgcg cgtattggcc tccgctcctg 180
tccccagaca agcggccatc ttgggtcccg cccctaccgt ggggtcttct gggaattgca 240
gtccccgctc tgctctgtcc ggtcacagga ctttttgccc tctgttcccg ggtccctcag 300
gcggccaccc agtgggcaca ctcccaggcg gcgctccggc cccgcgctcc ctccctctgc 360
ctttcattcc cagctgtcaa catcctggaa ggtaggggcg gggaggcaag cccaagtgga 420
atactgtttc tggggcgcgg gtctgggttt ccacgcgcgt caggtcatca ccccggagcc 480
cagtggggcc ggcgccgctc acggggcggt gggcttctgt cccgagtact cttccgcccc 540
acgagggtct cagggtgggg actcgggccc cccaattccc aagcaccgac cc 592

<210> 12
<211> 729
<212> DNA
<213> Homo sapiens

<400> 12
taggcaggct gtgcggttgg gcggggccct gtgccccact gcggagtgcg ggtcgggaag 60
cggagagaga agcagctgtg taatccgctg gatgcggacc agggcgctcc ccattcccgt 120
cgggagcccg ccgattggct gggtgtgggc gcacgtgacc gacatgtggc tgtattggtg 180
cagcccgcca gggtgtcact ggagacagaa tggaggtgct gccggactcg gaaatggggt 240

aggtgctgga gccaccatgg ccaggcttgc tgcgggggga ggggggaagg tggttttccc 300
tcgcactgtc ttaaaccgat ggcctttcct tggcacaggg tccactgcag catgccaaac 360
gaggaggcag gggcgtcgtc cccccgcccc ccactgcagc actggagatg gatttcctgt 420
acttcggatc cagggttttt gacagaagag gaagaagggg gaggggtaga agtgttaagg 480
ggagtctgct gagaaaagct gtttttgaag ccagaagggg tttttgtttt tataatgcca 540
tttgacagag tggaataaca gtatctaagg aaacgggtag aggacaacaa agaatggagc 600
atattcatgg cgaggagcaa aagctctacc ccattgaaag gcttcttttc ctccctggcg 660
acaaggacac atgcattggt ggccaaaaga gagaggagac aaaaccgctg cagatggctg 720
atgtgaatc 729

<210> 13
<211> 22
<212> DNA
<213> Artificial Sequence

<220>
<223> Designed biotinated oligonucleotide for fixation

<400> 13
atagggaaag tatggcttct tc 22

<210> 14
<211> 22
<212> DNA
<213> Artificial Sequence

<220>
<223> Designed biotinated oligonucleotide for fixation

<400> 14
aagtcatgag gaattaaact gg 22

<210> 15

<211> 22
<212> DNA
<213> Artificial Sequence

<220>
<223> Designed biotinated oligonucleotide for fixation

<400> 15
gtgcttcctc tgcagtatga ag                                                22

<210> 16
<211> 22
<212> DNA
<213> Artificial Sequence

<220>
<223> Designed biotinated oligonucleotide for fixation

<400> 16
acccttatca ctctgataag at                                                22

<210> 17
<211> 22
<212> DNA
<213> Artificial Sequence

<220>
<223> Designed biotinated oligonucleotide for fixation

<400> 17
tggctcctca gtagcatcag ca                                                22

<210> 18

<211> 22
<212> DNA
<213> Artificial Sequence

<220>
<223> Designed biotinated oligonucleotide for fixation

<400> 18
cctcctttcc agctgggaga ca                                                22

<210> 19
<211> 22
<212> DNA
<213> Artificial Sequence

<220>
<223> Designed biotinated oligonucleotide for fixation

<400> 19
cactgggcat gcccttgttc tc                                                22

<210> 20
<211> 22
<212> DNA
<213> Artificial Sequence

<220>
<223> Designed biotinated oligonucleotide for fixation

<400> 20
acctgtgaca cattgctaag ct                                                22

<210> 21

<211> 22
<212> DNA
<213> Artificial Sequence

<220>
<223> Designed biotinated oligonucleotide for fixation

<400> 21
tgggagcaat ctctctccag cc                                                22

<210> 22
<211> 22
<212> DNA
<213> Artificial Sequence

<220>
<223> Designed biotinated oligonucleotide for fixation

<400> 22
aacctgggtg cagggactgt gg                                                22

<210> 23
<211> 22
<212> DNA
<213> Artificial Sequence

<220>
<223> Designed biotinated oligonucleotide for fixation

<400> 23
acactgacca cagaagacaa gt                                                22

<210> 24

<211> 22

<212> DNA

<213> Artificial Sequence

<220>

<223> Designed biotinated oligonucleotide for fixation

<400> 24

cttagatact gttattccac tc                                                22

<210> 25

<211> 30

<212> DNA

<213> Artificial Sequence

<220>

<223> Designed oligonucleotide

<400> 25

ccaacgctgg ctctggcgag ggctgcttcc                                        30

<210> 26

<211> 30

<212> DNA

<213> Artificial Sequence

<220>

<223> Designed oligonucleotide

<400> 26

ggctggtgcc cccgggggag acccaacctg                                        30

<210> 27

<211> 30
<212> DNA
<213> Artificial Sequence

<220>
<223> Designed oligonucleotide

<400> 27
gggcgacttc aggggtgcca cattcgctaa                                        30

<210> 28
<211> 30
<212> DNA
<213> Artificial Sequence

<220>
<223> Designed oligonucleotide

<400> 28
gtgctcggag ttaatagcac ctcctccgag                                        30

<210> 29
<211> 30
<212> DNA
<213> Artificial Sequence

<220>
<223> Designed oligonucleotide

<400> 29
cactcgctca cggcgtcccc ttgcctggaa                                        30

<210> 30

<211> 30
<212> DNA
<213> Artificial Sequence

<220>
<223> Designed oligonucleotide

<400> 30
agataccgcg gtccctccag aggatttgag                                        30

<210> 31
<211> 30
<212> DNA
<213> Artificial Sequence

<220>
<223> Designed oligonucleotide

<400> 31
ggacagggtc ggagggggct cttccgccag                                        30

<210> 32
<211> 30
<212> DNA
<213> Artificial Sequence

<220>
<223> Designed oligonucleotide

<400> 32
caccggagga agaaagagga ggggctggct                                        30

<210> 33

<211> 30
<212> DNA
<213> Artificial Sequence

<220>
<223> Designed oligonucleotide

<400> 33
ggtcaccaga gggtggggcg gaccgcgtgc                                        30

<210> 34
<211> 30
<212> DNA
<213> Artificial Sequence

<220>
<223> Designed oligonucleotide

<400> 34
gctcggcggc tgcggagagg gggagagcag                                        30

<210> 35
<211> 30
<212> DNA
<213> Artificial Sequence

<220>
<223> Designed oligonucleotide

<400> 35
gcagcgggcg gcggggagca gcatggagcc                                        30

<210> 36

<211> 30
<212> DNA
<213> Artificial Sequence

<220>
<223> Designed oligonucleotide

<400> 36
ggcggcgggg agcagcatgg agccttcggc 30

<210> 37
<211> 30
<212> DNA
<213> Artificial Sequence

<220>
<223> Designed oligonucleotide

<400> 37
tgactggctg gccacggccg cggcccgggg 30

<210> 38
<211> 30
<212> DNA
<213> Artificial Sequence

<220>
<223> Designed oligonucleotide

<400> 38
tcgggtagag gaggtgcggg cgctgctgga 30

<210> 39

<211> 30

<212> DNA

<213> Artificial Sequence

<220>

<223> Designed oligonucleotide

<400> 39

ggcgggggcg ctgcccaacg caccgaatag 30

<210> 40

<211> 30

<212> DNA

<213> Artificial Sequence

<220>

<223> Designed oligonucleotide

<400> 40

ttacggtcgg aggccgatcc aggtgggtag 30

<210> 41

<211> 30

<212> DNA

<213> Artificial Sequence

<220>

<223> Designed oligonucleotide

<400> 41

agggtctgca gcgggagcag gggatggcgg 30

<210> 42

<211> 30

<212> DNA

<213> Artificial Sequence

<220>

<223> Designed oligonucleotide

<400> 42

gcgactctgg aggacgaagt ttgcagggga 30

<210> 43

<211> 30

<212> DNA

<213> Artificial Sequence

<220>

<223> Designed oligonucleotide

<400> 43

cacagacgcc ggcgggcccg ggagcctcgc 30

<210> 44

<211> 30

<212> DNA

<213> Artificial Sequence

<220>

<223> Designed oligonucleotide

<400> 44

ggacgtgacg ccgcgggcgg aagtgacgtt 30

<210> 45

<211> 30
<212> DNA
<213> Artificial Sequence

<220>
<223> Designed oligonucleotide

<400> 45
ttcccgcggt tggacgcggc gctcagttgc                                        30

<210> 46
<211> 30
<212> DNA
<213> Artificial Sequence

<220>
<223> Designed oligonucleotide

<400> 46
cgggcggggg agggcgcgtc cggtttttct                                        30

<210> 47
<211> 30
<212> DNA
<213> Artificial Sequence

<220>
<223> Designed oligonucleotide

<400> 47
caggggacgt tgaaattatt tttgtaacgg                                        30

<210> 48

<211> 30
<212> DNA
<213> Artificial Sequence

<220>
<223> Designed oligonucleotide

<400> 48
   gagtcgggag aggacggggc gtgccccgac                                    30

<210> 49
<211> 30
<212> DNA
<213> Artificial Sequence

<220>
<223> Designed oligonucleotide

<400> 49
gtgcgcgcgc gtcgtcctcc ccggcgctcc                                    30

<210> 50
<211> 30
<212> DNA
<213> Artificial Sequence

<220>
<223> Designed oligonucleotide

<400> 50
tccacagctc gctggctccc gccgcggaaa                                    30

<210> 51

<211> 30
<212> DNA
<213> Artificial Sequence

<220>
<223> Designed oligonucleotide

<400> 51
ggcgtcatgc cgcccaaaac cccccgaaaa                                        30

<210> 52
<211> 30
<212> DNA
<213> Artificial Sequence

<220>
<223> Designed oligonucleotide

<400> 52
acggccgcca ccgccgccgc tgccgccgcg                                        30

<210> 53
<211> 33
<212> DNA
<213> Artificial Sequence

<220>
<223> Designed oligonucleotide

<400> 53
gaacccccgg caccgccgcc gccgccccct cct                                    33

<210> 54

<211> 30
<212> DNA
<213> Artificial Sequence

<220>
<223> Designed oligonucleotide

<400> 54
ggacagcggc ccggaggacc tgcctctcgt                                        30

<210> 55
<211> 30
<212> DNA
<213> Artificial Sequence

<220>
<223> Designed oligonucleotide

<400> 55
caggtgagcg agcagagccg ccgtcgcctc                                        30

<210> 56
<211> 30
<212> DNA
<213> Artificial Sequence

<220>
<223> Designed oligonucleotide

<400> 56
acgcgggaag ggcgccccgg gtgtgcgtag                                        30

<210> 57

<211> 30
<212> DNA
<213> Artificial Sequence

<220>
<223> Designed oligonucleotide

<400> 57
ggcgggcgcc aaggcggctc ggcggggatc                                        30

<210> 58
<211> 30
<212> DNA
<213> Artificial Sequence

<220>
<223> Designed oligonucleotide

<400> 58
cgtcctcgcc aggggccggg tcccggcggg                                        30

<210> 59
<211> 30
<212> DNA
<213> Artificial Sequence

<220>
<223> Designed oligonucleotide

<400> 59
aggaggcgcc ctccctgccc cccgccacgg                                        30

<210> 60

<211> 30
<212> DNA
<213> Artificial Sequence

<220>
<223> Designed oligonucleotide

<400> 60
cggagcgtct gcagaatggt gacaggattc                                        30

<210> 61
<211> 34
<212> DNA
<213> Artificial Sequence

<220>
<223> Designed oligonucleotide

<400> 61
ctcccgggag gagaggcgaa cagcggacgc caat                                   34

<210> 62
<211> 30
<212> DNA
<213> Artificial Sequence

<220>
<223> Designed oligonucleotide

<400> 62
ttagcaccgc gggtcgctac gggcctcttg                                        30

<210> 63

<211> 30

<212> DNA

<213> Artificial Sequence

<220>

<223> Designed oligonucleotide

<400> 63

ctgtcgcggg atttcggtcc accttccgat 30

<210> 64

<211> 30

<212> DNA

<213> Artificial Sequence

<220>

<223> Designed oligonucleotide

<400> 64

tgggccgccg catcccggat cagatttcgc 30

<210> 65

<211> 30

<212> DNA

<213> Artificial Sequence

<220>

<223> Designed oligonucleotide

<400> 65

gggcgaccca cggaacccgc ggagccggga 30

<210> 66

<211> 30
<212> DNA
<213> Artificial Sequence

<220>
<223> Designed oligonucleotide

<400> 66
cgtgaaaggt tagaaggttt cccgttccca                                        30

<210> 67
<211> 30
<212> DNA
<213> Artificial Sequence

<220>
<223> Designed oligonucleotide

<400> 67
ctgaacgctt ctatcttggc gagaagcgcc                                        30

<210> 68
<211> 30
<212> DNA
<213> Artificial Sequence

<220>
<223> Designed oligonucleotide

<400> 68
tacgctcccc ctaccgagtc ccgcggtaat                                        30

<210> 69

<211> 30

<212> DNA

<213> Artificial Sequence

<220>

<223> Designed oligonucleotide

<400> 69

tcttaaagca cctgcaccgc ccccccgccg                                        30

<210> 70

<211> 30

<212> DNA

<213> Artificial Sequence

<220>

<223> Designed oligonucleotide

<400> 70

cctgcagagg gcgcagcagg tcttgcacct                                        30

<210> 71

<211> 30

<212> DNA

<213> Artificial Sequence

<220>

<223> Designed oligonucleotide

<400> 71

caaagggcgg cgcagcggct gccgagctcg                                        30

<210> 72

<211> 30

<212> DNA

<213> Artificial Sequence

<220>

<223> Designed oligonucleotide

<400> 72

gccctggagg cggcgagaac atggtgcgca 30

<210> 73

<211> 30

<212> DNA

<213> Artificial Sequence

<220>

<223> Designed oligonucleotide

<400> 73

ggttcttggt gaccctccgg attcggcgcg 30

<210> 74

<211> 30

<212> DNA

<213> Artificial Sequence

<220>

<223> Designed oligonucleotide

<400> 74

cgtgcggccc gccgcgagtg agggttttcg 30

<210> 75

<211> 30
<212> DNA
<213> Artificial Sequence

<220>
<223> Designed oligonucleotide

<400> 75
tggttcacat cccgcggctc acgggggagt                                        30

<210> 76
<211> 30
<212> DNA
<213> Artificial Sequence

<220>
<223> Designed oligonucleotide

<400> 76
gggcagcgcc aggggcgccc gccgctgtgg                                        30

<210> 77
<211> 30
<212> DNA
<213> Artificial Sequence

<220>
<223> Designed oligonucleotide

<400> 77
agggggagac cggagagaga acgtacgccg                                        30

<210> 78

<211> 30

<212> DNA

<213> Artificial Sequence

<220>

<223> Designed oligonucleotide

<400> 78

aggagtcagg cggcgggatc aaggggagtc 30

<210> 79

<211> 30

<212> DNA

<213> Artificial Sequence

<220>

<223> Designed oligonucleotide

<400> 79

ggggtgtctg ggcgcggggc agagcgtgga 30

<210> 80

<211> 30

<212> DNA

<213> Artificial Sequence

<220>

<223> Designed oligonucleotide

<400> 80

ggcggcagcg gccaacggtc gccaagacaa 30

<210> 81

<211> 30

<212> DNA

<213> Artificial Sequence

<220>

<223> Designed oligonucleotide

<400> 81

ccattctacg cgaggacgcg gcgacaggag 30

<210> 82

<211> 35

<212> DNA

<213> Artificial Sequence

<220>

<223> Designed oligonucleotide

<400> 82

gggagcggcc agcaggggag gggagcgcgg gggaa 35

<210> 83

<211> 30

<212> DNA

<213> Artificial Sequence

<220>

<223> Designed oligonucleotide

<400> 83

taggaaggag agagtgcgcc ggagcagcgt 30

<210> 84

<211> 30
<212> DNA
<213> Artificial Sequence

<220>
<223> Designed oligonucleotide

<400> 84
gggaaagaag ggaagagtgt cgttaagttt                                        30

<210> 85
<211> 30
<212> DNA
<213> Artificial Sequence

<220>
<223> Designed oligonucleotide

<400> 85
acggccaacg gtggattatc cgggccgctg                                        30

<210> 86
<211> 30
<212> DNA
<213> Artificial Sequence

<220>
<223> Designed oligonucleotide

<400> 86
cgcgtctggg ggctgcggaa tgcgcgagga                                        30

<210> 87

<211> 30
<212> DNA
<213> Artificial Sequence

<220>
<223> Designed oligonucleotide

<400> 87
gaacaagggc atgcccagtg ggggcggcag                                        30

<210> 88
<211> 35
<212> DNA
<213> Artificial Sequence

<220>
<223> Designed oligonucleotide

<400> 88
cgatgagggt ctggccagcg ccgcggcgcg gggac                                  35

<210> 89
<211> 33
<212> DNA
<213> Artificial Sequence

<220>
<223> Designed oligonucleotide

<400> 89
ccccattggc cgcctgccgg ccgccctccg ccc                                    33

<210> 90

<211> 30
<212> DNA
<213> Artificial Sequence

<220>
<223> Designed oligonucleotide

<400> 90
aaaaggcggc aaggagccga gaggctgctt                                        30

<210> 91
<211> 30
<212> DNA
<213> Artificial Sequence

<220>
<223> Designed oligonucleotide

<400> 91
cggagtgtga ggaggacagc cggaccgagc                                        30

<210> 92
<211> 30
<212> DNA
<213> Artificial Sequence

<220>
<223> Designed oligonucleotide

<400> 92
caacgccggg gactttgttc cctccgcgga                                        30

<210> 93

<211> 30
<212> DNA
<213> Artificial Sequence

<220>
<223> Designed oligonucleotide

<400> 93
ggggactcgg caactcgcag cggcagggtc                                        30

<210> 94
<211> 34
<212> DNA
<213> Artificial Sequence

<220>
<223> Designed oligonucleotide

<400> 94
tggggccggc gcctgggagg gatctgcgcc cccc                                   34

<210> 95
<211> 35
<212> DNA
<213> Artificial Sequence

<220>
<223> Designed oligonucleotide

<400> 95
tagtctccgg cgctggcgcc tatggtcggc ctccg                                  35

<210> 96

<211> 30
<212> DNA
<213> Artificial Sequence

<220>
<223> Designed oligonucleotide

<400> 96
acagcgctcc ggagggaccg ggggagctcc                                        30

<210> 97
<211> 30
<212> DNA
<213> Artificial Sequence

<220>
<223> Designed oligonucleotide

<400> 97
caggcgcccg ggtgagtagc caggcgcggc                                        30

<210> 98
<211> 30
<212> DNA
<213> Artificial Sequence

<220>
<223> Designed oligonucleotide

<400> 98
tccccggtcc ccccgacccc cggcgccagc                                        30

<210> 99

<211> 30

<212> DNA

<213> Artificial Sequence

<220>

<223> Designed oligonucleotide

<400> 99

ttttgctttc ccagccaggg cgcggtgggg                                        30

<210> 100

<211> 30

<212> DNA

<213> Artificial Sequence

<220>

<223> Designed oligonucleotide

<400> 100

tttgtccggg cagtgcctcg agcaactggg                                        30

<210> 101

<211> 35

<212> DNA

<213> Artificial Sequence

<220>

<223> Designed oligonucleotide

<400> 101

ctctgcgaga gcgcgcccag ccccgccttc gggcc                                  35

<210> 102

<211> 31
<212> DNA
<213> Artificial Sequence

<220>
<223> Designed oligonucleotide

<400> 102
ttgcgcggct ctcctcagct ccttcccgcc g                                    31

<210> 103
<211> 34
<212> DNA
<213> Artificial Sequence

<220>
<223> Designed oligonucleotide

<400> 103
cccagtctgg atcctggggg aggcgctgaa gtcg                                 34

<210> 104
<211> 35
<212> DNA
<213> Artificial Sequence

<220>
<223> Designed oligonucleotide

<400> 104
gggcccgccc tgtggccccg cccggcccgc gcttg                                35

<210> 105

<211> 30
<212> DNA
<213> Artificial Sequence

<220>
<223> Designed oligonucleotide

<400> 105
tccagaaaca cgggtatctc cgcgtggtgc                                        30

<210> 106
<211> 33
<212> DNA
<213> Artificial Sequence

<220>
<223> Designed oligonucleotide

<400> 106
tttgcggtcg ccgtcgttgt ggccgtccgg ggt                                    33

<210> 107
<211> 30
<212> DNA
<213> Artificial Sequence

<220>
<223> Designed oligonucleotide

<400> 107
ccaagtggaa tactgtttct ggggcgcggg                                        30

<210> 108

<211> 30
<212> DNA
<213> Artificial Sequence

<220>
<223> Designed oligonucleotide

<400> 108
tctgggtttc cacgcgcgtc aggtcatcac                                        30

<210> 109
<211> 30
<212> DNA
<213> Artificial Sequence

<220>
<223> Designed oligonucleotide

<400> 109
cccggagccc agtggggccg gcgccgctca                                        30

<210> 110
<211> 30
<212> DNA
<213> Artificial Sequence

<220>
<223> Designed oligonucleotide

<400> 110
cggggcggtg ggcttctgtc ccgagtactc                                        30

<210> 111

<211> 30
<212> DNA
<213> Artificial Sequence

<220>
<223> Designed oligonucleotide

<400> 111
ttccgcccca cgagggtctc agggtgggga                                        30

<210> 112
<211> 30
<212> DNA
<213> Artificial Sequence

<220>
<223> Designed oligonucleotide

<400> 112
ctcgggcccc ccaattccca agcaccgacc                                        30

<210> 113
<211> 30
<212> DNA
<213> Artificial Sequence

<220>
<223> Designed oligonucleotide

<400> 113
gttgggcggg gccctgtgcc ccactgcgga                                        30

<210> 114

<211> 30
<212> DNA
<213> Artificial Sequence

<220>
<223> Designed oligonucleotide

<400> 114
gtgcgggtcg ggaagcggag agagaagcag                                        30

<210> 115
<211> 30
<212> DNA
<213> Artificial Sequence

<220>
<223> Designed oligonucleotide

<400> 115
ctgtgtaatc cgctggatgc ggaccagggc                                        30

<210> 116
<211> 30
<212> DNA
<213> Artificial Sequence

<220>
<223> Designed oligonucleotide

<400> 116
gctccccatt cccgtcggga gcccgccgat                                        30

<210> 117

<211> 30

<212> DNA

<213> Artificial Sequence

<220>

<223> Designed oligonucleotide

<400> 117

tggctgggtg tgggcgcacg tgaccgacat 30

<210> 118

<211> 30

<212> DNA

<213> Artificial Sequence

<220>

<223> Designed oligonucleotide

<400> 118

gtggctgtat tggtgcagcc cgccagggtg 30

<210> 119

<211> 2661

<212> DNA

<213> Homo sapiens

<400> 119
   acagacatgt gccaccatgc ccagctaatt ttttgtttgt ttgtttgttt gtttgtattt 60
   ttagtagaga tggggttttg ccatgttggc caggctggtc tcgaactcct gacctcgaat 120
   gataatgatc cgccgcttgg cctccaaagt gctaggatta caggtgtgag ccactgcgcc 180
   aggcctgggc actttcttta gtagtttgag gagcaacatt tttgacagtg tccttctgct 240
   caagattcaa gatcccagat aaaattaaac catctagaga gatggcttga ttggccaaac 300
   ctggatctca tgaccacttc ttgaagtggg taagtctcat aaatgctcag tccttccact 360
   atgcaactga gtggggtggg tgggaagccc ctcaaaggaa aatccggttg ttcttactag 420
   aaagaaaagg aaaatggatg tgaggcagtc aaaatcagca gaggtccacc acaccaccaa 480
   aatgtggtga ttaaatatgg agagacagag actaacagag gtatgtgaat attgaagtat 540
   gtctggacaa tagcccaatg atgagaccaa taaaatggtt accaaaatct ggttttgagt 600
   agtagtgtta aatcagacca tttagtaacc attttttgtt gcaaagtttc tagcactgcc 660
   caaaccctga gtggtatatg aataactcgt ccattatgta tctctttcca gtcagcataa 720
   tttatccccc acctatattc ttttctgacc actcctactt ccttctcttt accaaaatct 780
   aaactctaag gctgtttctt cagcaacttc tttgtttaga ttggaagata aattaaacag 840
   catgcgatgt tttactgact ttcagtattt aacagaggtg atttaatttt tttttaaatc 900
   caaagtcaaa cttctttata agatgaagga gaaaaatgtc ttataaaatg catatgtgaa 960
   gatgccttct gagtgctttc tcatgcagac ttgttctagt ctttaatgaa tcttccttgt 1020
   agacactgtg gagatgaaag atggttctcc acttctactc aaagtacaaa tcaggccggc 1080
   attttgaaaa agagacaggt ttattcatag ctgcagcgtt agctggcttt gttccctgta 1140
   caatttcact tttggttatt aaaatattca ctgtaggaaa taaatttgta acccatttct 1200
   catattacct acacacagaa aaacaaaatt tgatatcctg gggtttattt gctgagggcg 1260
   cttcccataa aagcgagaga gtgtgcgttg ggaaatgtgt ctggttaact cttttatgga 1320
   taaactttag tcacaatcct cccccgcccc cctctcaccc ccagcaccct cccaacctcc 1380
   cgacttcccg cctctcaagg gctggtgacc taatagcatt tttcttcgtg catattttgg 1440
   cgtcgcccca tggcctggct gccttcgcct gtctgagttt tttgaaattc ctgcatgttc 1500
   gccccagatt aagccagtgt gtctcaggat gtgtgttccg ttttgttctt tccccttaac 1560
   gctccctgtg caacgtgtct gggggaggga gggcagggac gggagagagg gaggggcaga 1620
   ggcgaggagc tgtccgcctt gcacgtttcc aatcgcatta cgtgaacaaa tagctgaggg 1680
   gcggccgggc cagaacggct tgtgtaactt tgcaaacgtg ccagaaagtt taaatctctc 1740
   ctccttcctt cactccagac actgcccgct ctccgggact gccgcgcggc tccccgttgc 1800
   cttccaggac tgagaaaggg gaaagggaag ggtgccacgt ccgagcagcc gccttgactg 1860
   gggaagggtc tgaatcccac ccttggcatt gcttggtgga gactgagata cccgtgctcc 1920
   gctcgcctcc ttggttgaag atttctcctt ccctcacgtg atttgagccc cgtttttatt 1980
   ttctgtgagc cacgtcctcc tcgagcgggg tcaatctggc aaaaggagtg atgcgcttcg 2040
   cctggaccgt gctcctgctc gggcctttgc agctctgcgc gctagtgcac tgcgcccctc 2100
   ccgccgccgg ccaacagcag cccccgcgcg agccgccggc ggctccgggc gcctggcgcc 2160
   agcagatcca atgggagaac aacgggcagg tgttcagctt gctgagcctg ggctcacagt 2220
   accagcctca gcgccgccgg gacccgggcg ccgccgtccc tggtgcagcc aacgcctccg 2280
   cccagcagcc ccgcactccg atcctgctga tccgcgacaa ccgcaccgcc gcggcgcgaa 2340
   cgcggacggc cggctcatct ggagtcaccg ctggccgccc caggcccacc gcccgtcact 2400
   ggttccaagc tggctactcg acatctagag cccgcgaacc tggcgcctcg cgcgcggaga 2460
   accagacagc gccgggagaa gttcctgcgc tcagtaacct gcggccgccc agccgcgtgg 2520
   acggcatggt gggcgacgac ccttacaacc cctacaagta ctctgacgac aacccttatt 2580
   acaactacta cgatacttat gaaaggccca gacctggggg caggtaccgg cccggatacg 2640
   gcactggcta cttccagtac g 2661

<210> 120
<211> 1953
<212> DNA
<213> Homo sapiens

<400> 120
tataaattcc acgcaggcat tgaattgaat ttgttcttaa ccaaatgcgt tttatctata 60
cctggcagga atctagaagt gaaattacaa gatttatttc attttaattc tattatgaag 120
catttaatca caaataccct gaaaatgaaa agataattta tcattttacc ttgactgagc 180
aactctcctc acttcacatt catgaatcca taacgcagag aggagactgg atgattaagt 240
gtttgattag agaaaacaga ttaacctagc aaacataata aatttggctc ataagcagga 300
tggctttata aatgctcaca atacctctcc tgtataaaat catgaaccac ttcctacagt 360
gatgactcca tcgaaatagt tgagaaacat aaagcaaatg catgtttatg gctttctctt 420
tgagacatta aaagggtatt gaaaggcata tctgattcag cttataactc tggatatata 480
ttaaggaaca tgtaagaaaa tattaatgca taaaaaaagc tacaacttct caagtgttct 540
agtttccact ttgtcaataa ttacgttttc aatgtccttc tgtggactgt ttccaaaggt 600
gccaatccag acccaaagtt tcagatcact cagattcacc cttaaccttc ataacacaac 660
ccaatagctt tacgaaaaaa gttgcatatt taggtagttg ttatcccatt atgacaaaat 720
acataaaatt agcgagatat tttttagcct tcaaataagt gggaaaaaat ccttttagct 780
gagattccat ttacatcaga ataaaaatct aagttatgac taggttgaag caacgtcctg 840
tgcagcgctc cataaagttc acttagtctt caagggttcc ttacttagct aggttagtat 900
tcctggcctc tttttttagc agtgagaaaa aggatactct ccctgcccca gctttatttt 960
taaactcaca gccatatcct ggaggtctct gctggctatt tggcgcgtgg gggcggaggg 1020
gggccggggg aggggggcgg ggcggggtct ggaggtctgt gctggctatc tggcgtgtgt 1080
gtgtgtgtgt gtgtgtgtgt gtgtggttgg aggtctctgc tggctatctg gcgtgtgtgt 1140
gtgtgtggtg tggtgtgtgt aagcagtgag gttgttttag ggccagtcct tcctccgcca 1200

ctttgctgac tcaaagaccc agaggctttc ttggggtgca ggtaccatga ttccttgggc 1260
cctaagggaa tttttgttag gctagaagag tgggtgtact catgatgggt gtacccgaac 1320
attcctgggc tcaacaaaac cgattatctt tataaccgcg gcgcctagca cagcgcctgg 1380
tgccctaaac gttggctgcg ggaacgtccg agacgcgggt gcggagccgg gggcggaata 1440
actggttgcg cggcgctttg accgtaggcg ctggagcgcg tgcgttgcgt gcgcgcgcgg 1500
aggcggctgc gtcggggcgc gagaaggtgc agttccccgg cgggcgggcg ggcgggcggg 1560
cgaagctggg ctcggggcca agcgaggtct agccggagcg actgtgcccc gcctcctggg 1620
cggagcgggc ggctccccat ggtcagagcc tcgtgccggc tcggcagcgc ccggacgccg 1680
agcccagcgc gtcggccccc cggcgtgcgg gcgtctcaga gccgcggagg ggccgccggg 1740
accgtttcag cgtggcggcg ctggtgctgg cgttggccct ggaggacggc cccgagtgat 1800
ggctggcgcc tgcctcccgg gtgtctcccg ggtacagatg gagtcgtccc gcggccgccg 1860
gcggcaaggt cggcagctgc gaggccaaga gagaccccag gacacacaca gctgcctccc 1920
ggtgcgagaa gaagaccccg gcttgagagt gag 1953

<210> 121
<211> 889
<212> DNA
<213> Homo sapiens

<400> 121
cggccccatg gctccgtgtc gtgtccaagg gatgggctgg cacctcttgg accaggctta 60
ccaccagggc ccttctctga agccccagtc tgaccggcct gctgctggga atccccctct 120
gcccccacac taacctctgc tggggctgag ccagggcgcg tcggacagtc agggcgaccc 180
agccagggcg accgttggcc ccgctcctat ggggcagcag ggaccgacgt cagcagggtg 240
gggcgggcac ccgagtggta tgccccgccc tgccccgcct gcccgccctg gtggccgtct 300
gggggcgaca agtcctgaga gaaccagacg gaagcgcgct gggactgaca cgtggacttg 360
ggcggtgctg cccgggtggg tcagcctggg ctgggaggca gccccgggac acagctgtgc 420
ccacgccgtc tgagcacccc aagcccgatg cagccacccc cagacgaggc ccgcagggac 480
atggccgggg acacccagtg gtccaggtgt ggcgggggtg aggggagggg gggtgggagc 540
ggtggagatg gggccgtggg gagggagctg agatactgcc acgtgggacg atgctaggtg 600
gggagggctg agctgggcgg gctcctctgg ctgtggggcc ccctgtgttc cttgtgggag 660
gtggaaggaa gtgagtgccc tgtccttcct ccctgccatg agattccagg accggacctg 720
gcaagtgccc tatcccagcc agtgttcctg gggctcttcc aggcagggct atgttcccca 780

ggccaggggc attgtcctgg acagtcagga ggcatacccc tcgccaggtg gaaccaccct 840
gtgtatgcat gaccctgaca agcaggcgcc aggacagtca ggaggccag 889

<210> 122
<211> 863
<212> DNA
<213> Homo sapiens

<400> 122
gttgttgggt gtgaatggag aactgtgggc cctccccgac accttccagc gggacggcaa 60
cgggggccca gggggtgggc gccatcaacc ccgtcccacc gccaggacgg cgcgggggag 120
ggccggcggg ggcggggcgt cctgtaaggc gcggccccca cccgcgggcg gggcggcatt 180
cctgggaggc cggcgctctg acgtggaccc gggggccgcg ggcacggcgg gggggcggcg 240
gtccgggggc ttcttaaacc ccccgccccg gcccagcccg cacttcccga gcaccgctcc 300
gaccctggag ggagagagag ccagagagcg gccgagcgcc taggaggccc gccgagcctc 360
gccgagcccc gccagccccg gcgcgagaga agttggagag gagagcagcg cagcgcagcg 420
agtcccgtgg tcgcgcccca acagcgcccg acagcccccg atagcccaaa ccgcggccct 480
agccccggcc gcacccccag cccgcgccag catgatgaac aacagcggct actcagacgc 540
cggcctcggc ctgggcgatg agacagacga gatgccgtcc acggagaagg acctggcgga 600
ggacgcgccg tggaagaaga tccagcagaa cacattcacg cgctggtgca atgagcacct 660
caagtgcgtg ggcaagcgcc tgaccgacct gcagcgcgac ctcagcgacg ggctccggct 720
catcgcgctg ctcgaggtgc tcagccagaa gcgcatgtac cgcaagttcc atccgcgccc 780
caacttccgc caaatgaagc tggagaacgt gtccgtggcc ctcgagttcc tcgagcgcga 840
gcacatcaag ctcgtgtcca tag 863

<210> 123
<211> 2198
<212> DNA
<213> Homo sapiens

<400> 123
aagagaggca cactccctct accacaccga gggagggggc gttgagctga gaaaggttga 60
gagaatgagg gacccaggta ggtggacatc ggccaagaaa ggaaccacag cgggaggtaa 120

gaccgagagt ccccagcttg aagcgtcacc actccgggat tcccagattc caacgcgagc 180
ctggggaaag cccacagtgg agagagtccg gctggcaggg aatggcccta cccccggggt 240
gaaatctcgg agggtcgtgc agccgagtcg cgcctctgcg ctgatgcgtg agagatgccg 300
gacgtcgcgt ttgcctgtgc gagcctcgcg gatgctgtgc agtcttggtc ccctctgcgt 360
gtgtctaacg ccgaatgctg gtgtctcgag gtgtgagctt cggggccggt gtctttaaag 420
aaccaaagat tcttaaggag tgatgatctg ggtagagcgg cccgacgtag ccgcgctccc 480
aggtctcggt gcgagtcctg cggacagacc agaggagacc tgctggccag atgccccggg 540
cccaaggcgg acgccagact gtctctgcgc cagccgggct ggccttcgga atggatcagg 600
cacccgggag gccggagtgg atctcagacc ctcaagccgg gaacaaaccc gtcgatgccc 660
gtgggcctgg agtccgcctc ctccttcccg ccccacccct acccctgcct ccgaaaggct 720
tcttcgctgg tcagtagctg cgtgcccgtc tgcctgaggc tgggtcagaa ttggcgggct 780
ggtaacgacc ccgtgcacaa gcggctccca gtctctccag aaagggccga tgactaaggg 840
gtgggggtgg gggcggaggg ctggaaggtg ttagggaaga acgttagcgg cctatcctgt 900
cttcagcagc gccctctcat cttctagctc tgacgccgag cagagcagtt ggagctcggg 960
actgggaact gctggaattc ctatttagac ttctagacag tctagaaaca agaacctttc 1020
tttccctggg cctcagtttc cttgtctgta aaatcaaaag gcgggctcta ggtgtaggcc 1080
ttcttttcgc ttggtgattc tggattcctt tccttggatc cgtggggagg gggtggcagc 1140
aacagtccag ggcgttggcc gtcctgtgcc tcaagtacgt agtccccgtg cccgccccct 1200
caacaccccc agcagcccgc ccccctaagc ccgcagagca gggagctgag tgggaggggc 1260
agaggcgggg ccggttccca gtccctgctg gcggactaga gtggcgcggg ctgagcgtaa 1320
aacctgggat agccactccc ccttttcctt atccccgccc ccctgccatt ggctcccggg 1380
agaggttgac atcaaagccg cggtcttata taagccagat ccgcagggga gtccgcagaa 1440
gggttaaaca ggtctttggg cttcggcgac ctcgcccgcg gcagaaaccg gtaagaagac 1500
agtgggctgc gcgtctcatt ttcagccttg cccggactct cccaaagccg gcgcccagta 1560
gtggctccag agcccacagg tggcccccgg cagtctctgg ggcgcatgga gcggcgttaa 1620
tagggctggc ggcgcaggcc agtagccgct ccaacatgaa cctcgtgggc agctacgcac 1680
accatcacca ccatcaccac ccgcaccctg cgcaccccat gctccacgaa cccttcctct 1740
tcggtccggc ctcgcgctgt catcaggaaa ggccctactt ccagagctgg ctgctgagcc 1800
cggctgacgc tgccccggac ttccctgcgg gcgggccgcc gcccgcggcc gctgcagccg 1860
ccaccgccta tggtcctgac gccaggcctg ggcagagccc cgggcggctg gaggcgcttg 1920
gcggccgtct tggccggcgg aaaggctcag gacccaagaa ggagcggaga cgcactgaga 1980
gcattaacag cgcattcgcg gagttgcgcg agtgcatccc caacgtgccg gccgacacca 2040
agctctccaa gatcaagact ctgcgcctag ccaccagcta catcgcctac ctgatggacg 2100

tgctggccaa ggatgcacag tctggcgatc ccgaggcctt caaggctgaa ctcaagaagg 2160
cggatggcgg ccgtgagagc aagcggaaaa gggagctg 2198

<210> 124
<211> 1945
<212> DNA
<213> Homo sapiens

<400> 124
ctggatgaca gagtgagact ccgtctcaaa aaaaaagctc catttgggag gccgaggagg 60
gtggattacc tgaggtcagg agtttgagac cagcctggcc cacataggga aaccccatct 120
ctactaaaaa tacaaaaatt agtcaggtgt ggtggctgac acctataatc ccagctactt 180
gggaagctga ggcagggaga atcacttgaa ccggggaggt ggaggttgca gtgagctgag 240
atcatgccac tgcactccag cctgggcgac agggtgagat tctgtctcaa acaaacaaat 300
ttaaaagctc cgaatcctcc aaaaatacca agattttcct gtcggtaact agagatgggt 360
actgatgatt atttttaata ggtgattttc aaagatgtga acgttatcca tggagattta 420
agtctccaaa aggaaaaaaa atgcatacct ttatactaaa acttcatcac cagtcaaatt 480
tggatcatca ctaaattggc ttctacacct ctctcctaat ataaggtact tgtgtaagtt 540
tgcagttgtg agacacttat ttcctcattt ttaatgtctt ctcagtaggg ccactgatat 600
agtcactatt tgactgacca gaatggttgg cactggtgat tggctcataa agtgccctcg 660
atttaggggg ctcaattatc aaaggtttaa atcctagccc aaaccattgc tgtgatgggg 720
gttaatcaat gaaccactca gcttcacttg caaaagcggg atcacaatag ccgctttcgt 780
catgacccag cctaggtgag atttagtact taagtacact gccaggcaca caaggttaat 840
ttaacaattt aacacatttg tttcctcatc catttctcca aaccttccaa ctaatcctaa 900
cgttcgttcg gccaaatggg ccaggaattc acttaaacaa aaacaaaaaa caaaacaaac 960
aaaaaaacac tccctggggc ttggggaagg aggcaccgcc gcccatgtcg cagtctgggg 1020
gtggctcagt cctcagcacc cagatctacg gccataatgc tcttcgaggc caaggagccc 1080
ggatgcgggg cgttgccgaa ggcgtcttgc tcaggctgcg ggaaaggaga ggggtgggag 1140
cggggtgggg gcatcgcgac ccagggcaag gcggcgagtc gccgtcttcg agtcccacct 1200
gtccgaagcg gggtgagaaa aggcaaaaca tggcaaagcc atgcacctcc cagggtgggc 1260
aactcacggc cggtgaacgc cggaccctta gcagtttcca gacctttgga accggaagcg 1320
gagcctgaga gcgcgcccga gagggcgtga acgggaccgc tttcccggaa gtgcttgcgg 1380
cctctgccca gcgagctgcc ccggggtctc tctggtttcc taatcagggc aacgccgcgg 1440

gagagaacct ttaccttggc tgcactaagt tctcggtgcc actccctggc agggcgggac 1500
cttgtttagg ccctgtgatc gcgcggttcg tagtagcgca aggcgcagag tggaccttga 1560
cccgcctagg gcgggaagag tttggcccgc cgggtcccaa agggcagaat ggacgggctc 1620
ctaaatccca gggaatcctc taaattcatt gcagaaaaca gtcgggatgt gtttattgac 1680
agcggaggcg tacggagggt ggcagagctg ctgctggcca aggcggcggg gccagagctg 1740
cgcgtggagg ggtggaaagc ccttcatgag ctgaacccca gggcggccga cgaggccgcg 1800
gtcaactggg tgttcgtgac agacacgctc aacttctcct tttggtcgga gcaggacgag 1860
cacaagtgtg tggtgaggta cagagggaaa acatacagtg ggtactggtc cctgtgcgcc 1920
gccgtcaaca gagccctcga cgaag 1945

<210> 125
<211> 2379
<212> DNA
<213> Homo sapiens

<400> 125
aagcttgtgg tttacttgga cctctgcctc atctttcttc ttttgcgctt cagcctgcgc 60
attcgcttcc tccactaggc tctcatggtg cagaggtttc caagaagatg gtgtgaaggc 120
cgagatcatt tggttatatt ataaaataga atgcaaattc acacaagttt ttgtttttta 180
tttatttatt tttttagaga tgaggtcttg ctatgttgtt tagtctggtc tcgaactcct 240
ggcctcgtga tcctcccacc ttgacctccc aaagtgctgg gattacaggc ctgaggcctg 300
agccactaca cccaactgaa ttcacatttt tttttttctt ttctgagacg gagtctcact 360
ctgtcaccca gtatggagtg cagtggcgcg actgcggctc actgcaagct ccgtctctcg 420
ggttcaagtg attctcatgc ctcagccccc caagtagctg gaattacagg ggtgcactac 480
cacacctggc taatttttct gttttagtag agatggggtt tcaccatgtt gcctggtctc 540
aaactcctga ctttaagtga tccacacacc tcagcctccc aaagtgctgg gattacaggt 600
gtgagcctcc acacccggcc gaattcacat gaattttaaa gtgatgtctt caaagtggtt 660
tcactgtggg gatgggcagc tttttgttat acatctagaa cgttcctctt ctgtttctat 720
gaatactcgg ttggaaaggg ctgaaaaacg gtcttaagag attatctgat tcgtttccca 780
gttttattac tcacatatca gctgtaattt gagcacgttt tctgattgag acaagactca 840
gatggtatta aacattacta caacacatcc gggcacggtg gctcacgcct gtaatcccag 900
cactttggga ggccgaggcg ggcggatcac gaggtcagga gatcgagacc atcctggcta 960
acacggtgaa gccctgtctc tactaaaaat acaaaaaatt aggcgggcat ggtggcgggc 1020

gcctgtagtc ccagctactc gggaggctga ggcaggagaa tggcgtgaac ccgggaggcg 1080
gagcttgcag tgagccgaga tcgcgccact gcactccagc ctgggcgaca gagcaagact 1140
ccatctcaaa aaaaaaaaaa aaaaaaaaaa actacaacac tataaattca tatctattat 1200
aatagtactt tgtgcagggc cctaccctaa gtccttaacc gaacccggaa gcgagaagat 1260
gacttttgtt tgtttttaga gatgggcgcc tggctctgtc gccagcctgg agtgtggggg 1320
cgcgatctcg actcacagca gcctccacct cccgagttca ggcgatcttc ctgcctcagc 1380
ccctcgagga gctgggacca ccggcgcgct ccatcgcgcc cggctaggag ctgactttga 1440
atccgggctc tgcgcctggc cttctgcatc tctataaggg aagacatctg tgacctcggg 1500
gcaaaggtca aattagatcc tgggtaggat cctgttcccg ctgcccctcg ggctggcact 1560
gccaggagta ctcagagctc aaagctggga tctgcagtcc cttacccact cagtgcacgc 1620
cgcctaaggc tttgcgcttc acctttactc acctcgaagc cctggacatc cgcatctgcc 1680
ctaagacttc tcacctcagt agcagaagga agtcgcgtca gctggccaca gcctctctcc 1740
taggagaccg tccgggaaaa gcgagtcagg gtagaccctg aggcccctca gctccggcta 1800
ttttcagatc tgtcgctcct tcaccctcag cctttcaaac aggccactcc aaaaaaaagc 1860
ccaatcacag ccttccttct tctcctggcc ttccggcact gtccaatcaa cgtacgccat 1920
ctatcggtta gtggtgttgc ggggccaccc ttcccgctgg tttccctcgt ggtgtgtaaa 1980
ggcagagagg aaaggcgagg ggtgttgacg ccaggaaggt tccatcttgg ttaagggcag 2040
gagtccctta cggacttgtc tgaggaaaga caggaaagcg ccagcatctc caccttcccc 2100
ggaagcctcc ctttgccagg cagaaagggt ttcccatggg gccgcccctg gcgccgcgcc 2160
cggcccacgt acccggggag gccgggcccc ggaggacgag ggaaagcagg ccgggcgccg 2220
tgagcttcgc ggacgtggcc gtgtacttct ctcccgagga gtgggaatgc ctgcggccag 2280
cgcagagggc cctgtaccgg gacgtgatgc gggagacctt cggccacctg ggcgcgctgg 2340
gtgaggccgg gccctccggc cgggaccccc agtccgtcg 2379

<210> 126
<211> 933
<212> DNA
<213> Homo sapiens

<400> 126
gagacgtact ctggctctgt cgcccaggct ggagcgcaat ggcgccatct cggcgcactg 60
caacctccac ctcccgggtt caagcgattc tactgcctca gcctcccgag tagctgggac 120
tacaggcgcg cactaccaag cccggctaat ttcttttgta tttttagtag agactgggtt 180

tcacgatgtt ggccgggctg gtctggaagt cttgacctca agcgtgcgcc ctctccgcca 240
ctgggtaagg cggggggcgg aatagggggc ttgcaatttc acactagagg cgggcgccgt 300
gggggaaaga agagtcacgt ctcccacggt tcgtagagga aggcctgcct gagcctggag 360
cgggggcggg agagccacag tttggcatcc ccagggcatc ccccagcccg cagactacca 420
ggcctccaga ggacaggacc ccacccccgg ccacaggccc tgcccccagc actccccgca 480
ccccgcctcc aagactcctc cgcccactcc gcacccaact tataaaaacc gtcctcgggc 540
gcggcgggga gaagccgagc tgagcggatc ctcacacgac tgtgatccga ttctttccag 600
cggcttctgc aaccaagcgg gtcttacccc cggtcctccg cgtctccagt cctcgcacct 660
ggaaccccaa cgtccccgag agtccccgaa tccccgctcc caggctacct aagaggatga 720
gcggtgctcc gacggccggg gcagccctga tgctctgcgc cgccaccgcc gtgctactga 780
gcgctcaggg cggacccgtg cagtccaagt cgccgcgctt tgcgtcctgg gacgagatga 840
atgtcctggc gcacggactc ctgcggctcg gccaggggct gcgcgaacac gcggagcgca 900
cccgcagcca gctgagcgcg ctggagcggc gcc 933

<210> 127
<211> 6096
<212> DNA
<213> Homo sapiens

<400> 127
atctgcacct cctcatatag ggttgatcca agtttcacag acatcactga gttcttagtg 60
gactcagcta ttggggctgt tctcacactt tttttttctt tgcaagaatc agcaatgggt 120
gcaagtggac ctgtgtagga cgtccagtga aacattgtgt tggtgaatca gctagaatcc 180
atccaagaac tcagccagcc tggtgtgggg tgagatctga tccttgaatg tccctcagtg 240
gcttttaggg ctggcaggtt cagaagggcc ctctcatcac ccccccaggg cctcattcct 300
tgtttaacac tttgctatca cagtcttgaa tccttgtaat tgaacaatgg accccacatt 360
ttcactttgc actggtttct gattctgtaa ccgatcctgt ccccctctct tgtctcattc 420
actctgggaa ttgtccccac attctgagac ctttcagcag tgccccaacg aggttcctgc 480
ccttatctga agctccaccc tcacccccat ggcggcaccg caggcagccc tgcttttgcg 540
tcccgcgtag gcaggctgtg caccggagtc acgaccccct gattcagcct aggcagccac 600
agcttgactg ctcccgccgg acaagcccta ctgtgctatc tgccgctctt cccttcctct 660
tcccaggggg tccgcgtcag gggaggcgca gctgtgtgca ttccgggagc ttcagacccc 720
cgtgtccagc agctccttcg tttcctgggt gctggggcgg ccttcccagc gaagagctca 780

actcagcggg acgtttggag gctctctgcc ccaaggcgct ggggagtgtg cggcgggaca 840
gtcgtgcttg cctttttcac tttcagagtg tccacgcccc acccgtttgg tcactgcagg 900
tcagtccagt ccagcccggc ccaccccacc ggtgcgtgtc tgtcgcacgt ggcagacgcc 960
atactctctg ttcttgttta aagcccagga tctactgggc cctggaggca agaggtgaac 1020
gcagcggaat ccacgctgag ctgcccggga acggagcttc caaccccaga aggaggactc 1080
tgtgctccta caccttaacc ctttttagcc cgaaacttct ccaacttcct tggctttgtt 1140
tagagctcga cagcgccgcc ccctggcgct cgttgtgagg acagtagagg agagaggcaa 1200
gggtgttttt aaacagtttg cctctcacca ttatgggggc gacccgaggg ggagacccac 1260
tcttccgcat tcccggtaag tgaaccaccg gaagaggtcg aaagtgacgg attcccatgt 1320
cctcctccag cccccccccc accctgccca tccacaggac ggtggctctt cagtgccctt 1380
tgccgagcaa gtggcgtttc tatgcacgtg ggtatcaatt cggactctgg acgaaatgga 1440
aacctcctta gccgacccgg gtgggatcag ctgggatcct gcgcgctccc ctggggggtt 1500
gccagccact ctgttggggt gcaagaagca ccatccttcg gaagctgggc cgaaactggc 1560
caggctgact cgctcccacg cgcccgcccc tacccggcgc cgcagcaatt cacctgccac 1620
cgcctctgag ccgggtccgg acttcggcgc cctgacagtg tccccgcgac ttccccaccc 1680
gatgagatgg ggtctggcgt tggccagtgc gtgtccaggg actcgcgggt ccctggccag 1740
ccatggggca gagggcgctg gtgttaggcc agtcttcccc accctgcccc gtcaccccag 1800
ccacacccac tgtcctgtga ggccaagcgc gctccgctgg tttcctgagc caggcacctt 1860
ggccgcggac aggatccagc tgtctctcct tgcgatcctg tcttcgggga agtccacgtc 1920
ctaggcaggt cctcccaaag tgcccttggt gccgatcacc cctcccagcg tcttgcaggt 1980
cctgtgcacc acctccccca ctccccattc aaagccctct tctctgaagt ctccggttcc 2040
cagagctctt gcaatccagg ctttccttgg aagtggctgt aacatgtatg aaaagaaaga 2100
aaggaggacc aagagatgaa agagggctgc acgcgtgggg gcccgagtgg tgggcgggga 2160
cagtcgtctt gttacagggg tgctggcctt ccctggcgcc tgcccctgtc ggccccgccc 2220
gagaacctcc ctgcgccagg gcagggttta ctcatcccgg cgaggtgatc ccatgcgcga 2280
gggcgggcgc aagggcggcc agagaaccca gcaatccgag tatgcggcat cagcccttcc 2340
caccaggcac ttccttcctt ttcccgaacg tccagggagg gagggccggg cacttataaa 2400
ctcgagccct ggccgatccg catgtcagag gctgcctcgc aggggctgcg cgcagcggca 2460
agaagtgtct gggctgggac ggacaggaga ggctgtcgcc atcggcgtcc tgtgcccctc 2520
tgctccggca cggccctgtc gcagtgcccg cgctttcccc ggcgcctgca cgcggcgcgc 2580
ctgggtaaca tgcttggggt cctggtcctt ggcgcgctgg ccctggccgg cctggggttc 2640
cccgcacccg cagagccgca gccgggtggc agccagtgcg tcgagcacga ctgcttcgcg 2700
ctctacccgg gccccgcgac cttcctcaat gccagtcaga tctgcgacgg actgcggggc 2760

cacctaatga cagtgcgctc ctcggtggct gccgatgtca tttccttgct actgaacggc 2820
gacggcggcg ttggccgccg gcgcctctgg atcggcctgc agctgccacc cggctgcggc 2880
gaccccaagc gcctcgggcc cctgcgcggc ttccagtggg ttacgggaga caacaacacc 2940
agctatagca ggtgggcacg gctcgacctc aatggggctc ccctctgcgg cccgttgtgc 3000
gtcgctgtct ccgctgctga ggccactgtg cccagcgagc cgatctggga ggagcagcag 3060
tgcgaagtga aggccgatgg cttcctctgc gagttccact tcccagccac ctgcaggcca 3120
ctggctgtgg agcccggcgc cgcggctgcc gccgtctcga tcacctacgg caccccgttc 3180
gcggcccgcg gagcggactt ccaggcgctg ccggtgggca gctccgccgc ggtggctccc 3240
ctcggcttac agctaatgtg caccgcgccg cccggagcgg tccaggggca ctgggccagg 3300
gaggcgccgg gcgcttggga ctgcagcgtg gagaacggcg gctgcgagca cgcgtgcaat 3360
gcgatccctg gggctccccg ctgccagtgc ccagccggcg ccgccctgca ggcagacggg 3420
cgctcctgca ccgcatccgc gacgcagtcc tgcaacgacc tctgcgagca cttctgcgtt 3480
cccaaccccg accagccggg ctcctactcg tgcatgtgcg agaccggcta ccggctggcg 3540
gccgaccaac accggtgcga ggacgtggat gactgcatac tggagcccag tccgtgtccg 3600
cagcgctgtg tcaacacaca gggtggcttc gagtgccact gctaccctaa ctacgacctg 3660
gtggacggcg agtgtgtgga gcccgtggac ccgtgcttca gagccaactg cgagtaccag 3720
tgccagcccc tgaaccaaac tagctacctc tgcgtctgcg ccgagggctt cgcgcccatt 3780
ccccacgagc cgcacaggtg ccagatgttt tgcaaccaga ctgcctgtcc agccgactgc 3840
gaccccaaca cccaggctag ctgtgagtgc cctgaaggct acatcctgga cgacggtttc 3900
atctgcacgg acatcgacga gtgcgaaaac ggcggcttct gctccggggt gtgccacaac 3960
ctccccggta ccttcgagtg catctgcggg cccgactcgg cccttgcccg ccacattggc 4020
accgactgtg actccggcaa ggtggacggt ggcgacagcg gctctggcga gcccccgccc 4080
agcccgacgc ccggctccac cttgactcct ccggccgtgg ggctcgtgca ttcgggcttg 4140
ctcataggca tctccatcgc gagcctgtgc ctggtggtgg cgcttttggc gctcctctgc 4200
cacctgcgca agaagcaggg cgccgccagg gccaagatgg agtacaagtg cgcggcccct 4260
tccaaggagg tagtgctgca gcacgtgcgg accgagcgga cgccgcagag actctgagcg 4320
gcctccgtcc aggagcctgg ctccgtccag gagcctgtgc ctcctcaccc ccagctttgc 4380
taccaaagca ccttagctgg cattacagct ggagaagacc ctccccgcac cccccaagct 4440
gttttcttct attccatggc taactggcga gggggtgatt agagggagga gaatgagcct 4500
cggcctcttc cgtgacgtca ctggaccact gggcaatgat ggcaattttg taacgaagac 4560
acagactgcg atttgtccca ggtcctcact accgggcgca ggagggtgag cgttattggt 4620
cggcagcctt ctgggcagac cttgacctcg tgggctaggg atgactaaaa tatttatttt 4680
ttttaagtat ttaggttttt gtttgtttcc tttgttctta cctgtatgtc tccagtatcc 4740

actttgcaca gctctccggt ctctctctct ctacaaactc ccacttgtca tgtgacaggt 4800
aaactatctt ggtgaatttt tttttcctag ccctctcaca tttatgaagc aagccccact 4860
tattccccat tcttcctagt tttctcctcc caggaactgg gccaactcac ctgagtcacc 4920
ctacctgtgc ctgaccctac ttcttttgct cttagctgtc tgctcagaca gaacccctac 4980
atgaaacaga aacaaaaaca ctaaaaataa aaatggccat ttgctttttc accagatttg 5040
ctaatttatc ctgaaatttc agattcccag agcaaaataa ttttaaacaa aggttgagat 5100
gtaaaaggta ttaaattgat gttgctggac tgtcatagaa attacaccca aagaggtatt 5160
tatctttact tttaaacagt gagcctgaat tttgttgctg ttttgatttg tactgaaaaa 5220
tggtaattgt tgctaatctt cttatgcaat ttcctttttt gttattatta cttatttttg 5280
acagtgttga aaatgttcag aaggttgctc tagattgaga gaagagacaa acacctccca 5340
ggagacagtt caagaaagct tcaaactgca tgattcatgc caattagcaa ttgactgtca 5400
ctgttccttg tcactggtag accaaaataa aaccagctct actggtcttg tggaattggg 5460
agcttgggaa tggatcctgg aggatgccca attagggcct agccttaatc aggtcctcag 5520
agaatttcta ccatttcaga gaggcctttt ggaatgtggc ccctgaacaa gaattggaag 5580
ctgccctgcc catgggagct ggttagaaat gcagaatcct aggctccacc ccatccagtt 5640
catgagaatc tatatttaac aagatctgca gggggtgtgt ctgctcagta atttgaggac 5700
aaccattcca gactgcttcc aattttctgg aatacatgaa atatagatca gttataagta 5760
gcaggccaag tcaggccctt attttcaaga aactgaggaa ttttctttgt gtagctttgc 5820
tctttggtag aaaaggctag gtacacagct ctagacactg ccacacaggg tctgcaaggt 5880
ctttggttca gctaagctag gaatgaaatc ctgcttcagt gtatggaaat aaatgtatca 5940
tagaaatgta acttttgtaa gacaaaggtt ttcctcttct attttgtaaa ctcaaaatat 6000
ttgtacatag ttatttattt attggagata atctagaaca caggcaaaat ccttgcttat 6060
gacatcactt gtacaaaata aacaaataac aatgtg 6096

<210> 128
<211> 2500
<212> DNA
<213> Homo sapiens

<400> 128
   acccacttct gtgtgtggat agtatcctgc aggagagatg ttgtctgcag tgtgagctgg 60
   gcccaccgga gtgtgtgaat aggatcctgc aggagaaatg gaatccggag tgtgagctgc 120
   atccgctgta gagggtggat aaaatcctgc aggaaagatg gcatctggaa tgtcagcggg 180
   agccaccgac ctctgaggat gcaccccgca ggtgtgatgc ggggccagtt ccaaggctgg 240
   gttaggtttt accctggctt ctgtgttgta ctctcattct cttcctcttt cttctaatac 300
   ctgctctggg aggcatcagg ccatgtccag tgtgcaggcc atggagaccc acacggcaag 360
   gaactggaac cccctgccag cagcctcggg ggtccagtcc ttagatggtg ccctgtggtc 420
   agcaatgcac ctgtgacctc cgggctatgt ctcgtggtag ttgcttttgt gttttaacat 480
   agcaacagga aactagccta ttacccacca atcccattcc aggctgcttt caaacgcagc 540
   tcaggctaga acaccagcac ggggacacag ctgagacttg gggtttgcga cgggaacacg 600
   cccatgctgt gcctctgaat ctggcaccgt caccctgtgg cctgggttca gcaacttggc 660
   ctcaccttcc ttgtctgtga aattcagact gggtccttgt gagatgattg gagagaatgt 720
   atgaactatg tgagaacgcc acctttgtgc gtatctcacg cagtgtcttc cctcctttcc 780
   aaagtcttct gctgtctcta gacacacccg acgtgggggg ggggggttcc ctgggtctcc 840
   tcctaggtct gtcccaggag ggcacgcact gaaggccgcg agaatcccgg gggctgcatt 900
   gcgccgcgcc aaggactcca cacaggacct ttcattttcc caactgtgct gagccaggcg 960
   gccggcagag agcaggtggc tgacaggccc cggggagccg gaccgcctgg gtctaatctt 1020
   cccgcagact cccttgctgt gcgctttggg gcttgggcct cagtttcctc aaaaggaatg 1080
   aggggctttt ttggaacgtt aaataatttc ctacgtggtt gcgggtaggg agaaggagaa 1140
   agagaggagc gcgcctgcgc gcctggaatc gtgcccggat cagagcaagc gctctaaaag 1200
   tgttacaaac attaaggcgc caactaaaaa acccgtagtg agcgcaggca gaaaccacgg 1260
   gtaagagaag tggagaagct tcgcgtaggc cccagggtcc cgagccccga gtctcgagcg 1320
   cagaatcagg ggtgccaatg ctctcctccg cgcccccgag cgctcgcctt ggccatgcgg 1380
   gccgccccac cgggatgagg gcgctcaggc cggacgctgg ggcccccggt tctcgccccg 1440
   ccccgccctc ggggattcag aggggccggg aggagcctcg cgcatgtgca cagctggcgc 1500
   cccccgcccc ccgcgcacag ctgggacgtg ggccgcggcc gggcgggcgc agtcgggagc 1560
   cggccgtggt ggctccgtgc gtccgagcgt ccgtccgcgc cgtcggccat ggccaagcgc 1620
   tccaggggcc ccgggcgccg ctgcctgttg gcgctcgtgc tgttctgcgc ctgggggacg 1680
   ctggccgtgg tggcccagaa gccgggcgca gggtgtccga gccgctgcct gtgcttccgc 1740
   accaccgtgc gctgcatgca tctgctgctg gaggccgtgc ccgccgtggc gccgcagacc 1800
   tccatcctgt gagtgccgcg gggacgccgg ggggcgcggg gtccggggct tcgtggagat 1860
   ccgggagcgc aggggtgatc ggaggtgggg ggcgcggagg gtggaggggg catcgggcgc 1920
   gcggggggcc tggggacttg ggacgcagaa gggaacctcc gaagggggac gtggggggac 1980
   ctgggcgcgg ggacccgctg ggcctttgtt cgccctgcgg gagacgccga ggggcggaac 2040
   agagcgctgt gcgcgcggcc ttcgtagccg cctttgttcg gaactcggaa tccccgcagg 2100
   actgggaagt tgttggagcc tccggggctc cccccgctcg cctcccgccg ccccctctca 2160
   tgctccgccg gcctcccgct tccccctggt tcgcggcccc tcctccgctc acctttcccc 2220
   cgctcaggac ccctcggtcc ccctccgctc cccgagcgcg gcgcagcccc ctccgtcctc 2280
   ccagccccct ccgccccgtt cctcgtcctg ttcgctcccc tcctccgctc ctcttcctcc 2340
   tccccttcct cctcctcctc cccttcctcc tcctcctccc cttcctcctc ctcctccctt 2400
   cccctcctcc tccccccctt ccttctcctc ccccagcctc cgccctctcc ccctcccccg 2460
   ccccttggag cgcagtgccc accccatccc cccgcgccgg 2500

<210> 129
<211> 2200
<212> DNA
<213> Homo sapiens

<400> 129
cctcgccccc tccagccggc cccccgggcc cctcctctcg gcgcccggac cttggccctc 60
cctctccttt cccacttctc tctttgccct aacttcgccc ccatcccccg ctcatttcct 120
ctcgcacccg ggctcgccaa tccctctttc caagtccctc ttccagcccg gccttcctct 180
cgggttcgcc ccccttctcc ccaatctccg tcctcttccc tcccttcgcc ctccccccct 240
tccttcctct tcccctcacc caaccctggt tcccctcgtt cctcagtccc gatctctccc 300
ttactctgtc cccgcccact ctgcgccggc ctctcagtcc gggttgagcc ccacgtgtgg 360
acggccgcgc ccccactgac agccgccgcc cgccggcccg ccccgcgccc cgccgggcct 420
ctaaaacccc cgcgccgcgc cctccaccgc cgcatcttct ccagcgccca gcctcccgcc 480
ctctctcttg ctggccgcac gccccggccc cgcgcacctc cgcccggctc cgcagccgct 540
acccgcgctt cgttgccctg tgggactccg agcgagcccg gagggaaccc tcctcttctt 600
ctgggggcga cttttgtttg cttgcctgtt tctttctggt gacttttgca gctttccaat 660
atccgtcttc ggagcgcacg ggaatccgcc gagctctgcg tgcaggccct tttttctttt 720
gaggttcaca ttttttgaaa ttttacgcca gggcttttgt aatttcctcc cccgcccgct 780
gacggtcctg gagtcgctcg gggctttagg ccggttatgc aacgtgtacc gctcggggct 840
gccggctgca cctccgccgc gcctcgccgc tcactgcgct agacccggcg ccccgcgtct 900
cgcttcgcgg gcagtcaggg ggccggcgct ctgtcgaggt ctccagctag agcagggagc 960
ccgagcccga gggagtcccc ggagccgacg aagggcttat tagaccctga ctcttttctg 1020
aggcgcgcag attttgtctt tgatcactcc ctctccgcgg gtctacggcc gcgcgctttc 1080
ggcgccggcg atggggagaa gacggaggct gtgtctccag ctctacttcc tgtggctggg 1140
ctgtgtggtg ctctgggcgc agggcacggc cggccagcct cagcctcctc cgcccaagcc 1200

gccccggccc cagccgccgc cgcaacaggt tcggtccgct acagcaggct ctgaaggcgg 1260
gtttctagcg cccgagtatc gcgaggaggg tgccgcagtg gccagccgcg tccgccggcg 1320
aggacagcag gacgtgctcc gagggtaagt gggcaagcgg ctccgcacct agggctccgg 1380
cttgggggag gggggaatcc tcagtttggc ggctttctgg cccactccgt cccagaccct 1440
ttagctggag cctagagctg cagccccctt tgccagaata tccaaagacc cccaggagcg 1500
cgtccccctt ttccttccca accccgcagc tcagcgggcg gaaagccctc tctccggggg 1560
ttgggcggcg ggtggttagg gggtccaggg gtgccgatcg cagagcgtgt gcagagctcg 1620
cgctgcggga acaggttctg aatgtccggc ggcaggcggg cctgggtccg cctgctgcag 1680
gggccagaga agcctgcttg ctccccacgt cggggccgcc gctcgtgagc cttttgtttg 1740
aggacgtgtg cagggttcac agctcacctt ctcatcgtca acccgagcgc tccaccttgc 1800
gacgcgcttt ccttgacacg tcggggccaa agtaacagtt gaccaaggag gaatggattt 1860
gggaaggagg gcaaggattc tttggaacgg aatggtccct ttgttctctg catctggaag 1920
ctagaatagt agcaaattat atgtttccat gcctcttttc gccctttaaa aaggcaggca 1980
agggacgaca gatgaaaggc agtgtttaga catttctgac cctcctgcat tccagcatct 2040
agctcttttg cttccacgtc tgcctcccga tctccaataa tttgaagtgt aattttgatt 2100
tgtttgttgt cctgaaatct actcgctcgg ggcattgctt acgaagaccg tttatatgtt 2160
gctgcatccc tctacctatc tgttacgtga ccgcgcttgt 2200

<210> 130
<211> 2000
<212> DNA
<213> Homo sapiens

<400> 130
ttggaagaaa aggatctccg aggaaggggc tgagagaagg gcagggtgaa ctggactaaa 60
ggccagagta ggaaggagaa gaggggccaa aaaagaaggg gatgaaatta agcacagaag 120
atgggtaaag aaaaaagtat cagggaaagg gcaaaataag agaaagcctt gaggataaga 180
gggtagaagg ctaaagaaca aggggaccac tgggtcgggg aagcgctgcc tgaacggcgg 240
gacagtgaca aagaaagggc gctggcgata ttcgcaccaa gggtgcgaaa cgcaatcggg 300
aggtgagaaa tggaaagaag gcgaatgccc ggctacaagt agcctgggac tgaaagggga 360
cctgggggag gggctgggcc cagggcagaa aagtccaggt tcccatgcgg cctgggccca 420
cgtggagcgg gcgctgaatc accgttcagc cgcccccctc ccctcctccc cgaccggtgc 480
ccgcagtccc cgcctcctcg gccgccgcct ccacggggcg gggccctggc ccgggaccag 540

cgccgcggct ataaatgggc tgcggcgagg ccggcagaac gctgtgacag ccacacgccc 600
caaggcctcc aagatgagct acacgttgga ctcgctgggc aacccgtccg cctaccggcg 660
ggtaaccgag acccgctcga gcttcagccg cgtcagcggc tccccgtcca gtggcttccg 720
ctcgcagtcg tggtcccgcg gctcgcccag caccgtgtcc tcctcctata agcgcagcat 780
gctcgccccg cgcctcgctt acagctcggc catgctcagc tccgccgaga gcagccttga 840
cttcagccag tcctcgtccc tgctcaacgg cggctccgga cccggcggcg actacaagct 900
gtcccgctcc aacgagaagg agcagctgca ggggctgaac gaccgctttg ccggctacat 960
agagaaggtg cactacctgg agcagcagaa taaggagatt gaggcggaga tccaggcgct 1020
gcggcagaag caggcctcgc acgcccagct gggcgacgcg tacgaccagg agatccgcga 1080
gctgcgcgcc accctggaga tggtgaacca cgagaaggct caggtgcagc tggactcgga 1140
ccacctggag gaagacatcc accggctcaa ggagcgcttt gaggaggagg cgcggttgcg 1200
cgacgacact gaggcggcca tccgcgcgct gcgcaaagac atcgaggagg cgtcgctggt 1260
caaggtggag ctggacaaga aggtgcagtc gctgcaggat gaggtggcct tcctgcggag 1320
caaccacgag gaggaggtgg ccgaccttct ggcccagatc caggcatcgc acatcacggt 1380
ggagcgcaaa gactacctga agacagacat ctcgacggcg ctgaaggaaa tccgctccca 1440
gctcgaaagc cactcagacc agaatatgca ccaggccgaa gagtggttca aatgccgcta 1500
cgccaagctc accgaggcgg ccgagcagaa caaggaggcc atccgctccg ccaaggaaga 1560
gatcgccgag taccggcgcc agctgcagtc caagagcatc gagctagagt cggtgcgcgg 1620
caccaaggag tccctggagc ggcagctcag cgacatcgag gagcgccaca accacgacct 1680
cagcagctac caggtaggaa ccgcggctgc gcggccagcc tgcgccagcg ccagcgccgc 1740
gcgcccccga cacttgggct cgtgcccagg cgccctctcc gccgcgctcc ctggtggccg 1800
ctcgctagag cacgcgcgcc gcagacctag ggtatttgcg gatcagcgtc ctcgcccatc 1860
tcatcctcca cactccgccc ccacccacct gccccagctg ctaagggtct tgaccttttt 1920
cagaaacgtg catcttttcc agttctaatt ttgcacgctt gcacgtttaa agcaggaggg 1980
atgaattcgg tagtggataa 2000

<210> 131
<211> 2300
<212> DNA
<213> Homo sapiens

<400> 131
tcagattgtc attgggaggg tgaataaatg aatgcttgca ttatgagagt ttgggggcag 60

aaatatgcca cagactctta tctgaagcca tcagatttag tggctgcgaa cccaccgaag 120
tcagggattt acatttttta cagcaacgag agaaaacttc ccctttcctc tgcagaagtc 180
aggactggat ctcaaaaata gaaatgtgtc ctcctaaatg tgtgcccatc cccgtggttg 240
acaaacaacg gatttcccaa gatagctgcc acacacttgg tttctaatct ctgtattgct 300
tccccgccag aatgtcgaag tccttcccga atatgcccag tcatactttc tgaacttttg 360
agcaaacacc gtccggcttc ttgtgctttc ctcaaagacc ccaggcaccg gcagggagga 420
cacaggccgg ggcagagcgc ccctgcgcgg gggattcctg ccactccgcg ccagcctgcg 480
gcgcaaacgc tcttctcagc cgcagtccca cccgctgctg gcaatctgaa tgaggagccg 540
cgctattttt acctccccgg ctgcaatcct ttatatttac atgcaggaag caaatatata 600
agggattaag aaggagatgc gtggccttag tttatccaga gcaggaagag gttggaatag 660
gagagggtat gtgaagtctg ggtggtggaa aaaggcaggt ggacttcggc tggttgtttt 720
ctcccgatca tccctgtctc tggcctggaa acccccgtac tctctttctt ctggcttatc 780
cgtgactgcc ggctccccct ccaccgcccc catcttttga ggtaccaccc gtcacctccg 840
atgctgcttg ggctgctgca tcactctgct gctttacccc cttccccgcc ccccaacaaa 900
gcatgcgcag tgcgttccgg gccaggcaac agcagcagca cagcatccag caacagcatc 960
agcacccgaa gccccgctcg ggcgcgctct cggggggcgg ggcgcacgcc cgctccgcgc 1020
gtccccgcgc cgctcgctcc cgcgcgtccc cgcgccgctc gctcccgcgc gccgcctcag 1080
catcctcagg cccggcggca gcccccgcag tcgctgaagc ggccgcgccc gccgggggag 1140
ggagtagccg ctggggaggc tccaagttgg cggagcggcg aggacccctg gactcctctg 1200
cgtcccgccc cgggagtggc tgcgaggcta ggcgagccgg gaaagggggc gccgcccagc 1260
cccgagcccc gcgccccgtg ccccgagccc ggagccccct gcccgccgcg gcaccatgcg 1320
cgccgagccg gcgtgaccgg ctccgcccgc ggccgccccg cagctagccc ggcgctctcg 1380
ccggccacac ggagcggcgc ccgggagcta tgagccatga agccgcccgg cagcagctcg 1440
cggcagccgc ccctggcggg ctgcagcctt gccggcgctt cctgcggccc ccaacgcggc 1500
cccgccggct cggtgcctgc cagcgccccg gcccgcacgc cgccctgccg cctgcttctc 1560
gtccttctcc tgctgcctcc gctcgccgcc tcgtcccggc cccgcgcctg gggggctgct 1620
gcgcccagcg gtgggtatgg ccccgtgccc tttgcgttgg ctttcccgcg gggccctgca 1680
gaggaaagcg aagggcgcgc gggtccgtgt gctccgggct tgtccccggc tcggcctttc 1740
cttccctccc tgcctgtctt tccacccttc tcgttcccaa acccccattc atcccagttc 1800
acttttggaa gtccatttct gttgcattcg cgaaaaaccc attccaattc ttgttggttc 1860
cactgggagg tgtttagtgg atcctgggtc cctcagcgat ctctgtgcaa cttgcggagg 1920
ggcaaccagt ggatgggaaa tacagcgagg gagcaagttg ctacttgcgt ggtggaacct 1980
taatgtgaat gcggggagga tgtagtgata atagtggtaa tgggctgttt cctcaaattt 2040

cgtatccggc gcattcagtg cggttggaat taaggtgggg gaggcacact tcggggacca 2100
aagaattaag gtgctgaaga catacttcat gcacgacctt tggttctgat ttctcaaagt 2160
gcttgtcatt ataatgaaca attaatataa taccatcttc tatatattga tgattggaag 2220
tcactgaaag cagaaagctg gctttgtcag gaaaataaaa agaaattggg aagctgccag 2280
catctgtatc cctacatggc 2300

<210> 132
<211> 3000
<212> DNA
<213> Homo sapiens

<400> 132
tactgccgac tttaggtctc tctggatctc aggccccctt ctctaagatg catcctagag 60
gaccaaaaat acactttatt tgggcttcgc ctgcttttgt ggaagggtag tttactagag 120
gatataatct cgtgttttaa tttgctctct ctcctaaagg aaatgtggag aaaaaaaaaa 180
agcagaaatt ggaaataacc aatatttagt ttatttcatt cgattcttag gggaactggt 240
gaggagccta agatgatttt cccttcctag agaaagaatc caaagtccag ggaaatagcg 300
acaggggagt tcaagactgc ccctgctagt ccttccttgg ctactctccg ctgcgatcgc 360
aggatagctc tcattagcag gagaatcggg caagtgtgtg gataagtaga gagtgtgttg 420
aacaacttgt aacgttttat gaaatacgca ttgtcatggt tccctaaaag gctttgcgga 480
agccgtttgt ctttactaat caagtcttta cttacacaaa agtagaagta gaagtagttt 540
tagaaaacat actaacaatc ttctatcccc ttgaagacca gagtagcaga aaacaggtga 600
tttgcattat aaaattgcac tcactttttc ctcctttcag atttcacatt acattagccc 660
atttgtgtta cggtgtataa aaaatggaac aggcgcctcc actgcattgt tctcctttaa 720
aaatagatca cttacaccct aactttgttt tccttaaatt cgattcttaa caggagagct 780
ttctattatt tcagatggag tgaggttgca cgactgggat ggaagaaagg aatcccttaa 840
atttggggga atttctgttc tctgttccaa gaccatttta cttggggtgt gggggtgggc 900
gcggcggtca gggcagtgga acgcagtcgc ggctgcgcca tccctgcact tccaggcgcg 960
cgggagggac cggcggggac gcgagctgcg gactctggcg aactcggggg aggcagacag 1020
ggggaggcgg acacccagcc ggcaggcgtc tcagcctccc cgcagccggc gggcttttct 1080
cctgacagct ccaggaaagg cagacccctt ccccagccag ccaggtaagg taaagactgc 1140
tgttgagctt gctgttactg agggcgcaca gaccctgggg agaccgaagc ttgccactgc 1200
gggattctgt ggggtaacct gggtctacgg aagtttcctg aaagagggga gaagggtttg 1260

catttttcct atggaggatt cttctctctc tagcatttcg tttgatgtat tcaactggta 1320
gaagtgagat ttcaacaggt agcagagagc gctcacgtgg aggaggtttg gggcgccgcg 1380
gcgccacccc cacccctcct cgggaccgcg cctatttcta aagttacacg tcgacgaact 1440
aacctatgct ttaaattcct ctttccagcc ccgtgagtcc gcggcgacat tgggccgtgg 1500
ggtggctggg aacggtcccc tcctccggaa aaaccagaga acggcttgga gagctgaaac 1560
gagcgtccgc gagcaggtcc gtgcagaacc gggcttcagg accgctgagc tccgtagggc 1620
gtccttgggg gacgccaggt cgccggctcc tctgccctcg ttgagatgga caacgcctcg 1680
ttctcggagc cctggcccgc caacgcatcg ggcccggacc cggcgctgag ctgctccaac 1740
gcgtcgactc tggcgccgct gccggcgccg ctggcggtgg ctgtaccagt tgtctacgcg 1800
gtgatctgcg ccgtgggtct ggcgggcaac tccgccgtgc tgtacgtgtt gctgcgggcg 1860
ccccgcatga agaccgtcac caacctgttc atcctcaacc tggccatcgc cgacgagctc 1920
ttcacgctgg tgctgcccat caacatcgcc gacttcctgc tgcggcagtg gcccttcggg 1980
gagctcatgt gcaagctcat cgtggctatc gaccagtaca acaccttctc cagcctctac 2040
ttcctcaccg tcatgagcgc cgaccgctac ctggtggtgt tggccactgc ggagtcgcgc 2100
cgggtggccg gccgcaccta cagcgccgcg cgcgcggtga gcctggccgt gtgggggatc 2160
gtcacactcg tcgtgctgcc cttcgcagtc ttcgcccggc tagacgacga gcagggccgg 2220
cgccagtgcg tgctagtctt tccgcagccc gaggccttct ggtggcgcgc gagccgcctc 2280
tacacgctcg tgctgggctt cgccatcccc gtgtccacca tctgtgtcct ctataccacc 2340
ctgctgtgcc ggctgcatgc catgcggctg gacagccacg ccaaggccct ggagcgcgcc 2400
aagaagcggg tgaccttcct ggtggtggca atcctggcgg tgtgcctcct ctgctggacg 2460
ccctaccacc tgagcaccgt ggtggcgctc accaccgacc tcccgcagac gccgctggtc 2520
atcgctatct cctacttcat caccagcctg agctacgcca acagctgcct caaccccttc 2580
ctctacgcct tcctggacgc cagcttccgc aggaacctcc gccagctgat aacttgccgc 2640
gcggcagcct gactccccca gcgtccggct ccgcaactgc ccgccactcc tggccagcga 2700
gggaggagcc ggcgccagag tgcgggacca gacaggccgc ctaggcctcc tggggaaacc 2760
gactcgcgcc ccatacccga cctagcagat cggaagcgct gcgactgtgc ccgcaggttg 2820
accttgccaa gccctccagg tgatgcgcgg ccatgccggg tgaggagaac tgaggctgag 2880
atcgccacac tgagggctcc ctaaagccga ggtggaggaa gaggagggta gaggaggagg 2940
gcggtattgc tgggaaccgc cccctccctg ccctgctccc tgctgcccca cccgagccct 3000

<210> 133
<211> 3000
<212> DNA

<213> Homo sapiens

<400> 133
gaatacatta aagtaggggc aacccttgag cccagacttc tgccatgtga agaccctttg 60
aaaatcctga caaacacagg tactgcgtaa gtggtcagct aattaaagag gggaggtgga 120
gctgtccttt gtgtatccaa taagtaccca ttatctcatt tgagcatgaa aagaggccac 180
tgttattact ttcaagaagg aaagtaagca ggatagctca tatttttaga accattcctc 240
accaaatgga ataattccgg tgaaaagtgg gagtgaggaa gaaagaaaaa aaaaacttct 300
aatcataatg tttgggaata agaaaggaag aagaaactca cgtcaaagcc gactttctcc 360
tgcagctgta aaataaactc ttaagaccct tcctgctgaa actctggaga ggaaaactgg 420
agtggcgggt gggctttgcc tgcagctcaa ctctccctcg cggcgcgggc gcggctgggt 480
tcagcacctc ggaaagcgcc cctcgcggcg ccccgggatt acgcatgctc cttggggccc 540
gccgccttgg ccgtgcaagt gccaccgtaa ctggtgagag ccgctggcaa cccacccgga 600
gttgacaacc gcggagagac gcagacaccc actgacctcc aggaagctga gcgtggtgga 660
tggaactcta cgatctcttt ctctccaagg acggaaacct catccaagca gtcccagagg 720
aaacggataa aggtatttga aagggagcga gcggccccaa atcgcacaat tgagcggctg 780
ggggagttat gcgccagtgc cccagtgacc gcgggacacg gagaggggaa gtctgcgttg 840
tacataagga cctagggact ccgagcttgg cctgagaacc cttggacgcc gagtgcttgc 900
cttacgggct gcactcctca actctgctcc aaagcagccg ctgagctcaa ctcctgcgtc 960
cagggcgttc gctgcgcgcc aggacgcgct tagtacccag ttcctgggct ctctcttcag 1020
tagctgcttt gaaagctccc acgcacgtcc cgcaggctag cctggcaaca aaactggggt 1080
aaaccgtgtt atcttaggtc ttgtccccca gaacatgacc tagaggtacc tgcgcatgca 1140
gatggccgat gcagccacga tagccaccat gaataaggca gcaggcgggg acaagctagc 1200
agaactcttc agtctggtcc cggaccttct ggaggcggcc aacacgagtg gtaacgcgtc 1260
gctgcagctt ccggacttgt ggtgggagct ggggctggag ttgccggacg gcgcgccgcc 1320
aggacatccc ccgggcagcg gcggggcaga gagcgcggac acagaggccc gggtgcggat 1380
tctcatcagc gtggtgtact gggtggtgtg cgccctgggg ttggcgggca acctgctggt 1440
tctctacctg atgaagagca tgcagggctg gcgcaagtcc tctatcaacc tcttcgtcac 1500
caacctggcg ctgacggact ttcagtttgt gctcaccctg cccttctggg cggtggagaa 1560
cgctcttgac ttcaaatggc ccttcggcaa ggccatgtgt aagatcgtgt ccatggtgac 1620
gtccatgaac atgtacgcca gcgtgttctt cctcactgcc atgagtgtga cgcgctacca 1680
ttcggtggcc tcggctctga agagccaccg gacccgagga cacggccggg gcgactgctg 1740
cggccggagc ctgggggaca gctgctgctt ctcggccaag gcgctgtgtg tgtggatctg 1800

ggctttggcc gcgctggcct cgctgcccag tgccattttc tccaccacgg tcaaggtgat 1860
gggcgaggag ctgtgcctgg tgcgtttccc ggacaagttg ctgggccgcg acaggcagtt 1920
ctggctgggc ctctaccact cgcagaaggt gctgctgggc ttcgtgctgc cgctgggcat 1980
cattatcttg tgctacctgc tgctggtgcg cttcatcgcc gaccgccgcg cggcggggac 2040
caaaggaggg gccgcggtag ccggaggacg cccgaccgga gccagcgccc ggagactgtc 2100
gaaggtcacc aaatcagtga ccatcgttgt cctgtccttc ttcctgtgtt ggctgcccaa 2160
ccaggcgctc accacctgga gcatcctcat caagttcaac gcggtgccct tcagccagga 2220
gtatttcctg tgccaggtat acgcgttccc tgtgagcgtg tgcctagcgc actccaacag 2280
ctgcctcaac cccgtcctct actgcctcgt gcgccgcgag ttccgcaagg cgctcaagag 2340
cctgctgtgg cgcatcgcgt ctccttcgat caccagcatg cgccccttca ccgccactac 2400
caagccggag cacgaggatc aggggctgca ggccccggcg ccgccccacg cggccgcgga 2460
gccggacctg ctctactacc cacctggcgt cgtggtctac agcgggggcc gctacgacct 2520
gctgcccagc agctctgcct actgacgcag gcctcaggcc cagggcgcgc cgtcggggca 2580
aggtggcctt ccccgggcgg taaagaggtg aaaggatgaa ggagggctgg ggggggcccc 2640
atttaagaag taggtgggag gaggatgggc agagcatgga ggaggagcct gtggataggc 2700
cgaggacctt ctctggagag gagatgcttc gaaatcaggt ggagagagga aattggcaaa 2760
gggatagaga cgagccccac gggccagaca gccaacctcc gctccgcacc ccacagcctc 2820
tccttactct tcccacgctg agtagtgtgg gggcgcccag aagcgaagac aagcagcaaa 2880
aatgtagaga aattggcacg gggagcgggg cttagccaaa tgatgcacag acaattgtgc 2940
ccgtttattc cagcgacttc tgcggagagg gcagccgtcg gcacaaacac tcctttgcgt 3000

<210> 134
<211> 2200
<212> DNA
<213> Homo sapiens

<400> 134
gtcccccgat tccctcaccc atcatataac gtgtgtattt attatgtttc ccgtttcctc 60
tgtctccgcc agcagaatgt aaactccatg aggtcaggaa tctccgagtt atgttgcgcc 120
agtgtaatcc aagagcccgg aacagtgcct ggcacacagc gggcatatgg aagaacaaat 180
gtgtgaaggt gtgaatgaat gaataattga aagaataaat agtagttctc agcctcacag 240
aacacgggtc acaacctcaa atgacctgct accctgccca taaataacag agatgcagga 300
gtaagtgctg ggctgtgacc tgtcaacatg ctaagccgct caaacaaaac tgcccaacag 360

cccgctggcc gcctatttgc agcactgggc cctgagccgc acattcccat ttcgttgata 420
aagaaactga ccagatagtt taagtggcct gctgcggaag acagagctgg tgctgcaccg 480
gtcgctgctt ccccagtcct tttttggcct cctttctgac gcgacgcaga ccccagttct 540
ggagagtctg tcactcgctc cccgtggtgg gagatcagag gcctggtgtc cttgggagcg 600
gcgagcggtg ctcggcgcag gatagaaagg gagtgcgcgc ccgagtcccc cagatccctg 660
ggaacccgcg ccaccctccc gcccctgccc atccccggcc gcgctgtcag tctccattag 720
cgctaacagg ctccagacgg agcgggccgg gcgctgggtt aatgcaatcg gcgcgttacc 780
tggggcgcag gctacattac cagcccggcc cccgccaggc acggccagaa ccagtcagcc 840
cgcgccctgc cggccgcccc gcgcctccag ctcttccccg gccccgcccg aacgccacac 900
ggcggagccc agccccagcc cgcgccctag agcctgccaa ggcgccgccg gtcgggggcc 960
ggcagggcgc aaggcaccag ggatcccctc gccgccggac acgtgagtgc gccctgagcg 1020
cgggacaggg ctaggtctgc ctgggaggcc cgggccgaga cgcgccagca gagggctagc 1080
gagtttgtag tgcagtgacg ttaagtgtcc gagaaggctc ctgtggctgt tgaagtgtcg 1140
cggacctgag ctggggaggg ggtcggcacg ctgccctcag cctcggtgag ttcaatccca 1200
gccatttggg gcaggcgaga gtgggtgaac gaggaaaagt gctgcagggt cttcagccgc 1260
ccccagaggg ctgtcagaag tctccaactc ttgagttccg gcgtgcccca acctctgttt 1320
ccaaattttt ccagcggacg cgcgctcttt tctgggaacc ctgcgtccgc tcagcgcgcg 1380
ctcatcccag tgtctaaggc gctcccgggt ggtcttggga gttgcaagta gggaggaacg 1440
gccgggtaac cacctctttt ccctttatcc aagcagagcc tcggcgtgcc cccaggaccg 1500
gtaaagttcc tctcgccagc cgcatccatg cttctggcgc ggatgaaccc gcaggtgcag 1560
cccgagaaca acggggcgga cacgggtcca gagcagcccc ttcgggcgcg caaaactgcg 1620
gagctgctgg tggtgaagga gcgcaacggc gtccagtgcc tgctggcgcc ccgcgacggc 1680
gacgcgcagc cccgggagac ctggggcaag aagatcgact tcctgctgtc cgtagtcggc 1740
ttcgcagtgg acctggccaa cgtgtggcgc ttcccctacc tctgctacaa gaacggcggc 1800
ggtgagcgtg gggtcgggct gggaatttga atctgggagg tccactgtct gcagcggtgg 1860
ctgggacagg agctggaata cacacggaag ggaggcgagg agacaggggc aaatctgggg 1920
cgcagaaaga actggacagg gctaacggga aaaaaaaaag attggagtcc tctggaaggt 1980
cattttccca ggctctttgc agagtacctc gagctcattc cagcggaagt gtcaggattg 2040
ggcaccctgg aagcaaaaca gcagaagagt gaaatcgagt catgacccta aagtcatggt 2100
aggggtatgg atggaaagga cagaatctgg ggtgccaggt tgggtggggg agcctgacct 2160
tttgatggtc tgctggaagg gaggtggaga ttccaagagc 2200

## Description

### TECHNICAL FIELD

The present invention relates to a method of measuring the content of methylated DNA in a DNA region of interest in a genomic DNA contained in a biological specimen, and so on.

### BACKGROUND ART

As a method for evaluating the methylation state of DNA in a target DNA region in a genomic DNA contained in a biological specimen, for example, there is known a method of measuring the content of methylated DNA in a target DNA region in a genomic DNA (see, for example, Nucleic Acids Res., 1994, Aug 11; 22(15): 2990-7, and Proc. Natl. Acad. Sci. U.S.A., 1997, Mar 18; 94(6): 2284-9 for reference). In such a measuring method, first, it is necessary to extract DNA containing the target DNA region from a DNA sample derived from a genomic DNA, and the extracting operation is complicated.

As a method of measuring the content of methylated DNA in a target region of extracted DNA, for example, (1) a method of amplifying a target region by subjecting the DNA to a chain reaction for DNA synthesis by DNA polymerase after modification of the DNA with a sulfite or the like (Polymerase Chain Reaction; hereinafter also referred to as PCR), and (2) a method of amplifying a target region by subjecting the DNA to PCR after digestion of the DNA using a methylation sensitive restriction enzyme are known. Both of these methods require time and labor for DNA modification for detection of methylation, subsequent purification of the product, preparation of a reaction system for PCR, and checking of DNA amplification.

### DISCLOSURE OF THE INVENTION

It is an object of the present invention to provide a method of measuring the content of methylated DNA in a target DNA region (hereinafter, also referred to as a "target region") in a genomic DNA contained in a biological specimen in a simple and convenient manner.

That is, the present invention include the following inventions.

### [Invention 1]

A method of quantifying or detecting methylated DNA in a target DNA region possessed by genomic DNA contained in a biological specimen, comprising:
First step of separating double-stranded DNA derived from genomic DNA comprising the target DNA region contained in the biological specimen into single-stranded DNA;
Second step of mixing the single-stranded DNA, a methylated DNA antibody, and a specific oligonucleotide comprising a nucleotide sequence of a part of a complementary sequence to any of the following nucleotide sequences and not inhibiting binding between the methylated target DNA region in the single-stranded DNA and the methylated DNA antibody, to form a complex of the single-stranded DNA, the methylated DNA antibody, and the specific oligonucleotide; and
Third step of detecting or quantifying the methylated DNA in the target DNA region contained in the biological specimen by conducting detection or quantification depending on an identification function that is possessed by the methylated DNA antibody contained in the complex and available for detection:
   (1) the nucleotide sequence of SEQ ID NO:1 or a nucleotide sequence having 80% or more sequence identity to the sequence,
   (2) the nucleotide sequence of SEQ ID NO:2 or a nucleotide sequence having 80% or more sequence identity to the sequence,
   (3) the nucleotide sequence of SEQ ID NO:3 or a nucleotide sequence having 80% or more sequence identity to the sequence,
   (4) the nucleotide sequence of SEQ ID NO:4 or a nucleotide sequence having 80% or more sequence identity to the sequence,
   (5) the nucleotide sequence of SEQ ID NO:5 or a nucleotide sequence having 80% or more sequence identity to the sequence,
   (6) the nucleotide sequence of SEQ ID NO:6 or a nucleotide sequence having 80% or more sequence identity to the sequence,
   (7) the nucleotide sequence of SEQ ID NO:7 or a nucleotide sequence having 80% or more sequence identity to the sequence,
   (8) the nucleotide sequence of SEQ ID NO:8 or a nucleotide sequence having 80% or more sequence identity to the sequence,
   (9) the nucleotide sequence of SEQ ID NO:9 or a nucleotide sequence having 80% or more sequence identity to the sequence,
   (10) the nucleotide sequence of SEQ ID NO:10 or a nucleotide sequence having 80% or more sequence identity to the sequence,
   (11) the nucleotide sequence of SEQ ID NO:11 or a nucleotide sequence having 80% or more sequence identity to the sequence,
   (12) the nucleotide sequence of SEQ ID NO:12 or a nucleotide sequence having 80% or more sequence identity to the sequence,
   (13) the complementary sequence to the nucleotide sequence of SEQ ID NO:1 or a nucleotide sequence having 80% or more sequence identity to the complementary sequence,
   (11) the complementary sequence to the nucleotide sequence of SEQ ID NO:2 or a nucleotide sequence having 80% or more sequence identity to the complementary sequence,
   (15) the complementary sequence to the nucleotide sequence of SEQ ID NO:3 or a nucleotide sequence having 80% or more sequence identity to the complementary sequence,
   (16) the complementary sequence to the nucleotide sequence of SEQ ID NO:4 or a nucleotide sequence having 80% or more sequence identity to the complementary sequence,
   (17) the complementary sequence to the nucleotide sequence of SEQ ID NO:5 or a nucleotide sequence having 80% or more sequence identity to the complementary sequence,
   (18) the complementary sequence to the nucleotide sequence of SEQ ID NO:6 or a nucleotide sequence having 80% or more sequence identity to the complementary sequence,
   (19) the complementary sequence to the nucleotide sequence of SEQ ID NO:7 or a nucleotide sequence having 80% or more sequence identity to the complementary sequence,
   (20) the complementary sequence to the nucleotide sequence of SEQ ID NO:8 or a nucleotide sequence having 80% or more sequence identity to the complementary sequence,
   (21) the complementary sequence to the nucleotide sequence of SEQ ID NO:9 or a nucleotide sequence having 80% or more sequence identity to the complementary sequence,
   (22) the complementary sequence to the nucleotide sequence of SEQ ID NO:10 or a nucleotide sequence having 80% or more sequence identity to the complementary sequence,
   (23) the complementary sequence to the nucleotide sequence of SEQ ID NO:11 or a nucleotide sequence having 80% or more sequence identity to the complementary sequence, and
   (24) the complementary sequence to the nucleotide sequence of SEQ ID NO:12 or a nucleotide sequence having 80% or more sequence identity to the complementary sequence.

### [Invention 2]

The method according to Invention 1, wherein the specific oligonucleotide comprises any of the following nucleotide sequences:
(1) the nucleotide sequence of SEQ ID NO:13,
(2) the nucleotide sequence of SEQ ID NO:14,
(3) the nucleotide sequence of SEQ ID NO:15,
(4) the nucleotide sequence of SEQ ID NO:16,
(5) the nucleotide sequence of SEQ ID NO:17,
(6) the nucleotide sequence of SEQ ID NO:18,
(7) the nucleotide sequence of SEQ ID NO:19,
(8) the nucleotide sequence of SEQ ID NO:20,
(9) the nucleotide sequence of SEQ ID NO:21,
(10) the nucleotide sequence of SEQ ID NO:22,
(11) the nucleotide sequence of SEQ ID NO:23
(12) the nucleotide sequence of SEQ ID NO:24,
(13) the complementary sequence to the nucleotide sequence of SEQ ID NO:13,
(14) the complementary sequence to the nucleotide sequence of SEQ ID NO:14,
(15) the complementary sequence to the nucleotide sequence of SEQ ID NO:15,
(16) the complementary sequence to the nucleotide sequence of SEQ ID NO:16,
(17) the complementary sequence to the nucleotide sequence of SEQ ID NO:17,
(18) the complementary sequence to the nucleotide sequence of SEQ ID NO:18,
(19) the complementary sequence to the nucleotide sequence of SEQ ID NO:19,
(20) the complementary sequence to the nucleotide sequence of SEQ ID NO:20,
(21) the complementary sequence to the nucleotide sequence of SEQ ID NO:21,
(22) the complementary sequence to the nucleotide sequence of SEQ ID NO:22,
(23) the complementary sequence to the nucleotide sequence of SEQ ID NO:23, and
(24) the complementary sequence to the nucleotide sequence of SEQ ID NO:24.

### [Invention 3]

The method according to Invention 1 or 2, wherein a counter oligonucleotide is added in forming the complex in Second step.

### [Invention 4]

The method according to any one of Inventions 1 to 3, wherein formation of the complex in Second step is conducted in a reaction system containing a divalent cation.

### [Invention 5]

The method according to any one of Inventions 1 to 4, wherein the methylated DNA antibody in the complex formed in Second step has been made to bind to a support before Third step.

### [Invention 6]

The method according to any one of Inventions 1 to 5, further comprising, after First step and before Third step, the step of digesting unmethylated single-stranded DNA with a methylation sensitive restriction enzyme capable of digesting single-stranded DNA.

### [Invention 7]

The method according to any one of Inventions 1 to 6, further comprising, after First step and before Third step, the step of digesting unmethylated single-stranded DNA with a methylation-sensitive restriction enzyme in a reaction system containing a specific masking oligonucleotide comprising a recognition sequence for the methylation sensitive restriction enzyme as a part thereof.

### [Invention 8]

The method according to Invention 6, wherein the methylation sensitive restriction enzyme capable of digesting single-stranded DNA is HhaI.

### [Invention 9]

The method according to any one of Inventions 1 to 8, wherein the methylated DNA antibody is a methylcytosine antibody.

### [Invention 10]

The method according to any one of Inventions 1 to 9, wherein the biological specimen is mammalian blood, serum, plasma, bodily fluid, cell lysate or tissue lysate.

### [Invention 11]

The method according to any one of Inventions 1 to 10, wherein the DNA sample derived from genomic DNA contained in the biological specimen is digested in advance with a restriction enzyme recognition cleaving site for which is not present in the target DNA region of the genomic DNA.

### [Invention 12]

The method according to any one of Inventions 1 to 11, wherein the DNA sample derived from genomic DNA contained in the biological specimen has been digested with a methylation sensitive restriction enzyme.

### [Invention 13]

The method according to any one of Inventions 1 to 12, wherein the DNA sample derived from genomic DNA contained in the biological specimen has been digested with a methylation sensitive restriction enzyme in a reaction system containing a specific masking oligonucleotide.

### [Invention 14]

The method according to any one of Inventions 7, 12 and 13, wherein the methylation sensitive restriction enzyme is HpaII or HhaI.

### [Invention 15]

The method according to any one of Inventions 1 to 14, wherein the DNA sample derived from genomic DNA contained in the biological specimen is a DNA sample purified in advance.

### [Invention 16]

The method according to any one of Inventions 1 to 15, wherein the target DNA region possessed by genomic DNA comprises a recognition cleaving site for the methylation sensitive restriction enzyme.

### [Invention 17]

DNA comprising any of the following sequences:
(1) the nucleotide sequence of SEQ ID NO:13,
(2) the nucleotide sequence of SEQ ID NO:14,
(3) the nucleotide sequence of SEQ ID NO:15,
(4) the nucleotide sequence of SEQ ID NO:16,
(5) the nucleotide sequence of SEQ ID NO:17,
(6) the nucleotide sequence of SEQ ID NO:18,
(7) the nucleotide sequence of SEQ ID NO:19,
(8) the nucleotide sequence of SEQ ID NO:20,
(9) the nucleotide sequence of SEQ ID NO:21,
(10) the nucleotide sequence of SEQ ID NO:22,
(11) the nucleotide sequence of SEQ ID NO:23
(12) the nucleotide sequence of SEQ ID NO:24,
(13) the complementary sequence to the nucleotide sequence of SEQ ID NO:13,
(14) the complementary sequence to the nucleotide sequence of SEQ ID NO:14,
(15) the complementary sequence to the nucleotide sequence of SEQ ID NO:15,
(16) the complementary sequence to the nucleotide sequence of SEQ ID NO:16,
(17) the complementary sequence to the nucleotide sequence of SEQ ID NO:17,
(18) the complementary sequence to the nucleotide sequence of SEQ ID NO:18,
(19) the complementary sequence to the nucleotide sequence of SEQ ID NO:19,
(20) the complementary sequence to the nucleotide sequence of SEQ ID NO:20,
(21) the complementary sequence to the nucleotide sequence of SEQ ID NO:21,
(22) the complementary sequence to the nucleotide sequence of SEQ ID NO:22,
(23) the complementary sequence to the nucleotide sequence of SEQ ID NO:23, and
(24) the complementary sequence to the nucleotide sequence of SEQ ID NO:24.

### BRIEF DESCRIPTION OF THE DRAWINGS

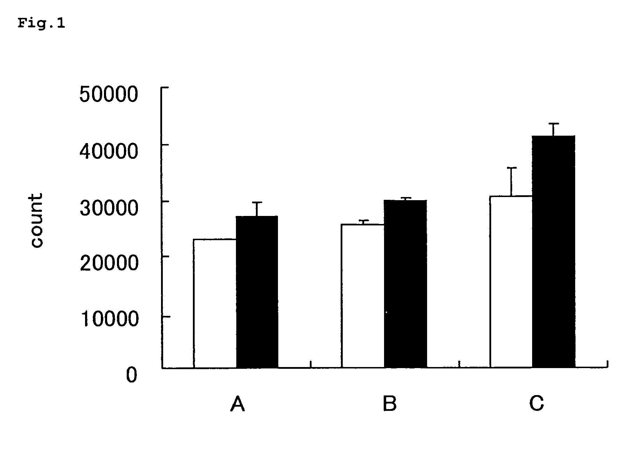

Fig. 1 is a drawing showing a result of Example 1. The open box represents a measurement result of an index value having correlation with methylation frequency using genomic DNA of a noncancerous tissue, and the closed box represents a measurement result of an index value having correlation with methylation frequency using genomic DNA extracted from Capan-2 (a cancer cell strain). In the drawing, from the left side, A represents an amount of the methylcytosine antibody binding to the target region detected by using a specific oligonucleotide solution A, B represents an amount of the methylcytosine antibody binding to the target region detected by using a specific oligonucleotide solution B, and C represents an amount of the methylcytosine antibody binding to the target region detected by using a specific oligonucleotide solution C, respectively measured by fluorescence (612 nm).

### MODE FOR CARRYING OUT THE INVENTION

As the "biological specimen", for example, a cell lysate, a tissue lysate (here the term "tissue" is used in a broad sense including blood and lymph node) or biological samples including bodily fluids such as blood, plasma, serum and lymph, and bodily secretions (urine, milk and so on) and genomic DNA obtained by extracting these biological samples in mammals. Other examples of the biological specimen include samples derived from microorganisms and viruses, and genomic DNA of microorganisms or viruses. When the specimen is human blood, use of the present invention in a health check or a simple clinical examination is expected.

As the DNA sample derived from genomic DNA contained in such a biological specimen, a sample treated with a methylation sensitive restriction enzyme or the like, a sample digested in advance with a restriction enzyme whose recognition cleaving site does not exist in the target DNA region possessed by the genomic DNA, a sample digested with a methylation sensitive restriction enzyme in a reaction system containing a specific masking oligonucleotide, a DNA sample purified in advance, and the like are recited.

For obtaining a genomic DNA from a specimen derived from a mammal, for example, DNA may be extracted using a commercially available DNA extraction kit.

When blood is used as a specimen, plasma or serum is prepared from blood in accordance with a commonly used method, and using the prepared plasma or serum as a specimen, free DNA (including DNA derived from cancer cells such as gastric cancer cells) contained in the specimen is analyzed. This enables analysis of DNA derived from cancer cells such as gastric cancer cells while avoiding DNA derived from hemocytes, and improves the sensitivity of detection of cancer cells such as gastric cancer cells and a tissue containing the same.

Usually, a gene (a genomic DNA) consists of four kinds of bases. In these bases, such a phenomenon is known that only cytosine is methylated, and such methylation modification of DNA is limited to cytosine in a nucleotide sequence represented by 5'-CG-3' (C represents cytosine, and G represents guanine. Hereinafter, the nucleotide sequence is also referred to as "CpG"). The site to be methylated in cytosine is its position 5. In DNA replication prior to cell division, only cytosine in "CpG" of a template chain is methylated immediately after replication, however, cytosine in "CpG" of a newly-generated strand is immediately methylated by the action of methyltransferase. Accordingly, the methylation state of DNA will be passed to new two sets of DNA even after DNA replication. The term "methylated DNA" in the present invention means DNA occurring by such methylation modification.

It is known that methylation abnormality of DNA occurs in various diseases (for example, cancer), and it is supposed that the level of a particular disease can be measured by detecting the DNA methylation abnormality. For example, when the present invention is practiced for the region where methylation occurs at 100% in a specimen derived from a disease, the amount of methylated DNA quantified or detected will be large, whereas when the present invention is practiced for the region where methylation does not occur at 100% in a specimen derived from a disease, the amount of methylated DNA quantified or detected will be close to approximately zero. For example, when there is a region where a methylation rate is low in a specimen of a healthy subject and high in a specimen of a diseased patient, and if the present invention is practiced for the region, the amount of methylated DNA quantified or detected will be a value near zero in a healthy subject, but will be significantly higher than that value in a diseased patient, so that the level of the disease can be determined based on a difference between these values. The term "level of disease" is similar to the meaning that is generally used in the art, and concretely means, for example, malignancy of a cell when the specimen is a cell, or means, for example, abundance or the like of disease cells in a tissue when the specimen is a tissue. Further, when the specimen is plasma or serum, it means the probability that the individual has the disease. Therefore, by examining the methylation abnormality of the specimen by practice of the present invention, it is possible to diagnose a variety of diseases.

The term "target DNA region" means a DNA region for which presence or absence of methylation of cytosine contained in the region is to be examined, and a DNA region containing at least one cytosine in a nucleotide sequence represented by CpG which is present in a nucleotide sequence of a promoter region, an untranslated region, or a translated region (coding region) of a useful protein gene such as Lysyl oxidase, HRAS-like suppressor, bA305P22.2.1, Gamma filamin, HAND1, Homologue of RIKEN 2210016F16, FLJ32130, PPARG angiopoietin-related protein, Thrombomodulin, p53-responsive gene 2, Fibrillin2, Neurofilament3, disintegrin and metalloproteinase domain 23, G protein-coupled receptor 7, G-protein coupled somatostatin and angiotensin-like peptide receptor, and Solute carrier family 6 neurotransmitter transporter noradrenalin member 2 can be recited. In addition to quantifying or detecting methylated DNA in the target DNA region individually, by using more target DNA regions in one detection system, for example, the quantification accuracy and detection sensitivity will improve accordingly.

To be more specific, when the useful protein gene is a Lysyl oxidase gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Lysyl oxidase gene derived from human, and a promoter region located 5' upstream of the same can be recited, and more concretely, the nucleotide sequence of SEQ ID NO:119 (corresponding to a nucleotide sequence represented by base No. 16001 to 18661 in the nucleotide sequence described in Genbank Accession No. AF270645) can be recited. In the nucleotide sequence of SEQ ID NO:119, ATG codon encoding methionine at amino terminal of Lysyl oxidase protein derived from human is represented in base No. 2031 to 2033, and a nucleotide sequence of the above exon 1 is represented in base No. 1957 to 2661. Cytosine in the nucleotide sequence represented by CpG which is present in the nucleotide sequence of SEQ ID NO:119, in particular, cytosine in CpG which is present in a region where CpGs are densely present in the nucleotide sequence of SEQ ID NO:119 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1560, 1574, 1600, 1623, 1635, 1644, 1654, 1661, 1682, 1686, 1696, 1717, 1767, 1774, 1783, 1785, 1787, 1795 and so on in the nucleotide sequence of SEQ ID NO:119 can be recited.

Also to be more specific, when the useful protein gene is a HRAS-like suppressor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HRAS-like suppressor gene derived from human, and a promoter region located 5' upstream of the same can be recited, and more concretely, the nucleotide sequence of SEQ ID NO:120 (corresponding to a nucleotide sequence represented by base No. 172001 to 173953 in the nucleotide sequence described in Genbank Accession No. AC068162) can be recited. In the nucleotide sequence of SEQ ID NO:120, the nucleotide sequence of exon 1 of a HRAS-like suppressor gene derived from human is represented in base No. 1743 to 1953. Cytosine in the nucleotide sequence represented by CpG which is present in the nucleotide sequence of SEQ ID NO:120, in particular, cytosine in CpG which is present in a region where CpGs are densely present in the nucleotide sequence of SEQ ID NO:120 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1316, 1341, 1357, 1359, 1362, 1374, 1390, 1399, 1405, 1409, 1414, 1416, 1422, 1428, 1434, 1449, 1451, 1454, 1463, 1469, 1477, 1479, 1483, 1488, 1492, 1494, 1496, 1498, 1504, 1510, 1513, 1518, 1520 and so on in the nucleotide sequence of SEQ ID NO:120 can be recited.

Also to be more specific, when the useful protein gene is a bA305P22.2.1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a bA305P22.2.1 gene derived from human, and a promoter region located 5' upstream of the same can be recited, and more concretely, the nucleotide sequence of SEQ ID NO:121 (corresponding to a nucleotide sequence represented by base No. 13001 to 13889 in the nucleotide sequence described in Genbank Accession No. AL121673) can be recited. In the nucleotide sequence of SEQ ID NO:121, ATG codon encoding methionine at amino terminal of bA305P22.2.1 protein derived from human is represented in base No. 849 to 851, and a nucleotide sequence of the above exon 1 is represented in base No. 663 to 889. Cytosine in the nucleotide sequence represented by CpG which is present in the nucleotide sequence of SEQ ID NO:121, in particular, cytosine in CpG which is present in a region where CpGs are densely present in the nucleotide sequence of SEQ ID NO:121 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 335, 337, 351, 363, 373, 405, 424, 427, 446, 465, 472, 486 and so on in the nucleotide sequence of SEQ ID NO:121 can be recited.

Also to be more specific, when the useful protein gene is a Gamma filamin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Gamma filamin gene derived from human, and a promoter region located 5' upstream of the same can be recited, and more concretely, the nucleotide sequence of SEQ ID NO:122 (corresponding to a complementary sequence to a nucleotide sequence represented by base No. 63528 to 64390 in the nucleotide sequence described in Genbank Accession No. AC074373) can be recited. In the nucleotide sequence of SEQ ID NO:122, ATG codon encoding methionine at amino terminal of Gamma filamin protein derived from human is represented in base No. 572 to 574, and a nucleotide sequence of the above exon 1 is represented in base No. 463 to 863. Cytosine in the nucleotide sequence represented by CpG which is present in the nucleotide sequence of SEQ ID NO:122, in particular, cytosine in CpG which is present in a region where CpGs are densely present in the nucleotide sequence of SEQ ID NO:122 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 333, 337, 350, 353, 360, 363, 370, 379, 382, 384, 409, 414, 419, 426, 432, 434, 445, 449, 459, 472, 474, 486, 490, 503, 505 and so on in the nucleotide sequence of SEQ ID NO:122 can be recited.

Also to be more specific, when the useful protein gene is a HAND1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HAND1 gene derived from human, and a promoter region located 5' upstream of the same can be recited, and more concretely, the nucleotide sequence of SEQ ID NO:123 (corresponding to a complementary sequence to a nucleotide sequence represented by base No. 24303 to 26500 in the nucleotide sequence described in Genbank Accession No. AC026688) can be recited. In the nucleotide sequence of SEQ ID NO:123, ATG codon encoding methionine at amino terminal of HAND1 protein derived from human is represented in base No. 1656 to 1658, and a nucleotide sequence of the above exon 1 is represented in base No. 1400 to 2198. Cytosine in the nucleotide sequence represented by CpG which is present in the nucleotide sequence of SEQ ID NO:123, in particular, cytosine in CpG which is present in a region where CpGs are densely present in the nucleotide sequence of SEQ ID NO:123 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1153, 1160, 1178, 1187, 1193, 1218, 1232, 1266, 1272, 1292, 1305, 1307, 1316, 1356, 1377, 1399, 1401, 1422, 1434 and so on in the nucleotide sequence of SEQ ID NO:123 can be recited.

Also to be more specific, when the useful protein gene is a Homologue of RIKEN 2210016F16 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Homologue of RIKEN 2210016F16 gene derived from human, and a promoter region located 5' upstream of the same can be recited, and more concretely, the nucleotide sequence of SEQ ID NO:124 (corresponding to a complementary nucleotide sequence to a nucleotide sequence represented by base No. 157056 to 159000 in the nucleotide sequence described in Genbank Accession No. AL354733) can be recited. In the nucleotide sequence of SEQ ID NO:124, a nucleotide sequence of exon 1 of a Homologue of a RIKEN 2210016F16 gene derived from human is represented in base No. 1392 to 1945. Cytosine in the nucleotide sequence represented by CpG which is present in the nucleotide sequence of SEQ ID NO:124, in particular, cytosine in CpG which is present in a region where CpGs are densely present in the nucleotide sequence of SEQ ID NO:124 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1172, 1175, 1180, 1183, 1189, 1204, 1209, 1267, 1271, 1278, 1281, 1313, 1319, 1332, 1334, 1338, 1346, 1352, 1358, 1366, 1378, 1392, 1402, 1433, 1436, 1438 and so on in the nucleotide sequence of SEQ ID NO:124 can be recited.

Also to be more specific, when the useful protein gene is a FLJ32130 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a FLJ32130 gene derived from human, and a promoter region located 5' upstream of the same can be recited, and more concretely, the nucleotide sequence of SEQ ID NO:125 (corresponding to a complementary nucleotide sequence to a nucleotide sequence represented by base No. 1 to 2379 in the nucleotide sequence described in Genbank Accession No. AC002310) can be recited. In the nucleotide sequence of SEQ ID NO:125, ATG codon encoding methionine at amino terminal of FLJ32130 protein derived from human is represented in base No. 2136 to 2138, and a nucleotide sequence assumed to be the above exon 1 is represented in base No. 2136 to 2379. Cytosine in the nucleotide sequence represented by CpG which is present in the nucleotide sequence of SEQ ID NO:125, in particular, cytosine in CpG which is present in a region where CpGs are densely present in the nucleotide sequence of SEQ ID NO:125 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1714, 1716, 1749, 1753, 1762, 1795, 1814, 1894, 1911, 1915, 1925, 1940, 1955, 1968 and so on in the nucleotide sequence of SEQ ID NO:125 can be recited.

Also to be more specific, when the useful protein gene is a PPARG angiopoietin-related protein gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a PPARG angiopoietin-related protein gene derived from human, and a promoter region located 5' upstream of the same can be recited, and more concretely, the nucleotide sequence of SEQ ID NO:126 can be recited. In the nucleotide sequence of SEQ ID NO:126, ATG codon encoding methionine at amino terminal of PPARG angiopoietin-related protein derived from human is represented in base No. 717 to 719, and a nucleotide sequence of the 5' side part of the above exon 1 is represented in base No. 1957 to 2661. Cytosine in the nucleotide sequence represented by CpG which is present in the nucleotide sequence of SEQ ID NO:126, in particular, cytosine in CpG which is present in a region where CpGs are densely present in the nucleotide sequence of SEQ ID NO:126 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 35, 43, 51, 54, 75, 85, 107, 127, 129, 143, 184, 194, 223, 227, 236, 251, 258 and so on in the nucleotide sequence of SEQ ID NO:126 can be recited.

Also to be more specific, when the useful protein gene is a Thrombomodulin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Thrombomodulin gene derived from human, and a promoter region located 5' upstream of the same can be recited, and more concretely, the nucleotide sequence of SEQ ID NO:127 (corresponding to a nucleotide sequence represented by base No. 1 to 6096 in the nucleotide sequence described in Genbank Accession No. AF495471) can be recited. In the nucleotide sequence of SEQ ID NO:127, ATG codon encoding methionine at amino terminal of Thrombomodulin protein derived from human is represented in base No. 2590 to 2592, and a nucleotide sequence of the above exon 1 is represented in base No. 2048 to 6096. Cytosine in the nucleotide sequence represented by CpG which is present in the nucleotide sequence of SEQ ID NO:127, in particular, cytosine in CpG which is present in a region where CpGs are densely present in the nucleotide sequence of SEQ ID NO:127 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1551, 1571, 1579, 1581, 1585, 1595, 1598, 1601, 1621, 1632, 1638, 1645, 1648, 1665, 1667, 1680, 1698, 1710, 1724, 1726, 1756 and so on in the nucleotide sequence of SEQ ID NO:127 can be recited.

Also to be more specific, when the useful protein gene is a p53-responsive gene 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a p53-responsive gene 2 gene derived from human, and a promoter region located 5' upstream of the same can be recited, and more concretely, the nucleotide sequence of SEQ ID NO:128 (corresponding to a complementary sequence to a nucleotide sequence represented by base No. 113501 to 116000 in the nucleotide sequence described in Genbank Accession No. AC009471) can be recited. In the nucleotide sequence of SEQ ID NO:128, a nucleotide sequence of exon 1 of a p53-responsive gene 2 gene derived from human is represented in base No. 1558 to 1808. Cytosine in the nucleotide sequence represented by CpG which is present in the nucleotide sequence of SEQ ID NO:128 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1282, 1284, 1301, 1308, 1315, 1319, 1349, 1351, 1357, 1361, 1365, 1378, 1383 and so on in the nucleotide sequence of SEQ ID NO:128 can be recited.

Also to be more specific, when the useful protein gene is a Fibrillin2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Fibrillin2 gene derived from human, and a promoter region located 5' upstream of the same can be recited, and more concretely, the nucleotide sequence of SEQ ID NO:129 (corresponding to a complementary sequence to a nucleotide sequence represented by base No. 118801 to 121000 in the nucleotide sequence described in Genbank Accession No. AC113387) can be recited. In the nucleotide sequence of SEQ ID NO:129, a nucleotide sequence of exon 1 of a Fibrillin2 gene derived from human is represented in base No. 1091 to 1345. Cytosine in the nucleotide sequence represented by CpG which is present in the nucleotide sequence of SEQ ID NO:129 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 679, 687, 690, 699, 746, 773, 777, 783, 795, 799, 812, 823, 830, 834, 843 and so on in the nucleotide sequence of SEQ ID NO:129 can be recited.

Also to be more specific, when the useful protein gene is a Neurofilament3 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Neurofilament3 gene derived from human, and a promoter region located 5' upstream of the same can be recited, and more concretely, the nucleotide sequence of SEQ ID NO:130 (corresponding to a complementary sequence to a nucleotide sequence represented by base No. 28001 to 30000 in the nucleotide sequence described in Genbank Accession No. AF106564) can be recited. In the nucleotide sequence of SEQ ID NO:130, a nucleotide sequence of exon 1 of a Neurofilament3 gene derived from human is represented in base No. 614 to 1694. Cytosine in the nucleotide sequence represented by CpG which is present in the nucleotide sequence of SEQ ID NO:130 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 428, 432, 443, 451, 471, 475, 482, 491, 499, 503, 506, 514, 519, 532, 541, 544, 546, 563, 566, 572, 580 and so on in the nucleotide sequence of SEQ ID NO:130 can be recited.

Also to be more specific, when the useful protein gene is a disintegrin and metalloproteinase domain 23 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a disintegrin and metalloproteinase domain 23 gene derived from human, and a promoter region located 5' upstream of the same can be recited, and more concretely, the nucleotide sequence of SEQ ID NO:131 (corresponding to a nucleotide sequence represented by base No. 21001 to 23300 in the nucleotide sequence described in Genbank Accession No. AC009225) can be recited. In the nucleotide sequence of SEQ ID NO:131, a nucleotide sequence of exon 1 of a disintegrin and metalloproteinase domain 23 gene derived from human is represented in base No. 1194 to 1630. Cytosine in the nucleotide sequence represented by CpG which is present in the nucleotide sequence of SEQ ID NO:131 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 998, 1003, 1007, 1011, 1016, 1018, 1020, 1026, 1028, 1031, 1035, 1041, 1043, 1045, 1051, 1053, 1056, 1060, 1066, 1068, 1070, 1073, 1093, 1096, 1106, 1112, 1120, 1124, 1126 and so on in the nucleotide sequence of SEQ ID NO:131 can be recited.

Also to be more specific, when the useful protein gene is a G protein-coupled receptor 7 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G protein-coupled receptor 7 gene derived from human, and a promoter region located 5' upstream of the same can be recited, and more concretely, the nucleotide sequence of SEQ ID NO:132 (corresponding to a nucleotide sequence represented by base No. 75001 to 78000 in the nucleotide sequence described in Genbank Accession No. AC009800) can be recited. In the nucleotide sequence of SEQ ID NO:132, a nucleotide sequence of exon 1 of a G protein-coupled receptor 7 gene derived from human is represented in base No. 1666 to 2652. Cytosine in the nucleotide sequence represented by CpG which is present in the nucleotide sequence of SEQ ID NO:132 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1480, 1482, 1485, 1496, 1513, 1526, 1542, 1560, 1564, 1568, 1570, 1580, 1590, 1603, 1613, 1620 and so on in the nucleotide sequence of SEQ ID NO:132 can be recited.

Also to be more specific, when the useful protein gene is a G-protein coupled somatostatin and angiotensin-like peptide receptor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G-protein coupled somatostatin and angiotensin-like peptide receptor gene derived from human, and a promoter region located 5' upstream of the same can be recited, and more concretely, the nucleotide sequence of SEQ ID NO:133 (corresponding to a complementary sequence to a nucleotide sequence represented by base No. 57001 to 60000 in the nucleotide sequence described in Genbank Accession No. AC008971) can be recited. In the nucleotide sequence of SEQ ID NO:133, a nucleotide sequence of exon 1 of a G-protein coupled somatostatin and angiotensin-like peptide receptor gene derived from human is represented in base No. 776 to 2632. Cytosine in the nucleotide sequence represented by CpG which is present in the nucleotide sequence of SEQ ID NO:133 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 470, 472, 490, 497, 504, 506, 509, 514, 522, 540, 543, 552, 566, 582, 597, 610, 612 and so on in the nucleotide sequence of SEQ ID NO:133 can be recited.

Also to be more specific, when the useful protein gene is a Solute carrier family 6 neurotransmitter transporter noradrenalin member 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Solute carrier family 6 neurotransmitter transporter noradrenalin member 2 gene derived from human, and a promoter region located 5' upstream of the same can be recited, and more concretely, the nucleotide sequence of SEQ ID NO:134 (corresponding to a complementary sequence to a nucleotide sequence represented by base No. 78801 to 81000 in the nucleotide sequence described in Genbank Accession No. AC026802) can be recited. In the nucleotide sequence of SEQ ID NO:134, a nucleotide sequence of exon 1 of a Solute carrier family 6 neurotransmitter transporter noradrenalin member 2 gene derived from human is represented in base No. 1479 to 1804. Cytosine in the nucleotide sequence represented by CpG which is present in the nucleotide sequence of SEQ ID NO:134 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1002, 1010, 1019, 1021, 1051, 1056, 1061, 1063, 1080, 1099, 1110, 1139, 1141, 1164, 1169, 1184 and so on in the nucleotide sequence of SEQ ID NO:134 can be recited.

As concrete examples of the "target DNA region" in genomic DNA, the following nucleotide sequences of SEQ ID NOs:1 to 12, and complementary sequences thereof can be recited, and also nucleotide sequences having sequence identity of 80% or more, 90% or more, 95% or more or 98% or more to these nucleotide sequences can be recited.
SEQ ID NO:1 corresponds to the nucleotide sequence represented by base No.21965410 to 21964273 in the nucleotide sequence described in Genbank Accession No.NT_008413.17.
SEQ ID NO:2 corresponds to the nucleotide sequence represented by base No.29857545 to 29858420 in the nucleotide sequence described in Genbank Accession No.NT_024524.13.
SEQ ID NO:3 corresponds to the nucleotide sequence represented by base No.7188979 to 7189574 in the nucleotide sequence described in Genbank Accession No.NT_010718.15.
SEQ ID NO:4 corresponds to the nucleotide sequence represented by base No.7534656 to 7535486 in the nucleotide sequence described in Genbank Accession No.NW_926584.1.
SEQ ID NO:5 corresponds to the nucleotide sequence represented by base No.21984168 to 21984790 in the nucleotide sequence described in Genbank Accession No.NT_008413.17.
SEQ ID NO:6 corresponds to the nucleotide sequence represented by base No.21818162 to 21818836 in the nucleotide sequence described in Genbank Accession No.NW_924062.1.
SEQ ID NO:7 corresponds to the nucleotide sequence represented by base No.21998877 to 21999061 in the nucleotide sequence described in Genbank Accession No.NT_008413.17.
SEQ ID NO:8 corresponds to the nucleotide sequence represented by base No.19277002 to 19277451 in the nucleotide sequence described in Genbank Accession No.NT_023935.17.
SEQ ID NO:9 corresponds to the nucleotide sequence represented by base No.19064265 to 19065579 in the nucleotide sequence described in Genbank Accession No.NW_924484.1.
SEQ ID NO:10 corresponds to the nucleotide sequence represented by base No.50318280 to 50318573 in the nucleotide sequence described in Genbank Accession No.NT_022517.17.
SEQ ID NO:11 corresponds to the nucleotide sequence represented by base No.61176814 to 61177405 in the nucleotide sequence described in Genbank Accession No.NT_022517.17.
SEQ ID NO:12 corresponds to the nucleotide sequence represented by base No.14488398 to 14489126 in the nucleotide sequence described in Genbank Accession No.NT_034772.5.

As the "target DNA region" (hereinafter, also referred to as a "target region"), a DNA region containing at least one cytosine in a nucleotide sequence represented by CpG which is present in a nucleotide sequence of a promoter region, an untranslated region, or a translated region (coding region) of a gene such as MLH1, RUNX3, CDH1, TIMP3, CSPG, RARβ, 14-3-3o, CALCA, HIC1, ESR1, PTEN, SOCS1, BLT1, ESR2, MTMG, TWIST, INK4, CDKN2, GSTP, DCR2, TP73, PGR, HIC2, MTHFR, TFF1, MLLT7, SLC5A8, THBS1, SOCS2, ACTB, CDH13, FGF18, GSTM3, HSD17B4, HSPA2, PPP1R13B, PTGS2, SYK, TERT, TITF1, BRACA1, AATF, ABCB1, ABCC1, ABI1, ABL1, AF1Q, AF3P21, AF4, AF9, AFF3, AKAP12, AKAP9, ALEX3, ALK, ALOX15, APAF1, APC, ARHA, ARHGAP26, ARHGEF12, ARNT, ATBF1, ATF1, ATM, AXIN2, BCAS3, BCAS4, BCL1, BCL10, BCL11A, BCL11B, BCL2, BCL5, BCL7A, BCR, BIRC3, BMPR1A, BRCA2, BRD4, BRIP1, BTG1, BUB1B, CAGE-1, CARS, CASC5, CCDC6, CCND2, CCND3, CDH11, CDKN1B, CDKN2A, CDX2, CEP110, CKN1, CLP1, CLTC, CLTCL1, CNC, COL1A1, COX6C, CREBBP, CXXC6, DAB2IP, DDIT3, DDX43, DIRC1, DIRC2, DKK1, E2F3, EEN, EGFR, ELL, EPS15, ERBB2, ERC1, ERCC1, ERCC4, ERG, ETV1, ETV6, EVI1, EWSR1, EXT1, EXT2, FANCA, FANCD2, FANCF, FAS, FBP17, FCRL4, FEV, FGFR1, FHIT, FLI1, FOXO3A, FUS, FVT1, GAS7, GLI1, GNAS, GOLGA5, GOPC, GRB2, HCMOGT-1, HISTIH4I, HLF, HMGA2, HOXA13, HOXC11, HOXC13, HOXD13, HSPBAP1, HSPCB, HSPD1, HSPH1, IKZF2, INTS6, IRF4, JAG1, JAG2, JAK2, JARID1A, JAZF1, JMJD2C, JUN, KIT, KITLG, KLF5, KLF6, LASP1, LDB1, LHFP, LMO1, LMO2, LPHN2, LPP, LYL1, MADH4, MAF, MAFB, MDM2, MDS2, MET, MKL1, MLF1, MLH1, MLL, MLLT10, MMP2, MN1, MRE11B, MSF, MSH2, MSH6, MSI2, MUC1, MUTYH, MXI1, MYC, MYH9, MYST3, NF1, NFKB1, NIN, NKX2-5, NONO, NOTCH1, N-RAS, NTRK3, NUMA1, NUP214, NUP98, OLIG2, P53, PALB2, PAX2, PAX5, PAX7, PAX9, PBX1, PCM1, PCSK7, PDGFB, PDGFRA, PDGFRB, PHOX2B, PICALM, PLAG1, PLCB1, PLK, PML, PMS1, POLH, POU5F1P1, POU6F2, PPP1R13L, PRDM16, PRRX1, PSIP1, PTCH, RABEP1, RAD51L1, RAD53, RANBP17, RAP1GDS1, RAP2B, RARA, RASSF1, RB1, RBL2, RBM15, RBM5, RCHY1, RECQL, RECQL5, RET, RUNX, RUNX1T1, SBDS, SDHC, SDHD, SET, SHH, SIL, SIX1, SNAI2, SPI1, SPINK7, STARD3, STAT3, STK11, STK4, SUFU, SYK, SYNPO2, TBX2, TCF3, THBS2, THRAP3, TMPRSS2, TNF, TOP1, TPM4, TPR, TRIM24, TRIM33, TRIP11, TSC1, TSC2, TSHR, TTL, VAV1, VHL, WFDC1, WT1, WWOX, XPC, ZBTB16, ZNF146 and ZNF217 can be recited. In addition to quantifying or detecting methylated DNA in the target DNA region individually, by using more target DNA regions in one detection system, for example, the quantification accuracy and detection sensitivity will improve accordingly.

The "target DNA region" may be a repetitive sequence interspersed in a genome. In particular, a methylation degree of a repetitive sequence that is apparently methylated by affection can be utilized as a surrogate marker of the disease. For example, in cancer, methylation of SINE and LINE is known, and concretely, subfamilies such as AluJb, AluJo, AluSc, AluSg, AluSg/x, AluSp, AluSq, AluSq/x, AluSx, AluY, FRAM, FLAM_A, MIR, MIRb, MIR3, L1Med, L1M4, L1M5, L1MA3, L1MA7, L1MA9, L1MC, L1MC2, L1MC4, L1MC4a, L1MC5, LIMDa, L1MD1, L1MD2, L1MEe, L1ME3A, L1PA15, L1PREC2, L2, L3, and HAL1 are known.

Repetitive sequences in a genome are generally classified into simple repetitive sequences (called a tandem repetitive sequence, or a tandem repeat) and interspersed repetitive sequences.

Simple repetitive sequences are characterized in that the same sequences neighbor in the same orientation, and for example, broadly classified into the following five kinds: (1) repetitive sequences in which a relatively short sequence such as CCA is repeated, (2) repetitive sequences derived from a transcription factor, (3) a series of repetitive sequences such as centromere, telomere, kinetochore, and ribosome group genes, (4) inert processed pseudogenes reversely transferred from RNA or protein, and (5) gene sequences amplified by gene duplication.

First, as a repeat composed of a relatively short nucleotide sequence, sequences such as (A)n, (T)n, (GA)n, (CA)n, (TAA)n, (GGA)n, (CAGC)n, (CATA)n, (GAAA)n, (TATG)n, (TTTG)n, (TTTA)n, (TTTC)n, (TAAA)n, (TTCA)n, (TATAA)n, (TCTCC)n, (TTTCC)n, (TTTAA)n, (TTTTC)n, (TTTTA)n, (TTTTG)n, (CAAAA)n, (CACCC)n, (TATATG)n, (CATATA)n, (TCTCTG)n, (AGGGGG)n, (CCCCCA)n, and (TGGGGG)n (n means the number of repetition) are known. Next, as the sequence derived from a transcription factor, MER1-Charlie and Zaphod of hAT group, and MER2-Tigger, Tc-1 and Mariner of Tc-1 group are appropriate. As other repetitive sequences derived from a transcription factor on a genome, Tigger1, Tigger2a, Tigger5, Charlie4a, Charlie7 and the like are known. Since these repetitive sequences formed of a short nucleotide sequence, or repetitive sequences derived from a transcription factor are generally short and simple nucleotide sequences, setting of a specific oligonucleotide of the present method is difficult, however, if a practicable specific oligonucleotide can be set, these are not necessarily excluded as an object of the present method. Here, satellite DNA, minisatellite, microsatellite and the like are repetitive sequences that are classified into simple repetitive sequences.

As to a sequence that exists in multicopy in genes, if a specific oligonucleotide of the present method can be set for genes existing in multicopy in a genome as a result of gene duplication, as well as ALR6 as a sequence existing in centromere, U2 and U6 as snRNAs, and other genes that are known to exist in multicopy in a genome such as tRNA and rRNA, the detection sensitivity can be improved compared to detecting one gene in one genome.

It is also known that a retrovirus, a retrotransposon having LTR (Long terminal repeat) in its terminal, an endogenous sequence such as MaLRs (Mammalian apparent LTR-Retrotransposons) considered to be derived from viruses, and LTR derived from a retrovirus exist in multicopy in one genome.

For example, as the LTR derived from a retrovirus, concretely, subfamilies such as LTR1, LTR1B, LTR5, LTR7, LTR8, LTR16A1, LTR16A1, LTR16C, LTR26, LTR26E, MER48, and MLT2CB are known. The LTRs derived from a retrotransposon are classified into classes of ERV, ERVK and ERVL, and concrete examples include subfamilies such as LTR8A, LTR28, MER21B, MER83, MER31B, MER49, MER66B, HERVH, ERVL, LTR16A1, LTR33A, LTR50, LTR52, MLT2A1, MLT2E, MER11C, and MER11C. Further, MaLRs indicate DNA factors including LTRs in both ends likewise a typical retrotransposon, wherein an internal sequence sandwiched between LTRs is not derived from a retrovirus. For example, subfamilies such as MLT1A1, MLT1A2, MLT1B, MLT1C, MLT1D, MLT1F, MLT1G, MLT1H, MLT1J, MLT1K, MLT1I, MLT2CB, MSTA, MSTA-int, MSTB, THE1A, THE1B, THE1B-internal, and THE1 can be recited.

The interspersed repetitive sequences are characterized by being interspersed without neighboring each other, and are considered to be derived from a retrotransposon. Further, the interspersed repetitive sequences are classified into SINE (Short Interspersed Repetitive Element: short chain interspersed repetitive sequences) and LINE (Long Interspersed Elements: long-chain interspersed repetitive sequences) according to the length. Most of SINEs are sequences belonging to the Alu family. A common feature of the Alu family is that it has a sequence of 3'-side or a sequence of 5'-side of 7SL RNA, and that it has an AT-Rich region sandwiched between a Left-monomer and a Right-monomer. As subfamilies of the Alu family, Alu, AluJb, AluJo, AluSc, AluSg, AluSp, AluSq, AluSx, AluY, and FAM (Fossil Alu Monomer), FLAM (Free Left Alu Monomer) having a sequence of FAM, and FRAM (Free Right Alu Monomer) can be recited. As SINEs other than the Alu family, MIR, and Ther/MIR3 are known, and MIR and MIR3 are known as respective subfamilies. As subfamilies of the Alu family including other biological species than human being, B1, B2, B4, PB1, PB1D and so on are known. As LINES, subfamilies of LINE1 to Line23 are reported, and it is known that subfamilies such as LINE-1, LINE2, and LINE3 broadly exist in a genome. As for LINE-1, for example, L1M1, L1M2, L1M3, L1M3d, L1M4, L1M4c, L1MA2, L1MA7, L1MA8, L1MA9, L1MB1, L1MB1, L1MB3, L1MB4, L1MB5, L1MB6, L1MB7, LIMCa, L1MCb, L1MC2, L1MC3, L1MC4, L1MC4a, L1MC5, LIMDa, L1ME, L1MEc, LIMEd, LIMEg, L1ME1, L1ME2, L1ME3, L1ME3A, L1ME3B, L1ME4a, L1PB3, L1P4, L1PA2, L1PA3, L1PA4, L1PA5, L1PA6, L1PA7, L1PA10, L1PA12, L1PA13, L1PA14, L1PA16, L1PB1, L1PB3, L1PB4, L1PREC2, and HAL1 are known, and as LINE-2, subfamilies such as L2 and L2c are known.

Among these repetitive sequences interspersed in a genome, the one that will be methylated by affection can be utilized as a highly sensitive detection system as a surrogate marker of the disease by the present method.

The term quantification in "quantify or detect DNA" is estimation of methylation frequency or an index value correlated therewith in the target DNA region in the specimen, from the quantified methylated DNA antibody, and means methylation frequency or an index value correlated therewith of DNA of the target region contained in 1 mL of serum when the specimen is 1 mL of serum.

The term "detect" in "quantify or detect DNA" means that whether methylated DNA exists in the target DNA region can be distinguished from whether the methylated DNA antibody is detected or not, and represents that methylated DNA exists in the target DNA region in the specimen when the methylated DNA antibody is detected, and represents that the abundance of methylated DNA in the target DNA region in the specimen is less than a detection limit when the methylated DNA antibody or the specific oligonucleotide is not detected.

In First step, double-stranded DNA derived from genomic DNA that comprises the target DNA region and is contained in the biological specimen is temporarily separated into a single-stranded state. Concretely, for example, by adding an annealing buffer to the double-stranded DNA, a mixture is obtained. Next, the obtained mixture is boiled at 95°C for about 30 seconds, and then rapidly cooled on ice chilled water for several minutes. For example, free DNA contained in blood or the like can be single-stranded DNA. Therefore, when genomic DNA contained in the biological specimen is single-stranded DNA, this operation is not required.

The term "methylated DNA antibody" in Second step means an antibody that binds with a methylated base in DNA as an antigen. More desirably, it is an antibody that has a property of binding by recognition of cytosine whose 5-position is methylated in single-stranded DNA, and more concretely it may be the methylcytosine antibody. Also a commercially available methylated DNA antibody capable of specifically recognizing DNA in a methylation state described in the present invention and specifically binding thereto is applicable. The methylated DNA antibody may be produced by a usual method from a methylated base, methylated DNA or the like as an antigen. Concretely, it may be produced by selection according to the specific binding to methylcytosine in DNA as an index, from antibodies generated by using 5-methylcytidine, 5-methylcytosine, or DNA and the like containing 5-methylcytosine as an antigen. As antibodies that can be obtained by immunizing an animal with an antigen, a method using an antibody of IgG fraction (polyclonal antibody) following immunization with a purified antigen and a method using an antibody producing a single clone (monoclonal antibody) are known. In the present invention, since an antibody capable of specifically recognizing methylated DNA or methylcytosine is desired, use of a monoclonal antibody is desired.

As a method of preparing a monoclonal antibody, a procedure based on a cell fusion method can be recited. For example, in the cell fusion method, a hybridoma is prepared by allowing cell fusion between a spleen cell (B cell) derived from an immunized mouse and a myeloma cell, and an antibody produced by the hybridoma is selected for preparation of a methyl cytosine antibody (monoclonal antibody). When a monoclonal antibody is prepared by a cell fusion method, it is not necessary to purify an antigen, and for example, a mixture of 5-methyl cytidine, 5-methyl cytosine or DNA or the like containing 5-methyl cytosine may be administered as an antigen to an animal used for immunization. As an administration method, 5-methyl cytidine, 5-methyl cytosine or DNA or the like containing 5-methyl cytosine is directly administered to a mouse for production of an antibody. When an antibody is difficult to be produced, an antigen bound to a support may be used for immunization. Also, by thoroughly mixing an adjuvant solution (prepared, for example, by mixing liquid paraffin and Aracel A, and mixing killed tubercle bacilli as an adjuvant) and an antigen, and immunizing via liposome incorporating the same, immunity of an antigen can be improved. Also a method involving adding equivalent amounts of a solution containing an antigen and an adjuvant solution, fully emulsifying them, and subcutaneously or intraperitoneally injecting the resultant mixture to a mouse, and a method of adding killed Bordetella pertussis as an adjuvant after mixing well with alum water are known. A mouse may be boosted intraperitoneally or intravenously after an appropriate term from initial immunization. When the amount of an antigen is small, a solution in which the antigen is suspended may be directly injected into a mouse spleen to effect immunization.

After exenterating a spleen and peeling an adipose tissue off after several days from the final immunization, a spleen cell suspension is prepared. The spleen cell is fused, for example, with an HGPRT-deficient myeloma cell to prepare a hybridoma. As a cell fusion agent, any means capable of efficiently fusing a spleen cell (B cell) and a myeloma cell is applicable, and for example, a method of using a hemagglutinating virus of Japan (HVJ), polyethyleneglycol (PEG) and the like are recited. Cell fusion may be conducted by a method using a high voltage pulse.

After the cell fusion operation, cells are cultured in an HAT medium, a clone of a hybridoma in which a spleen cell and a myeloma cell are fused is selected, and the cell is allowed to grow until screening becomes possible. In a method of detecting an antibody for selecting a hybridoma that produces an intended antibody, or a method of measuring a titer of an antibody, an antigen-antibody reaction system may be used. Concretely, as a method of measuring an antibody against a soluble antigen, a radioisotope immune assay (RIA), an enzyme-linked immunosorbent assay (ELISA) and the like can be recited.

Considering the property of the methylated DNA antibody (one antibody binds to one methylated base (cytosine)), it is desired to select a region where a number of methylated bases (cytosine), namely CpG are present as a target DNA region, and thus an improvement in quantification accuracy and detection sensitivity is expected.

The term "specific oligonucleotide" means an oligonucleotide comprising a nucleotide sequence capable of base-pairing with single-stranded DNA containing the target DNA region, and not inhibiting binding of the methylated DNA antibody to a methylated base (cytosine) in single-stranded DNA containing the target DNA region in base-pairing with single-stranded DNA containing the target DNA region. In other words, it is an oligonucleotide that will not inhibit binding between the methylated base in the target DNA region in single-stranded DNA and the methylated DNA antibody. More concretely, it is, for example, an oligonucleotide that will not inhibit binding between methylcytosine in the target DNA region and the methylcytosine antibody, and is capable of binding with single-stranded DNA containing the target DNA region by complementation.

The wording "nucleotide sequence capable of binding with single-stranded DNA containing the target DNA region" means a nucleotide sequence required for forming a bound body (double strand) with single-stranded DNA containing the target DNA region, namely, a nucleotide sequence containing a nucleotide sequence complementary to a part of the nucleotide sequence of the target DNA region, or a nucleotide sequence containing a nucleotide sequence complementary to a part of a.nucleotide sequence of a DNA region on a further 5'-end side than 5'-end of the target DNA region, or a nucleotide sequence containing a nucleotide sequence complementary to a part of a nucleotide sequence of a DNA region on a further 3'-end side than 3'-end of the target DNA region. The wording "not inhibiting binding of the methylated DNA antibody to a methylated base (cytosine) in the single-stranded DNA containing the target DNA region" means that the nucleotide sequence of the specific oligonucleotide is such a nucleotide sequence that complementary binding between the specific oligonucleotide and the single-stranded DNA will not occur in an occupied space required for binding of the methylated DNA antibody with methylated single-stranded DNA. That is, it is supposed that for the methylated DNA antibody to bind with the methylated base (cytosine), not only the directly binding methylated base (cytosine), but also a peripheral space where the methylated base (cytosine) exists is also occupied. Therefore, the specific oligonucleotide should be the one that fails to complementarily bind with the single-stranded DNA in the occupied space required for the methylated DNA antibody in binding with the methylated DNA.

The present invention enables simultaneous detection of at least one target DNA region using at least one specific oligonucleotide. That is, the specific oligonucleotide to be bound to the single-stranded DNA is not necessarily one kind, but two or more kinds may be used unless binding of the methylated DNA antibody is inhibited. By using plural kinds of specific oligonucleotides, it is possible to improve the quantification accuracy and detection accuracy. Further, by detecting plural target regions simultaneously, it is possible to increase the amount of methylated DNA to be measured.

As concrete examples of the "specific oligonucleotide", a nucleotide sequence of any of SEQ ID NOs: 13 to 24, and complementary sequences thereof can be recited, or nucleotide sequences having a sequence identity of 80% or more, 90% or more, 95% or more, or 98% or more with such nucleotide sequences are also recited.

In Second step, as a preferred form in forming the complex of the single-stranded DNA, the methylated DNA antibody, and the specific oligonucleotide, formation in a reaction system containing a divalent cation is recited. More preferably, the divalent cation is a magnesium ion. Here, the "reaction system containing a divalent cation" means such a reaction system that a divalent cation is contained in an annealing buffer used for binding between the single-stranded DNA containing the target DNA region and the specific oligonucleotide, and concretely, for example, a salt composed of a magnesium ion (for example, MgOAc₂, MgCl₂ or the like) may be contained in a concentration of 1 mM to 600 mM.

Besides this, in Second step, as a preferred form in forming the complex of the single-stranded DNA, the methylated DNA antibody and the specific oligonucleotide, a counter oligonucleotide may be added. The counter oligonucleotide is obtained by dividing the same nucleotide sequence as the target DNA region into short oligonucleotides. It may be designed to have usually 10 to 100 bases, and more preferably 20 to 50 bases. The counter oligonucleotide is not designed on the nucleotide sequence where the specific oligonucleotide and the target region bind. The counter oligonucleotide is added in large excess with respect to genomic DNA, and is added for preventing a complementary strand (minus strand) of the target DNA region and a single strand (plus strand) of the target DNA region from rebinding by complementation in causing binding with the specific oligonucleotide (minus strand) after making the target DNA region into the single strand (plus strand). This is because in causing binding of the methylated DNA antibody with the target DNA region, and measuring methylation frequency of DNA and an index value correlated therewith, the target region in a single strand state is more likely to bind with the methylated DNA antibody. The counter oligonucleotide is preferably added in an amount of at least 10 times, and usually 100 times or more compared to the target DNA region.

The wording "formation of a complex" means a mixture in such a condition that the single-stranded DNA containing the methylated target DNA region, the specific oligonucleotide, and the methylated DNA antibody bind. In the present invention, it is preferred to immobilize the complex to a support by binding the methylated DNA antibody or the specific oligonucleotide in the complex with the support before Third step to be described later.

The material and form of the support are not particularly limited as far as a complex can bind thereto. For example, any form suited for use purpose may be employed, including the forms of tube, test plate, filter, disc, bead and so on. As the material, those used as supports for a usual immune measuring method, for example, synthetic resins such as polystyrene, polypropylene, polyacrylamide, polymethylmethacrylate, polysulfone, polyacrylonitrile and nylon, or the synthetic resins incorporating a reactive functional group such as a sulfonic group, or an amino group can be recited. Also, glass, polysaccharides or derivatives thereof (cellulose, nitrocellulose and so on), silica gel, porous ceramics, metal oxides and the like may be used.

As a method of immobilizing the complex to the support by binding, a method of immobilizing the specific oligonucleotide to the support is recited. For binding the single-stranded DNA containing the methylated target DNA region to the support via the specific oligonucleotide, the methylated DNA antibody is quantified or detected by its identification function (to be described later).

For allowing the specific oligonucleotide to be immobilized to the support, the specific oligonucleotide may be immobilized to the support eventually in the condition that the single-stranded DNA containing the methylated target DNA region and the methylated DNA antibody form a complex, and
(1) the specific oligonucleotide may be immobilized to the support in the stage before binding of the single-stranded DNA and the specific oligonucleotide, or
(2) the specific oligonucleotide may be immobilized to the support in the stage after binding of the single-stranded DNA and the specific oligonucleotide.

For immobilizing a specific oligonucleotide to a support, concretely a method of immobilizing a biotinylated oligonucleotide obtained by biotinylating 5'-end or 3'-end of the specific oligonucleotide to a support coated with streptavidin (for example, a PCR tube coated with streptavidin, magnetic beads coated with streptavidin, a chromatostrip partially coated with streptavidin, or the like) is recited.

Also there is a method of letting 5'-end or 3'-end of a specific oligonucleotide covalently bind with a molecule having an active functional group such as an amino group, a thiol group, or an aldehyde group, and then letting it covalently bind to a support made of glass, a polysaccharide derivative, silica gel, the synthetic resin or thermostable plastic whose surface is activated by a silane coupling agent or the like. Covalent binding is achieved, for example, by a spacer formed by serially connecting five triglycerides, a cross linker or the like. Also there is a method of chemical synthesis from the terminal side of the specific oligonucleotide directly on a support of glass or silicon.

Other concrete methods may be practiced in the following manners, using "biotinylated specific oligonucleotide" whose terminal end is labeled with biotin as a specific oligonucleotide that is immobilizable to a support.
(a) A mixture is obtained by adding an annealing buffer (for example, 33 mM Tris-Acetate pH 7.9, 66 mM KOAc, 10 mM MgOAc₂, 0.5 mM Dithiothreitol) and a biotinylated specific oligonucleotide to the DNA sample derived from genomic DNA contained in the biological specimen. Then the obtained mixture is heated at 95°C, for example, for several minutes to make double-stranded DNA derived from genomic DNA contained in the biological specimen into single-stranded DNA. Then the mixture is rapidly cooled to a temperature that is lower than the Tm value of the biotinylated specific oligonucleotide by about 10 to 20°C, and kept at this temperature, for example, for several minutes, and then the temperature is returned to room temperature for allowing formation of a bound body between the single-stranded DNA containing the target DNA region and the biotinylated specific oligonucleotide (the bound body formed in this stage includes a bound body between single-stranded DNA containing an unmethylated target DNA region and the specific oligonucleotide, as well as a bound body between single-stranded DNA containing the methylated target DNA region and the specific oligonucleotide).
(b) By adding the mixture obtained in the above (a) to a support coated with streptavidin, and retaining it at 37°C for example, for several minutes, the bound body between the single-stranded DNA containing the target DNA region and the biotinylated specific oligonucleotide is immobilized to the support coated with streptavidin. Thereafter, the remaining solution is removed and washing is performed. A washing buffer (for example, a 0.05% Tween 20-containing phosphate buffer (1 mM KH₂PO₄, 3 mM Na₂HPO·7H₂O, 154 mM NaCl pH 7.4)) is added, for example, in a proportion of 300 µL/well, and the solution is removed. This washing operation is repeated several times, to leave the bound body between the biotinylated specific oligonucleotide immobilized to the support, and the single-stranded DNA containing the target DNA region, on the well (also in this stage, the bound body formed in this state includes a bound body between single-stranded DNA containing an unmethylated target DNA region and the specific oligonucleotide, as well as a bound body between single-stranded DNA containing the methylated target DNA region and the specific oligonucleotide).
(c) An appropriate amount (for example, 100 µL/well of a 4 µg/mL solution) of a methylated DNA antibody is added to the well, and then left still at room temperature, for example, for about 3 hours, to promote formation of a complex of the methylated DNA antibody, single-stranded DNA containing the methylated target DNA region, of the single-stranded DNA, and the biotinylated specific oligonucleotide (formation of a complex) (in this stage, the bound body between the single-stranded DNA containing an unmethylated target DNA region and the specific oligonucleotide fails to form a complex). Thereafter, the remaining solution is removed and washing is performed. A washing buffer (for example, a 0.05% Tween 20-containing phosphate buffer (1 mM KH₂PO₄, 3 mM Na₂HPO·7H₂O, 154 mM NaCl pH 7.4)) is added, for example, in a proportion of 300 µL/well, and the solution is removed. This washing operation is repeated several times, to leave the complex on the well (selection of a complex).

The annealing buffer used in (a) is not limited to the annealing buffer described above as far as it is suited for binding between the specific oligonucleotide and single-stranded DNA containing the target DNA region derived from a biological sample. Use of a buffer in which a divalent ion, preferably a magnesium ion is dissolved in a concentration of 1 to 600 mM will improve the binding stability.

The washing operation in (b) and (c) is important for removing the methylated DNA antibody that is not immobilized and suspended in the solution, single-stranded DNA that is not bound to the methylated DNA antibody and suspended in the solution, or DNA that has been digested with a restriction enzyme to be described later and suspended in the solution, from the reaction solution. The washing buffer is not limited to the washing buffer described above, insofar as it is suited for removing the free methylated DNA antibody, single-stranded DNA and so on suspended in the solution, and a DELFIA buffer (available from PerkinElmer, Tris-HCl pH 7.8 with Tween 80), a TE buffer and the like may be used.

As described above, in the above (a) to (c), binding between single-stranded DNA containing the target DNA region and the biotinylated specific oligonucleotide is conducted in the stage previous to the immobilization of the biotinylated specific oligonucleotide to the support coated with streptavidin, however, this order may be inverted. In other words, for example, a mixture is obtained by adding the DNA sample derived from genomic DNA contained in the biological specimen to the biotinylated specific oligonucleotide immobilized to the support coated with streptavidin. For making double-stranded DNA containing the target DNA region possessed by genomic DNA contained in the biological specimen into a single strand, the obtained mixture is heated at 95°C, for example, for several minutes, and then for allowing formation of a bound body with the biotinylated specific oligonucleotide, the reaction is rapidly cooled to a temperature lower than the Tm value of the biotinylated specific oligonucleotide by about 10 to 20°C, and retained at this temperature, for example, for several minutes (the bound body formed in this stage includes a bound body between methylated single-stranded DNA other than the target DNA region and the methylated antibody, as well as a bound body between single-stranded DNA containing the methylated target DNA region and the methylated antibody). Thereafter, the operation of (c) may be practiced, to form and select a complex (in this stage, a bound body between single-stranded DNA containing an unmethylated target DNA region and the specific oligonucleotide fails to form a complex).

The operations of the above (a) to (c) may be conducted using a chromatostrip. In this case, the operation is conducted in the following manner. The solution in which the bound body between the single-stranded DNA containing the target DNA region possessed by genomic DNA contained in the biological specimen and the biotinylated specific oligonucleotide is formed is developed by a chromatostrip partially coated with streptavidin. By this operation, the bound body between the single-stranded DNA containing the target DNA region possessed by genomic DNA contained in the biological specimen and the biotinylated specific oligonucleotide is immobilized to the part coated with streptavidin (the bound body formed in this stage includes a bound body between methylated single-stranded DNA other than the target DNA region and the methylated antibody, as well as a bound body between single-stranded DNA containing the methylated target DNA region and the methylated antibody). Then an appropriate amount of the methylated DNA antibody is developed by a chromatostrip as described above. Through these operations, the complex of single-stranded DNA containing the methylated target DNA region possessed by genomic DNA contained the biological specimen, the biotinylated specific oligonucleotide, and the methylated DNA antibody is immobilized to the part coated with streptavidin (formation and selection of a complex) (in this stage, the bound body between the single-stranded DNA containing an unmethylated target DNA region and the specific oligonucleotide fails to form a complex). Also for these operations, the order of formation of a complex may be inverted. For example, after forming a complex made up of single-stranded DNA containing the methylated target DNA region, a biotinylated specific oligonucleotide, and a methylated DNA antibody, the complex may be developed by a chromatostrip, and immobilized to the part coated with streptavidin. In these operations, unnecessary components can be removed by developing the solution by a chromatostrip, and a washing operation can be omitted. Of course, a washing operation (development of a chromatostrip by a washing buffer (for example, a 0.05% Tween 20-containing phosphate buffer (1 mM KH₂PO₄, 3 mM Na₂HPO·7H₂O, 154 mM NaCl pH 7.4))) may be conducted between operations without causing any problem.

The "identification function" in Third step is a function based on labeling used for detection or quantification, and concretely, in the case of a methylated DNA antibody, a fluorescent or a chromogenic property of a label in a methylated DNA antibody labeled with europium, gold colloid, latex beads, a radioisotope, a fluorescent substance (FITC or the like), horseradish Peroxidase (HRP), alkaline phosphatase, biotin or the like can be recited, and the property that the antibody itself binds to a secondary antibody and the like are also recited as the function. As a means for quantifying or detecting such a function, for example, measurement by a radiation detector or a spectrophotometer, or visual check may be used.

In the case of a methylated DNA antibody, as described above, the property that the antibody itself binds to a secondary antibody or the like is considered as the function. That is, even when the antibody itself is not labeled, a methylated DNA antibody can be indirectly quantified or detected by using a secondary antibody to which the quantifiable or detectable recognition function is imparted.

When a methylated DNA antibody is quantified or detected depending on its identification function, concretely, when the property of the antibody itself is used as the function, the operation may be conducted in the following manner. A secondary antibody against a methylated DNA antibody (for example, an Eu-N1-labeled mouse IgG antibody: available from PerkinElmer) is added to a complex, and left still at room temperature for about an hour, to promote binding of the secondary antibody to the complex. Then, Enhancement Solution (available from PerkinElmer) is added and mixed, and left still, for example, for about 45 minutes at room temperature. Thereafter, the fluorescence (excitation 340 nm/fluorescence 612 nm) is measured by a fluorescence detector to quantify or detect the methylated DNA antibody. When an antibody to which FITC is bound is used as a secondary antibody, the methylated DNA antibody may be quantified or detected by measuring fluorescence of FITC by a known method. For example, when biotin is not used for immobilization of the specific oligonucleotide, a biotinylated methylated DNA antibody may be used for quantification or detection. When the biotinylated methylated DNA antibody is quantified or detected, a biotinylated methylated DNA antibody can be quantified or detected, for example, by adding and mixing HRP-labeled streptavidin with the biotinylated methylated DNA antibody, and measuring the activity of HRP by a known method after formation of a bound body between the biotinylated methylated DNA antibody and HRP-labeled streptavidin.

The present invention includes a modified method (Modified method 1) additionally comprising a step of digesting unmethylated single-stranded DNA by a methylation sensitive restriction enzyme capable of digesting single-stranded DNA after First step and before Third step, and a modified method (Modified method 2) additionally comprising a step of digesting an unmethylated single-stranded DNA by a methylation sensitive restriction enzyme in a reaction system containing a specific masking oligonucleotide comprising a recognition sequence for the methylation sensitive restriction enzyme as its part after First step and before Third step of the present invention.

The "methylation sensitive restriction enzyme" in these modified methods (Modified method 1 and Modified method 2) means, for example, a restriction enzyme or the like that fails to digest a recognition sequence containing methylated cytosine, but digests only a recognition sequence containing unmethylated cytosine. In other words, in the case of DNA wherein cytosine contained in a recognition sequence inherently recognizable by the methylation sensitive restriction enzyme is methylated, the DNA will not be cleaved even when the methylation sensitive restriction enzyme is caused to act on the DNA. On the other hand, in the case of DNA wherein cytosine contained in a recognition sequence inherently recognizable by the methylation sensitive restriction enzyme is not methylated, the DNA will be cleaved when the methylation sensitive restriction enzyme is caused to act on the DNA. Concrete examples of such methylation sensitive enzymes include HpaII, BstUI, NarI, SacII, and HhaI. The methylation sensitive restriction enzyme fails to cleave double-stranded DNA in a hemimethyl state (double-stranded DNA wherein cytosine (CpG) in one strand is methylated, and complementary cytosine (CpG) in the other strand is not methylated), and this is already revealed by Gruenbaum et al. (Nucleic Acid Research, 9, 2509-2515).

The wording "methylation sensitive restriction enzyme capable of digesting single-stranded DNA" means, for example, a restriction enzyme or the like capable of digesting only a recognition sequence containing unmethylated cytosine without digesting a recognition sequence containing methylated cytosine in single-stranded DNA. In other words, some methylation sensitive restriction enzymes digest single-stranded DNA. For example, HhaI or the like is recited.

As a fear in a digestion treatment with a methylation sensitive restriction enzyme, the possibility that the recognition sequence containing unmethylated cytosine may not be completely digested (so called "DNA remaining undigested") is recited. When such a possibility is problematic, the target DNA region has at least one recognition site of a methylation sensitive restriction enzyme, and as many as possible recognition sites would be preferred because it is possible to suppress the "DNA remaining undigested" as much as possible by presence of abundant recognition sites of a methylation sensitive restriction enzyme.

As described above, as to the target DNA region, from the viewpoint of the property of the methylated DNA antibody (one antibody binds to one methylated base (cytosine)), a region where a number of methylated bases (cytosine, CpG) exist is desirably selected as a target DNA region, and from the viewpoint of minimizing "DNA remaining undigested", a region where a number of recognition sites of a methylation sensitive restriction enzyme exist is desirably selected.

The term "specific masking oligonucleotide" in Modified method 2 means an oligonucleotide comprising a recognition sequence for a methylation sensitive restriction enzyme as its part. Concretely, it is an oligonucleotide that forms double-stranded DNA by complementary binding with one specific site (one or more sites may be concurrently contained in the following 8 to 30 bases depending on the nucleotide sequence of the target region) of several recognition sequences of a methylation sensitive restriction enzyme contained in the target DNA region in single-stranded DNA (namely, the site is made into a double-stranded state), thereby enabling the methylation sensitive restriction enzyme that uses only double-stranded DNA as a substrate to digest the site, and improving digestion efficiency at the site for the methylation sensitive restriction enzyme capable of digesting single-stranded DNA (the methylation sensitive restriction enzyme capable of digesting single-stranded DNA also digests double-stranded DNA, and digestion efficiency thereof is higher for double-stranded DNA than for single-stranded DNA), and is a nucleotide not inhibiting formation of a bound body between single-stranded DNA containing DNA of a target region and the specific oligonucleotide.

As the specific masking oligonucleotide, more concretely, oligonucleotides comprising a nucleotide sequence complementary to a nucleotide sequence having a length of 8 to 30 bases containing one specific site (one or more sites may be concurrently contained in the following 8 to 30 bases depending on the nucleotide sequence of the target) among several recognition sequences of the methylation sensitive restriction enzyme contained in the target DNA region in single-stranded DNA, and not inhibiting formation of the bound body between the single-stranded DNA containing DNA of the target region and the specific oligonucleotide can be recited.

The specific masking oligonucleotide to be mixed with the DNA sample derived from genomic DNA may be one kind or plural kinds. When plural kinds are used, many of recognition sites of the methylation sensitive restriction enzyme in single-stranded DNA containing a target DNA region become a double-stranded DNA state, and "DNA remaining undigested" as described above by a methylation sensitive restriction enzyme can be minimized. However, since the methylated DNA antibody is no longer able to bind to the site where the specific masking oligonucleotide forms double-stranded DNA, it is not appropriate to use the specific masking oligonucleotide for too many recognition sites of the methylation sensitive restriction enzyme, and it is preferred to use appropriate kinds. If the specific masking oligonucleotide is used for every recognition site of the methylation sensitive restriction enzyme contained in the target DNA region, binding between a methylated base (cytosine) and the methylated DNA antibody in the recognition site is inhibited, and the probability of formation of a complex comprising a methylated target DNA region can be decreased (of course, the methylated DNA antibody binds to CpG other than the recognition site, however, when the methylated DNA antibody is quantified or detected, the quantification accuracy and the detection sensitivity are decreased). For example, it is particularly useful to use the specific masking oligonucleotide designed for the site where it is necessarily intended not to be digested when it is methylated and intended to be digested when it is not methylated among several recognition sequences of the methylation sensitive restriction enzyme contained in the target DNA region (for example, the site that is methylated at 100% in an affected diseased patient specimen, but is not methylated at 100% in a healthy subject specimen).

In these Modified methods (Modified method 1 and Modified method 2), by conducting the step of digesting by at least one kind of methylation sensitive restriction enzyme after First step and before Third step, it is possible to minimize (exclude) formation of a complex comprising an unmethylated target DNA region, so that the quantification accuracy and the detection sensitivity of methylated DNA in the target DNA region are improved.

In Modified method 1, the operation of digesting the single-stranded DNA obtained in First step by at least one kind of methylation sensitive restriction enzyme capable of digesting single-stranded DNA executed after end of First step and before start of Third step may be concretely executed in the following manner. To 10 µL of a specimen solution containing single-stranded DNA obtained in First step, 3 µL of an optimum 10 x buffer, 15 U of a methylation sensitive enzyme (HhaI) capable of digesting a single strand, and an appropriate amount of BSA or the like as necessary are added, and then the resultant mixture is added with sterile ultrapure water to a liquid volume of 30 µL, and then incubated at 37°C, for example, for an hour to overnight. As a result, the HhaI recognition site unmethylated in the target DNA region will be digested (a treatment solution). For example, when this operation is conducted before formation of a complex, the treatment solution may be directly used for formation of a complex because a washing operation is executed at the time of formation of a complex and (or) selection. When this operation is conducted after selection of a complex, since it is necessary to remove single-stranded DNA (digested and generated single-stranded DNA because the recognition site of the methylation sensitive restriction enzyme is not methylated) suspended in the treatment solution from the treatment solution, it is necessary to conduct a washing operation similar to that executed in formation of a complex and (or) selection again after end of this operation.

In Modified method 2, the operation of conducting a digestion treatment by adding the single-stranded DNA obtained in First step, with at least one kind of methylation sensitive restriction enzyme and the specific masking oligonucleotide comprising a recognition sequence for the methylation sensitive restriction enzyme as its part, after end of First step and before start of Third step, may be concretely conducted, for example, in the following manner. To 10 µL of a specimen solution containing single-stranded DNA obtained in First step, 3 µL of an optimum 10 x buffer, 15 U of a methylation sensitive enzyme (HhaI, HhaI or the like), about 10 pmol of the specific masking oligonucleotide for one specific site among recognition sequences of the methylation sensitive enzyme, and an appropriate amount of BSA or the like as necessary are added, and then the resultant mixture is added with sterile ultrapure water to a liquid volume of 30 µL, and then incubated at 37°C, for example, for an hour to overnight. As a result, the site unmethylated in the target DNA region will be digested (a treatment solution). For example, when this operation is conducted before formation of a complex, the treatment solution may be directly used for formation of a complex because the washing operation is conducted at the time of formation of a complex and (or) selection. When this operation is conducted after selection of a complex, since it is necessary to remove single-stranded DNA (digested and generated single-stranded DNA because a recognition site of the methylation sensitive restriction enzyme is not methylated) suspended in the treatment solution from the treatment solution, it is necessary to conduct a washing operation similar to that executed in formation of a complex and (or) selection again after end of this operation.

In the present invention, in one preferred embodiment, "a DNA sample derived from genomic DNA contained in the biological specimen" is a DNA sample that is preliminarily digested with a restriction enzyme recognition cleaving site for which is not present in the target DNA region of the genomic DNA. In forming a bound body between single-stranded DNA containing the target DNA region possessed by genomic DNA contained in the biological specimen and the specific oligonucleotide, it is expected that the shorter the single-stranded DNA is, the better the operability is and the more easily a complex is formed as far as the target DNA region is contained. To shorten the single-stranded DNA, it is efficient to make it short when it is in former genomic DNA. Therefore, a digestion treatment may be preliminarily conducted using a restriction enzyme recognition cleaving site for which is not present in the target DNA region on a DNA sample derived from genomic DNA contained in the biological specimen. As a method of the digestion treatment by the restriction enzyme whose recognition cleaving site is not present in the target DNA region, a generally known restriction enzyme treatment method may be used. When the specimen is a DNA sample preliminarily purified, a digestion treatment may be executed using a generally used amount of a restriction enzyme, whereas when the specimen is a tissue lysate, a cell lysate or the like, a digestion treatment may be conducted using a large excess of a methylation sensitive restriction enzyme, for example, an amount of 500 times or more the DNA amount of a restriction enzyme.

In one preferred embodiment, "a DNA sample derived from genomic DNA contained in the biological specimen" is a DNA sample that has been digested with at least one kind of methylation sensitive restriction enzyme. By preliminarily digesting a biological specimen itself with a methylation sensitive restriction enzyme, it is possible to quantify or detect methylation at high accuracy in Final step. This method is useful for eliminating "DNA remaining undigested" as described above. As a method of digesting the specimen itself with a methylation sensitive restriction enzyme, when genomic DNA in the specimen exists in a state of double-stranded DNA, a generally known restriction enzyme treatment may be conducted using a methylation sensitive restriction enzyme. On the other hand, when genomic DNA in the specimen exists in a state of single-stranded DNA or when genomic DNA in the specimen exists in combined states of double-stranded DNA and single-stranded DNA, a digestion treatment may be conducted by the method corresponding to Modified method 1 or Modified method 2. Concretely, for example, to 10 µL of a specimen solution, 5 µL of an optimum 10 x buffer, 15 U of a methylation sensitive restriction enzyme (HhaI) capable of digesting single-stranded DNA, and an appropriate amount of BSA or the like as necessary are added, and then the resultant mixture is added with sterile ultrapure water to a liquid volume of 50 µL, and then incubated at 37°C, for example, for an hour to overnight. Alternatively, to 10 µL of a specimen, 5 µL of an optimum 10 x buffer, 15 U of a methylation sensitive restriction enzyme (HpaII, HhaI or the like), about 15 pmol of a specific masking oligonucleotide for one specific site among recognition sequences of the methylation sensitive enzyme, and an appropriate amount of BSA or the like as necessary are added and then the resultant mixture is added with sterile ultrapure water to a liquid volume of 50 µL, and then incubated at 37°C, for example, for an hour to overnight. When the specimen is a preliminarily purified DNA sample, the treatment may be executed using a generally used amount of a restriction enzyme, whereas when the specimen is a tissue lysate, a cell lysate or the like, the treatment may be conducted using a large excess of a methylation sensitive restriction enzyme, for example, an amount of 500 times or more the DNA amount of a methylation sensitive restriction enzyme.

As a method of quantifying or detecting minor substances contained in a biological sample such as blood or urine, immunological measuring methods are generally used. Among such methods, a so-called immunochromatography using chromatography is widely used in various situations including, for example, clinical examinations in hospitals, assays in laboratories and so on because of its simple operation and short time required for assay. In recent years, a so-called hybrid chromatography has been utilized in which labeled DNA (gene) is developed on a chromatostrip, and target DNA (gene) is detected by hybridization using a probe capable of capturing the target DNA (gene). Also this method is now coming to be widely used in situations including, for example, clinical examinations in hospitals, assays in laboratories and so on because of its simple operation and short time required for assay. The present invention conceptually enables a combined method of the immunochromatography and the hybrid chromatography. In the present invention, since the order of formation of a complex and selection of a complex is not particularly limited, various methods are possible. Concretely, such methods may be executed in the following manner.

Method 1: A sample after end of First step is added with the biotinylated specific oligonucleotide, to cause formation of a bound body between single-stranded DNA containing the target DNA region and the biotinylated specific oligonucleotide (the bound body formed in this stage includes not only a bound body between single-stranded DNA containing the methylated target DNA region and the specific oligonucleotide, but also a bound body between single-stranded DNA containing an unmethylated target DNA region and the specific oligonucleotide), and added with a methylated antibody having the function, to cause formation of a complex of the single-stranded DNA containing the methylated target DNA region, the biotinylated specific oligonucleotide, and a methylated DNA antibody having the function (in this stage, the bound body between the single-stranded DNA containing an unmethylated target DNA region and the specific oligonucleotide fails to form a complex). Upon dropping (introduction) of the obtained sample into an introduction part of a chromatostrip, the complex migrates in a development part by a capillary phenomenon, and is trapped in the part preliminarily coated with streptavidin. Thereafter, by quantifying or detecting the methylated DNA antibody forming the obtained complex according to its function, methylated DNA in the target DNA region can be quantified or detected.

Method 2: A sample after end of First step is added with the biotinylated specific oligonucleotide, to cause formation of a bound body between single-stranded DNA containing the target DNA region and the biotinylated specific oligonucleotide (the bound body formed in this stage includes not only a bound body between single-stranded DNA containing the methylated target DNA region and the specific oligonucleotide, but also a bound body between single-stranded DNA containing an unmethylated target DNA region and the specific oligonucleotide). Upon dropping (introduction) of the obtained sample into an introduction part of a chromatostrip, the bound body migrates in a development part by a capillary phenomenon, and is trapped in the part preliminarily coated with streptavidin (also in this stage, the bound body includes not only a bound body between single-stranded DNA containing the methylated target DNA region and the specific oligonucleotide, but also a bound body between single-stranded DNA containing an unmethylated target DNA region and the specific oligonucleotide). Then, upon dropping (introduction) of a methylated antibody having the function into an introduction part, it migrates in a development part and binds to methylated cytosine of the bound body, to form a complex of single-stranded DNA containing the methylated target DNA region, the biotinylated specific oligonucleotide, and a methylated DNA antibody having the function (in this stage, the bound body between the single-stranded DNA containing an unmethylated target DNA region and the specific oligonucleotide fails to form a complex). By quantifying or detecting a methylated DNA antibody contained in the obtained complex according to its function, methylated DNA in the target DNA region can be quantified or detected.

Method 3: Upon dropping (introduction) of the biotinylated specific oligonucleotide into an introduction part of a chromatostrip, the oligonucleotide migrates in a development part by a capillary phenomenon, and is trapped in the part preliminarily coated with streptavidin. Then, upon dropping (introduction) of a sample after end of First step into an introduction part, it migrates in a development part, and is trapped by the biotinylated specific oligonucleotide that has been already trapped in the condition that single-stranded DNA containing the target DNA region forms a bound body (the bound body formed in this stage includes not only a bound body between single-stranded DNA containing the methylated target DNA region and the specific oligonucleotide, but also a bound body between single-stranded DNA containing an unmethylated target DNA region and the specific oligonucleotide). Then, upon dropping (introduction) of a methylated antibody having the function into an introduction part, it migrates in a development part, and binds to methylated cytosine of the bound body, to form a complex of single-stranded DNA containing the methylated target DNA region, the biotinylated specific oligonucleotide, and the methylated DNA antibody having the function (in this stage, the bound body between the single-stranded DNA containing an unmethylated target DNA region and the specific oligonucleotide fails to form a complex). By quantifying or detecting the methylated DNA antibody forming the obtained complex according to its function, methylated DNA in the target DNA region can be quantified or detected.

Method 4: Upon dropping (introduction) of the biotinylated specific oligonucleotide into an introduction part of a chromatostrip, the oligonucleotide migrates in a development part by a capillary phenomenon, and is trapped in the part preliminarily coated with streptavidin. A sample after end of First step is added with the methylated DNA antibody having the function, to form a bound body between single-stranded DNA having methylated cytosine (in which single-stranded DNA containing the target DNA region and single-stranded DNA other than the target exist) and the methylated DNA antibody having the function (the bound body formed in this stage includes not only a bound body between single-stranded DNA containing the methylated target DNA region and the methylated antibody, but also a bound body between methylated single-stranded DNA other than the target DNA region and the methylated antibody). Upon dropping (introduction) of the obtained bound body into an introduction part, it migrates in a development part, and single-stranded DNA containing the methylated target DNA region binds to the biotinylated specific oligonucleotide that has been already trapped, to form a complex of single-stranded DNA containing the methylated target DNA region, the biotinylated specific oligonucleotide, and the methylated DNA antibody having the function (in this stage, the bound body between methylated single-stranded DNA other than in the target DNA region and a methylated antibody fails to form a complex). By quantifying or detecting the methylated DNA antibody contained in the obtained complex according to its function, methylated DNA in the target DNA region can be quantified or detected.

Also a plurality of detection sites may be provided on a single chromatostrip (the specific oligonucleotides capable of trapping different target DNA regions are immobilized to a support), and each target DNA region may be sequentially quantified or detected, and by enabling one detection site to trap a plurality of target DNA regions, or by immobilizing a number of the specific oligonucleotides capable of trapping a plurality of target DNA regions on one detection site, it is possible to dramatically improve the detection sensitivity. Also, using a number of the specific oligonucleotides having the function capable of trapping a plurality of target DNA regions so as to allow formation of a complex with a plurality of target DNA regions will also improve the detection sensitivity dramatically. Further, designing a number of the specific oligonucleotides in a single target region, and using these on the side of a support or on the side of detection will also improve the detection sensitivity dramatically.

The restriction enzyme, the specific oligonucleotide, or the methylated DNA antibody which may be used in the present invention as described above is useful as a reagent in a detection kit. The present invention also provides a detection kit containing such a restriction enzyme, the specific oligonucleotide, or the methylated DNA antibody and so on as a regent, and a detection chip including a support on which the specific oligonucleotide, or the methylated DNA antibody and so on is immobilized, and the scope of the present invention implies use in the form of a detection kit or a detection chip as described above utilizing a substantial principle of the method.

### Examples

In the following, the present invention will be explained in detail by way of examples, which are not intended to limit the present invention.

### Example 1

A colon-adenocarcinoma cell strain Capan-2 (ATCC NO. HTB-80) derived from a mammal and purchased from ATCC was cultured to be confluent in a culture medium in which equivalent amounts of McCoy's5a (Gibco, Cat. No. 16600-082) and FBS (ATCC, CatNo. 30-2020) were mixed, to obtain about 1 x 10⁷ of cells. The obtained cells were added with a SEDTA buffer [10 mM Tris-HCl pH 8.0, 10 mM EDTA pH 8.0, 100 mM NaCl] in a volume of 10 times, and homogenized.

The obtained mixture was added with proteinase K (Sigma) in a concentration of 500 µg/mL and sodium dodecyl sulfate in a concentration of 1% (w/v), and shaken at 55°C for about 16 hours, and then subjected to a phenol/chloroform extraction treatment using phenol saturated with 1 M Tris-HCl, pH 8.0. The aqueous layer was collected, and added with NaCl in a concentration of 0.5 N, and the precipitate (genomic DNA) generated by an ethanol precipitation treatment was collected.

The collected precipitate was dissolved in a TE buffer (10 mM Tris, 1 mM EDTA, pH 8.0), and added with RNase A (Sigma) in a concentration of 40 µg/ml, and incubated at 37°C for 1 hour, and then subjected to a phenol/chloroform extraction treatment. The aqueous layer was collected, added with NaCl in a concentration of 0.5 N, and the precipitate (genomic DNA) generated by an ethanol precipitation treatment was collected. The collected precipitate was rinsed with 70% ethanol, to obtain Capan-2 genomic DNA.

Each of 500 ng/20 µL solutions in TE buffer of Genomic DNA derived from human blood purchased from Clontech, and the above-described Capan-2 genomic DNA was prepared in duplicate.

Each 20 µL of the prepared genomic DNA solution, 10 U of restriction enzyme XspI, and 5 µL of 10 x buffer optimum for XspI (200 mM Tris-HCl pH 8.5, 100 mM MgCl₂, 10 mM Dithiothreitol, 1000 mM KCl) were mixed, and added with sterilized ultrapure water to make the liquid volume 50 µL, and the liquid was incubated at 37°C for 1 hour, to prepare a genome solution.

As a specific oligonucleotide used for obtaining a target DNA region of either of SEQ ID NOs: 1 to 12, 5'-end biotinylated oligonucleotide having a nucleotide sequence of SEQ ID NOs: 13 to 24, and biding to the DNA region by complementation was synthesized, and each 0.1 pmoL/µL solution in TE buffer was prepared in the following manner.

A solution of 5'-end biotinylated oligonucleotide having a nucleotide sequence of SEQ ID NO: 13 for obtaining a target region of SEQ ID NO: 1 was named a specific oligonucleotide solution 1.

A solution of 5'-end biotinylated oligonucleotide having a nucleotide sequence of SEQ ID NO: 13 for obtaining a target region of SEQ ID NO: 1 was named a specific oligonucleotide solution 1.

A solution of 5'-end biotinylated oligonucleotide having a nucleotide sequence of SEQ ID NO: 14 for obtaining a target region of SEQ ID NO: 2 was named a specific oligonucleotide solution 2.

A solution of 5'-end biotinylated oligonucleotide having a nucleotide sequence of SEQ ID NO: 15 for obtaining a target region of SEQ ID NO: 3 was named a specific oligonucleotide solution 3.

A solution of 5'-end biotinylated oligonucleotide having a nucleotide sequence of SEQ ID NO: 16 for obtaining a target region of SEQ ID NO: 4 was named a specific oligonucleotide solution 4.

A solution of 5'-end biotinylated oligonucleotide having a nucleotide sequence of SEQ ID NO: 17 for obtaining a target region of SEQ ID NO: 5 was named a specific oligonucleotide solution 5.

A solution of 5'-end biotinylated oligonucleotide having a nucleotide sequence of SEQ ID NO: 18 for obtaining a target region of SEQ ID NO: 6 was named a specific oligonucleotide solution 6.

A solution of 5'-end biotinylated oligonucleotide having a nucleotide sequence of SEQ ID NO: 19 for obtaining a target region of SEQ ID NO: 7 was named a specific oligonucleotide solution 7.

A solution of 5'-end biotinylated oligonucleotide having a nucleotide sequence of SEQ ID NO: 20 for obtaining a target region of SEQ ID NO: 8 was named a specific oligonucleotide solution 8.

A solution of 5'-end biotinylated oligonucleotide having a nucleotide sequence of SEQ ID NO: 21 for obtaining a target region of SEQ ID NO: 9 was named a specific oligonucleotide solution 9.

A solution of 5'-end biotinylated oligonucleotide having a nucleotide sequence of SEQ ID NO: 22 for obtaining a target region of SEQ ID NO: 10 was named a specific oligonucleotide solution 10.

A solution of 5'-end biotinylated oligonucleotide having a nucleotide sequence of SEQ ID NO: 23 for obtaining a target region of SEQ ID NO: 11 was named a specific oligonucleotide solution 11.

A solution of 5'-end biotinylated oligonucleotide having a nucleotide sequence of SEQ ID NO: 24 for obtaining a target region of SEQ ID NO: 12 was named a specific oligonucleotide solution 12.

Equivalent amounts of the specific oligonucleotide solutions 1 to 12 were mixed, to prepare a specific oligonucleotide solution 13.

As a counter oligonucleotide designed with respect to the nucleotide sequence in the target region of either of SEQ ID NOs: 1 to 12, an oligonucleotide of either of SEQ ID NOs: 25 to 118 was synthesized, and respective 10 pmol/µL solutions in TE buffer were prepared. Equivalent amounts of the counter oligonucleotide solutions targeting the same DNA region were mixed, to prepare respective counter oligonucleotide mixed solutions shown below.

A mixed solution of oligonucleotides of SEQ ID NOs: 25 to 42 was named a counter oligonucleotide mixed solution 1.

A mixed solution of oligonucleotides of SEQ ID NOs: 43 to 60 was named a counter oligonucleotide mixed solution 2.

A mixed solution of oligonucleotides of SEQ ID NOs: 61 to 66 was named a counter oligonucleotide mixed solution 3.

A mixed solution of oligonucleotides of SEQ ID NOs: 67 to 70 was named a counter oligonucleotide mixed solution 4.

A mixed solution of oligonucleotides of SEQ ID NOs: 71 to 76 was named a counter oligonucleotide mixed solution 5.

A mixed solution of oligonucleotides of SEQ ID NOs: 77 to 82 was named a counter oligonucleotide mixed solution 6.

A mixed solution of oligonucleotides of SEQ ID NOs: 83 to 88 was named a counter oligonucleotide mixed solution 7.

A mixed solution of oligonucleotides of SEQ ID NOs: 89 to 94 was named a counter oligonucleotide mixed solution 8.

A mixed solution of oligonucleotides of SEQ ID NOs: 95 to 100 was named a counter oligonucleotide mixed solution 9.

A mixed solution of oligonucleotides of SEQ ID NOs: 101 to 106 was named a counter oligonucleotide mixed solution 10.

A mixed solution of oligonucleotides of SEQ ID NOs: 107 to 112 was named a counter oligonucleotide mixed solution 11.

A mixed solution of oligonucleotides of SEQ ID NOs: 113 to 118 was named a counter oligonucleotide mixed solution 12.

Equivalent amounts of the above counter oligonucleotide mixed solutions 1 to 12 were mixed, to prepare a counter oligonucleotide mixed solution 13.

Equivalent amounts of a specific oligonucleotide solution and a counter oligonucleotide mixed solution were mixed, to prepare the following Solution A, Solution B and Solution C.

Solution A: specific oligonucleotide solution 1 and counter oligonucleotide mixed solution 1

Solution B: specific oligonucleotide solution 2 and counter oligonucleotide mixed solution 2

Solution C: specific oligonucleotide solution 13 and counter oligonucleotide mixed solution 13

In a PCR tube, 30 µL of the genome solution prepared as described above, 30 µL of either one of the Solutions A to C, 15 µL of a buffer (330 mM Tris-Acetate pH 7.9, 660 mM KOAc, 100 mM MgOAc₂, 5 mM Dithiothreitol), 60 µL of a 10 mM MgCl₂ solution, and 15 µL of a 1 mg/mL BSA solution were added, to make the liquid volume 150 µL, and mixed. Then, the PCR tube was heated at 95°C for 10 minutes, rapidly cooled to 70°C, and retained for 10 minutes at that temperature. Then the reaction was cooled to 50°C and retained for 10 minutes, and further retained at 37°C for 10 minutes, and then the reaction was returned to room temperature, to promote formation of a bound body between the 5'-end biotin-labeled oligonucleotide and the DNA fragment.

All the obtained mixtures were transferred to wells coated with streptavidin, and left still for about 30 minutes at room temperature, to immobilize the bound bodies between the 5'-end biotin-labeled oligonucleotides and the DNA fragments to wells. Thereafter, the solution was removed by pipetting, and each well was washed with 200 µL of a washing buffer [0.05% Tween 20-containing phosphate buffer (1 mM KH₂PO₄, 3 mM Na₂HPO·7H₂O, 154 mM NaCl pH 7.4)] three times.

Each well was added with 100 µL of a methylcytosine antibody [available from Aviva Systems Biology, 0.5 µg/mL 0.1% BSA-containing phosphate buffer (1 mM KH₂PO₄, 3 mM Na₂HPO·7H₂O, 154 mM NaCl pH 7.4) solution], and left still for 1 hour at room temperature. Solutions in wells were removed by pipetting, and each well was washed with 200 µL of a washing buffer [0.05% Tween 20-containing phosphate buffer (1 mM KH₂PO₄, 3 mM Na₂HPO·7H₂O, 154 mM NaCl p H7.4)] three times.

Each well was added with 100 µL of an Eu-N1-labeled mouse IgG antibody [available from PerkinElmer, 0.05 µg/mL 0.1% BSA-containing phosphate buffer (1 mM KH₂PO₄, 3 mM Na₂HPO·7H₂O, 154 mM NaCl pH 7.4) solution], and left still at room temperature for 1 hour, and then solutions in the wells were removed by pipetting, and each well was washed with 200 µL of a washing buffer [0.05% Tween 20-containing phosphate buffer (1 mM KH₂PO₄, 3 mM Na₂HPO·7H₂O, 154 mM NaCl pH 7.4)] three times.

Each well was added with 150 µL of Enhancement Solution (available from PerkinElmer), stirred for 5 minutes at room temperature, and left still for 15 minutes at room temperature, and then fluorescence was measured at excitation 340 nm/fluorescence 612 nm.

The result is shown in Fig. 1. Methylation frequency or an index value correlated therewith was high in the Solution C that simultaneously detects a plurality of target regions to be detected, compared to the Solution A and Solution B in which the target region to be detected is one kind. The index value correlated with methylation frequency of the target region to be detected was higher in genomic DNA extracted from Capan-2 cancer cell strain, than in human genomic DNA derived from a noncancerous cell.

In the present experiment, by simultaneously measuring methylation frequencies or index values correlated therewith of a plurality of target DNA regions, it is possible to measure methylation frequency or an index value correlated therewith more effectively than measuring methylation frequency or an index value correlated therewith of one target DNA region. Also, the higher methylation frequency or the index value correlated therewith in genomic DNA derived from Capan-2 cell strain which is a pancreatic cancer cell than in genomic DNA derived from a noncancerous tissue reveals that measurement of methylation frequency or an index value correlated therewith by mixing the target DNA regions used in the present examination will be a diagnostic index of cancer. Also it was confirmed that mixing of a specific oligonucleotide and a counter oligonucleotide, and the specific oligonucleotides utilized in the present examination is useful for cancer diagnosis.

### INDUSTRIAL APPLICABILITY

Based on the present invention, it becomes possible to provide a method of quantifying or detecting methylated DNA in a target DNA region in a genomic DNA contained in a biological specimen in a simple and convenient manner, and so on.

### Free Text in Sequence Listing

SEQ ID NOs:13 to 24
   Designed biotinated oligonucleotide for fixation
SEQ ID NOs:25 to 118
   Designed oligonucleotide

## Claims

1. A method of quantifying or detecting methylated DNA in a target DNA region possessed by genomic DNA contained in a biological specimen, comprising:
First step of separating double-stranded DNA derived from genomic DNA comprising the target DNA region contained in the biological specimen into single-stranded DNA;
Second step of mixing the single-stranded DNA, a methylated DNA antibody, and a specific oligonucleotide comprising a nucleotide sequence of a part of a complementary sequence to any of the following nucleotide sequences and not inhibiting binding between the methylated target DNA region in the single-stranded DNA and the methylated DNA antibody, to form a complex of the single-stranded DNA, the methylated DNA antibody, and the specific oligonucleotide; and
Third step of detecting or quantifying the methylated DNA in the target DNA region contained in the biological specimen by conducting detection or quantification depending on an identification function that is possessed by the methylated DNA antibody contained in the complex and available for detection:
(1) the nucleotide sequence of SEQ ID NO:1 or a nucleotide sequence having 80% or more sequence identity to the sequence,
(2) the nucleotide sequence of SEQ ID NO:2 or a nucleotide sequence having 80% or more sequence identity to the sequence,
(3) the nucleotide sequence of SEQ ID NO:3 or a nucleotide sequence having 80% or more sequence identity to the sequence,
(4) the nucleotide sequence of SEQ ID NO:4 or a nucleotide sequence having 80% or more sequence identity to the sequence,
(5) the nucleotide sequence of SEQ ID NO:5 or a nucleotide sequence having 80% or more sequence identity to the sequence,
(6) the nucleotide sequence of SEQ ID NO:6 or a nucleotide sequence having 80% or more sequence identity to the sequence,
(7) the nucleotide sequence of SEQ ID NO:7 or a nucleotide sequence having 80% or more sequence identity to the sequence,
(8) the nucleotide sequence of SEQ ID NO:8 or a nucleotide sequence having 80% or more sequence identity to the sequence,
(9) the nucleotide sequence of SEQ ID NO:9 or a nucleotide sequence having 80% or more sequence identity to the sequence,
(10) the nucleotide sequence of SEQ ID NO:10 or a nucleotide sequence having 80% or more sequence identity to the sequence,
(11) the nucleotide sequence of SEQ ID NO:11 or a nucleotide sequence having 80% or more sequence identity to the sequence,
(12) the nucleotide sequence of SEQ ID NO:12 or a nucleotide sequence having 80% or more sequence identity to the sequence,
(13) the complementary sequence to the nucleotide sequence of SEQ ID NO:1 or a nucleotide sequence having 80% or more sequence identity to the complementary sequence,
(11) the complementary sequence to the nucleotide sequence of SEQ ID NO:2 or a nucleotide sequence having 80% or more sequence identity to the complementary sequence,
(15) the complementary sequence to the nucleotide sequence of SEQ ID NO:3 or a nucleotide sequence having 80% or more sequence identity to the complementary sequence,
(16) the complementary sequence to the nucleotide sequence of SEQ ID NO:4 or a nucleotide sequence having 80% or more sequence identity to the complementary sequence,
(17) the complementary sequence to the nucleotide sequence of SEQ ID NO:5 or a nucleotide sequence having 80% or more sequence identity to the complementary sequence,
(18) the complementary sequence to the nucleotide sequence of SEQ ID NO:6 or a nucleotide sequence having 80% or more sequence identity to the complementary sequence,
(19) the complementary sequence to the nucleotide sequence of SEQ ID NO:7 or a nucleotide sequence having 80% or more sequence identity to the complementary sequence,
(20) the complementary sequence to the nucleotide sequence of SEQ ID NO:8 or a nucleotide sequence having 80% or more sequence identity to the complementary sequence,
(21) the complementary sequence to the nucleotide sequence of SEQ ID NO:9 or a nucleotide sequence having 80% or more sequence identity to the complementary sequence,
(22) the complementary sequence to the nucleotide sequence of SEQ ID NO:10 or a nucleotide sequence having 80% or more sequence identity to the complementary sequence,
(23) the complementary sequence to the nucleotide sequence of SEQ ID NO:11 or a nucleotide sequence having 80% or more sequence identity to the complementary sequence, and
(24) the complementary sequence to the nucleotide sequence of SEQ ID NO:12 or a nucleotide sequence having 80% or more sequence identity to the complementary sequence.

2. The method according to claim 1, wherein the specific oligonucleotide comprises any of the following nucleotide sequences:
(1) the nucleotide sequence of SEQ ID NO:13,
(2) the nucleotide sequence of SEQ ID NO:14,
(3) the nucleotide sequence of SEQ ID NO:15,
(4) the nucleotide sequence of SEQ ID NO:16,
(5) the nucleotide sequence of SEQ ID NO:17,
(6) the nucleotide sequence of SEQ ID NO:18,
(7) the nucleotide sequence of SEQ ID NO:19,
(8) the nucleotide sequence of SEQ ID NO:20,
(9) the nucleotide sequence of SEQ ID NO:21,
(10) the nucleotide sequence of SEQ ID NO:22,
(11) the nucleotide sequence of SEQ ID NO:23
(12) the nucleotide sequence of SEQ ID NO:24,
(13) the complementary sequence to the nucleotide sequence of SEQ ID NO:13,
(14) the complementary sequence to the nucleotide sequence of SEQ ID NO:14,
(15) the complementary sequence to the nucleotide sequence of SEQ ID NO:15,
(16) the complementary sequence to the nucleotide sequence of SEQ ID NO:16,
(17) the complementary sequence to the nucleotide sequence of SEQ ID NO:17,
(18) the complementary sequence to the nucleotide sequence of SEQ ID NO:18,
(19) the complementary sequence to the nucleotide sequence of SEQ ID NO:19,
(20) the complementary sequence to the nucleotide sequence of SEQ ID NO:20,
(21) the complementary sequence to the nucleotide sequence of SEQ ID NO:21,
(22) the complementary sequence to the nucleotide sequence of SEQ ID NO:22,
(23) the complementary sequence to the nucleotide sequence of SEQ ID NO:23, and
(24) the complementary sequence to the nucleotide sequence of SEQ ID NO:24.

3. The method according to claim 1 or 2, wherein a counter oligonucleotide is added in forming the complex in Second step.

4. The method according to any one of claims 1 to 3, wherein formation of the complex in Second step is conducted in a reaction system containing a divalent cation.

5. The method according to any one of claims 1 to 4, wherein the methylated DNA antibody in the complex formed in Second step has been made to bind to a support before Third step.

6. The method according to any one of claims 1 to 5, further comprising, after First step and before Third step, the step of digesting unmethylated single-stranded DNA with a methylation sensitive restriction enzyme capable of digesting single-stranded DNA.

7. The method according to any one of claims 1 to 6, further comprising, after First step and before Third step, the step of digesting unmethylated single-stranded DNA with a methylation-sensitive restriction enzyme in a reaction system containing a specific masking oligonucleotide comprising a recognition sequence for the methylation sensitive restriction enzyme as a part thereof.

8. The method according to claim 6, wherein the methylation sensitive restriction enzyme capable of digesting single-stranded DNA is HhaI.

9. The method according to any one of claims 1 to 8, wherein the methylated DNA antibody is a methylcytosine antibody.

10. The method according to any one of claims 1 to 9, wherein the biological specimen is mammalian blood, serum, plasma, bodily fluid, cell lysate or tissue lysate.

11. The method according to any one of claims 1 to 10, wherein the DNA sample derived from genomic DNA contained in the biological specimen is digested in advance with a restriction enzyme recognition cleaving site for which is not present in the target DNA region of the genomic DNA.

12. The method according to any one of claims 1 to 11, wherein the DNA sample derived from genomic DNA contained in the biological specimen has been digested with a methylation sensitive restriction enzyme.

13. The method according to any one of claims 1 to 12, wherein the DNA sample derived from genomic DNA contained in the biological specimen has been digested with a methylation sensitive restriction enzyme in a reaction system containing a specific masking oligonucleotide.

14. The method according to any one of claims 7, 12 and 13, wherein the methylation sensitive restriction enzyme is HpaII or HhaI.

15. The method according to any one of claims 1 to 14, wherein the DNA sample derived from genomic DNA contained in the biological specimen is a DNA sample purified in advance.

16. The method according to any one of claims 1 to 15, wherein the target DNA region possessed by genomic DNA comprises a recognition cleaving site for the methylation sensitive restriction enzyme.

17. DNA comprising any of the following nucleotide sequences:
(1) the nucleotide sequence of SEQ ID NO:13,
(2) the nucleotide sequence of SEQ ID NO:14,
(3) the nucleotide sequence of SEQ ID NO:15,
(4) the nucleotide sequence of SEQ ID NO:16,
(5) the nucleotide sequence of SEQ ID NO:17,
(6) the nucleotide sequence of SEQ ID NO:18,
(7) the nucleotide sequence of SEQ ID NO:19,
(8) the nucleotide sequence of SEQ ID NO:20,
(9) the nucleotide sequence of SEQ ID NO:21,
(10) the nucleotide sequence of SEQ ID NO:22,
(11) the nucleotide sequence of SEQ ID NO:23
(12) the nucleotide sequence of SEQ ID NO:24,
(13) the complementary sequence to the nucleotide sequence of SEQ ID NO:13,
(14) the complementary sequence to the nucleotide sequence of SEQ ID NO:14,
(15) the complementary sequence to the nucleotide sequence of SEQ ID NO:15,
(16) the complementary sequence to the nucleotide sequence of SEQ ID NO:16,
(17) the complementary sequence to the nucleotide sequence of SEQ ID NO:17,
(18) the complementary sequence to the nucleotide sequence of SEQ ID NO:18,
(19) the complementary sequence to the nucleotide sequence of SEQ ID NO:19,
(20) the complementary sequence to the nucleotide sequence of SEQ ID NO:20,
(21) the complementary sequence to the nucleotide sequence of SEQ ID NO:21,
(22) the complementary sequence to the nucleotide sequence of SEQ ID NO:22,
(23) the complementary sequence to the nucleotide sequence of SEQ ID NO:23, and
(24) the complementary sequence to the nucleotide sequence of SEQ ID NO:24.
